(19) Europäisches Patentamt / European Patent Office / Office européen des brevets

(11) EP 2 292 589 A1

(12) **EUROPEAN PATENT APPLICATION**

(43) Date of publication:
**09.03.2011 Bulletin 2011/10**

(21) Application number: **09169269.9**

(22) Date of filing: **02.09.2009**

(51) Int Cl.:
*C07C 237/20* (2006.01) *C07C 237/22* (2006.01)
*C07C 271/20* (2006.01) *C07D 213/56* (2006.01)
*C07D 239/26* (2006.01) *C07D 263/24* (2006.01)
*C07D 277/06* (2006.01) *C07D 333/24* (2006.01)
*C07D 295/185* (2006.01) *A61P 3/10* (2006.01)
*A61P 17/00* (2006.01) *A61P 29/00* (2006.01)
*A61P 37/00* (2006.01) *A61K 31/164* (2006.01)
*A61K 31/165* (2006.01) *A61K 31/167* (2006.01)
*A61K 31/4965* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **IMTM GmbH**
**39120 Magdeburg (DE)**

(72) Inventors:
- **Ansorge, Siegfried**
  **39291 Hohenwarthe (DE)**
- **Bank, Ute**
  **39419 Stassfurt (DE)**
- **Nordhoff, Karsten**
  **39118 Magdeburg (DE)**
- **Röhnert, Peter**
  **39112 Magdeburg (DE)**
- **Stefin, Sofia**
  **39114 Magdeburg (DE)**
- **Striggow, Frank**
  **39175 Gerwisch (DE)**
- **Täger, Michael**
  **39326 Heinrichsberg (DE)**

(74) Representative: **Koepe & Partner**
**Patentanwälte**
**Postfach 22 12 64**
**80502 München (DE)**

(54) **Novel multifunctional peptidase inhibitors, especially for medical use**

(57) The invention relates to compounds of the general formula (1)

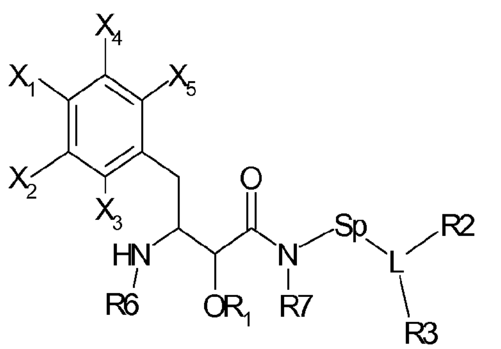

(1)

or the acid addition salts thereof with organic and/or inorganic acids; as well as to the use of the compounds of the general formula (1) in medicine.



Printed by Jouve, 75001 PARIS (FR)

## Description

The invention relates to novel substances and compounds which are capable of concertedly inhibiting the enzymes (ia) dipeptidyl peptidase IV (DPIV) as well as (ib) peptidases having an enzymatic effect analogous to dipeptidyl peptidase IV (DPIV) ("analogous enzymatic effect") and (iia) alanyl aminopeptidase N (APN) as well as (iib) peptidases having an enzymatic effect analogous to alanyl aminopeptidase N (APN) ("analogous enzymatic effect") ("dual inhibitors"). The novel substances are also capable of separately inhibiting the enzymes (ia) dipeptidyl peptidase IV (DPIV) as well as (ib) peptidases having an enzymatic effect analogous to dipeptidyl peptidase IV (DPIV) or (iia) alanyl aminopeptidase N (APN) as well as (iib) peptidases having an enzymatic effect analogous to alanyl aminopeptidase N (APN) ("solitary inhibitors"). Furthermore, the invention relates to processes to prepare the novel dual inhibitors of DPIV and APN. The invention also relates to the afore-mentioned novel compounds for a use in the medical field. Moreover, the invention relates to a use of the afore-mentioned dual inhibitors for a prophylaxis and a therapy as well as for the manufacture of a medicament for a prophylaxis and a therapy, of autoimmune diseases, of diseases showing an excessive immune response and/or having an inflammatory genesis, of neuronal diseases and of cerebral damage, of tumor diseases, of skin diseases, of diabetes of the type I and of SARS.

The enzyme dipeptidyl peptidase IV (DPIV, CD26, EC 3.4.14.5) is a serine protease existing ubiquitously and catalyzing the hydrolysis of peptides specifically after proline and - to a lesser extent - after alanine or - with restrictions - after further amino acids like serine, threonine, valine and glycine at the second position of the N-terminus. Enzymes belonging to the gene family of enzymes having DPIV-analogous enzymatic effect are - *inter alia* - DP II, DP 8, DP 9 and FAP/seprase [T. Chen et al.: Adv. Exp. Med. Biol. 524, 79, 2003]. A substrate specifity analogous to DPIV was also found for attractin (mahagony protein) [J. S. Duke-Cohan et al.: J. Immunol. 156, 1714, 1996]. Said enzyme is also inhibited by DPIV inhibitors.

Belonging to the group of alanyl aminopeptidases (also existing ubiquitously) are the aminopeptidase N (APN, CD13, EC 3.4.11.2) predominantly appearing as a membrane protein of the type II, and the cytosolic soluble alanyl aminopeptidase (EC 3.4.11.14, puromycine-sensitive aminopeptidase, amino-peptidase PS, encephaline-degrading aminopeptidase). Alanyl aminopeptidases act in dependency on a metal, for example in dependency on zinc, and catalyze the hydrolysis of peptide bonds after the N-terminal amino acids of oligopeptides, in the case of APN with a preference of alanine at the N-terminus [A. J. Barrett et al.: Handbook of Proteolytic Enzymes, Academic Press 1998]. All inhibitors of aminopeptidase N also inhibit the cytosolic alanyl aminopeptidase, while specific inhibitors of the cytosolic aminopeptidase exist [M. Komodo et al.: Bioorg. and Med. Chem. 9, 121, 2001].

For both groups of enzymes, important biologic functions were proved in different cell systems. This is true for the immune system [U. Lendeckel et al.: Intern. J. Mol. Med. 4, 17, 1999; T. Kähne et al.: Intern. J. Mol. Med. 4, 3, 1999; I. De Meester et al.: Advanc. Exp. Med. Biol. 524, 3, 2002; International Patent Application No. WO 01/89,569; International Patent Application No. WO 02/053,170; International Patent Application No. PCT/EP 03/07,199]; the neuronal system [International Patent Application No. WO 02/053,169 and German Patent Application No. 103 37 074.9]; the fibroblasts [German Patent Application No. 103 30 842.3]; the keratinocytes [International Patent Application No. WO 02/053,170]; the sebaceous gland cells/sebocytes [International Patent Application No. PCT/EP 03/02,356]; tumors as well as for virus-caused infections as, for example corona virusses [D. P. Kontoyiannis et al.: Lancet 361, 1558, 2003].

The capability of DPIV of specifically inactivating the incretory hormones GIP and GLP led to the development of a new therapeutic concept for treating glucose metabolic disorders [D. M. Evans: Drugs 5, 577, 2002].

For both groups of enzymes, there are known different inhibitors [reviews are found in: D. M. Evans: Drugs 5, 577, 2002; and: M.-C. Fournie-Zaluski and B. P. Roques: in J. Langner and S. Ansorge: Ectopeptidases, Kluwer Academic/Plenum Publishers, p. 51, 2002].

The isolated inhibition of the alanyl aminopeptidases and of the dipeptidyl peptidase IV as well as the inhibition of enzymes having an analogous substrate specificity, in particular the combined inhibition of enzymes of both groups of enzymes, results into a strong inhibition of the DNA synthesis of immune cells and, hence, into a strong inhibition of the cell proliferation as well as into a change of the cytokine production, particularly into an induction of the immunosuppressive cytokine TGF-β1 [International Patent Application No. WO 01/89,569; International Patent Application No. WO 02/053,170] as well as into an inhibition of the generation and release of inflammatory cytokines of the type TH1, e. g. interleukine-2 (IL-2), and TH2, e. g. interleukine-4 (IL-4) [International Patent Application No. WO 02/053,170 and German Patent Application No. 101 02 392.8]. Inhibitors of alanyl aminopeptidase effect a strong induction of TGFβ1 at regulatory T-cells [International Patent Application No. PCT/EP 03/07,199] and an activation of the immunosuppressive phenotype of regulatory T-cells [German Patent Application No. 10 2006 703 942]. In the neuronal system, a decrease or retardation of acute and chronic cerebral damage processes was proved by an inhibition of both enzyme systems [International Patent Application No. WO 02/053,169 and German Patent Application No. 103 37 074.9]. Moreover, it was proved for fibroblasts [German Patent Application No. 103 30 842.3], keratinocytes [International Patent Application No. WO 02/053,170) and sebocytes [International Patent Application No. PCT/EP 03/02,356] that the combined inhibition of alanyl aminopeptidase N and DPIV effects an inhibition of the cell growth and a change of the cytokine production.

This results into the surprising fact that the alanyl aminopeptidases and the dipeptidyl peptidase IV as well as enzymes having an analogous enzymatic effect perform fundamental central biologic functions in different organs and cell systems, and that a combined inhibition of both groups of enzymes represents a new effective therapeutic principle for the treatment of various inflammatory and /or neurodegenerative diseases.

Chronic inflammatory diseases like autoimmune diseases (e. g. multiple sclerosis, rheumatoid arthritis, and psoriasis), allergies and allo-graft rejections are due to unwanted immune responses, particularly by the action of activated lymphocyte populations which leads to damaging cells and tissues.

For example, mainly T helper 1 ($TH_1$) and T helper 17 ($TH_{17}$) lymphocytes are involved in the pathogenic process in cases of autoimmune diseases, and T helper 2 ($TH_2$) lymphocytes are involved in the pathogenic process in cases of allergies like bronchial asthma, in combination with their selective cytokines.

Consequently, strategies of treating chronic inflammatory diseases are based in three main pharmacological interventions:

(1) reduction of T cell growth (proliferation);
(2) suppression of the production of inflammatory cytokines (like interferongamma, IL-17 and IL-4); and
(3) activation of so-called T regulatory cells (Treg) which are capable of suppressing helper and effector cells.

Well-known anti-inflammatory / immunosuppressive drugs are, for example, cyclosporine A and rapamycin. Both compounds exert their pharmacological effects by blocking T cell proliferation and DNA synthesis, respectively, as well as by suppressing an expression of several cytokine genes that are, normally, induced upon T cell activation [C.A. Janeway, P. Travers, M. Walport, M. Shlomchick: Immunobiology, Garland Publishing, New York (2001), pages 553 to 566].

Moreover, rapamycin is also capable of activating Treg cells (D. A. A. Vignali, L. W. Collison, C. J. Workman: How regulatory cells work, Nature Review Immunology 8, 523 - 532 (2008)].

In accepted animal models, the applicants could show in the meantime that, in particular, the combined administration of inhibitors of both groups of said peptidases results into an inhibition of the growth of different cell systems and into a suppression of an excessive immune response, of chronic inflammatory processes and of cerebral damages, also *in vivo* [International Patent Application No. WO 01/89,569]. The isolated administration of single known inhibitors results into a diminished effect.

The results reported formerly were obtained predominantly by means of known inhibitors of alanyl aminopeptidase N and dipeptidyl peptidase IV alone, being described in the literature and being - in part - commercially available but in particular by combinations of inhibitors of enzymes of both groups.

In the European patent application No. 06 829 004.8, novel, predominantly non-peptidic low molecular weight substances were reported, which may be employed as prodrugs and may act under physiological and pathological conditions as effective agents or a mixture of effective agents and which inhibit alanyl aminopeptidase N and enzymes having an analogous substrate specificity, and dipeptidyl peptidase IV and enzymes having an analogous substrate specificity as well, in a dual manner. The conversion of the prodrugs is conducted by a reduction of -S-S- or -Se-Se-bridges, preferably by cellular thiols (compounds bearing -SH- groups).

It was the object of the present invention to provide novel substances suitable as multifunctional dual inhibitors for the above two groups of aminopeptidases, i. e. (ia) dipeptidyl peptidase IV (DPIV) as well as (ib) peptidases having an enzymatic effect analogous to dipeptidyl peptidase IV (DPIV) ("analogous enzymatic effect") and (iia) alanyl aminopeptidase N (APN) as well as (iib) peptidases having an enzymatic effect analogous to alanyl aminopeptidase N (APN) ("analogous enzymatic effect") ("dual inhibitors") which should be suitable for a use in the medical field, e.g. should be capable of being useable for a prophylaxis and a therapy of autoimmune diseases, of diseases showing an excessive immune response and/or having an inflammatory genesis, of neuronal diseases and of cerebral damage, of tumor diseases, of skin diseases, of diabetes of the type I and of SARS.

Hence, the invention relates to novel substances which are capable of specifically inhibiting peptidases cleaving Ala-p-nitroanilide as well as peptidases cleaving Gly-Pro-p-nitroanilide ("dual inhibition") and, hence, combine the capability of a concerted inhibition of both groups of peptidases in one substance, only. Of course, the novel substances are capable, too, of inhibiting each singular group of peptidases, i. e. peptidases cleaving Ala-p-nitroanilide or peptidases cleaving Gly-Pro-p-nitroanilide ("solitary inhibition").

The multifunctional peptidase inhibitors as described herein are novel substances. They are capable of combating inflammation by suppressing a T cell growth (proliferation) which is combined with suppressing a production of inflammatory cytokines. Moreover, these new chemical compounds are also capable of activating Treg cells which fact makes these substances and agents comprising these substances powerful drug candidates for a treatment of inflammatory diseases.

Moreover, the invention relates to novel substances which may be used as such, but also may be used as starting materials for other substances, for a prophylaxis and a therapy of diseases having an excessive immune response

(autoimmune diseases, allergies and transplant rejections, sepsis), of other chronic-inflammatory diseases, including arteriosclerosis, neuronal diseases, and cerebral damage, skin diseases (*inter alia* acne and psoriasis), of tumor diseases and specific virus infections (*inter alia* SARS) as well as type I diabetes.

The invention relates to compounds of the general formula (1),

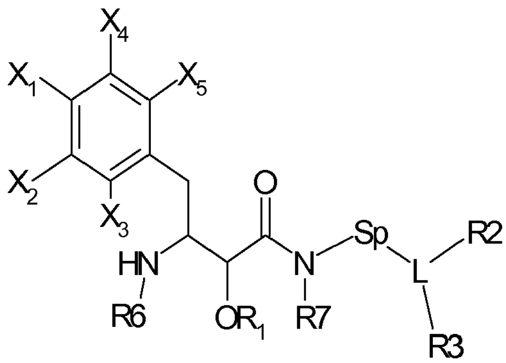

(1)

wherein

- $X_1$, $X_2$, $X_3$, $X_4$ and $X_5$ may be identical to or different from each other and are selected from the group consisting of -H, -OH, $-NO_2$, -halogens, $-NH_2$, $-OR^4$, $-NHR^4$, $-NR^4R^5$, $-CH_2NHR^4$, $-CH_2NR^4R^5$, -SH, - $SR^4$, $-CH_2(C=O)R^4$, -P(=O)$(OH)_2$, -P(=O)(OH)($OR^4$), -P(=O)($OR^4$)($OR^5$), -P(=O)(=O)(OH), -P(=O)(=O)($OR^4$), -P(=O)(=O)(H) and -P(=O)(=O)($R^4$), homocyclic and heterocyclic, aromatic and non-aromatic, condensed and non-condensed ring systems, in the case of heterocyclic moieties being allowed to have one, two or several heteroatoms selected from the group consisting of N, O, S, P, substituted with substituents $R^4$ and $R^5$; wherein $R^4$ and $R^5$ may be identical to or different from each other and are selected from the group consisting of -H, -OH, $-NH_2$, $-NO_2$, substituted and unsubstituted straight-chain or once- or multiple-branched aliphatic hydrocarbon, ester, amide, carbonate and carbamate residues having no, one or multiple double or triple carbon-carbon bonds and having from 1 to 29 carbon atoms which may bear O, S, NH or a secondary amino moiety at any chemically possible position of the chain between two chain carbon atoms, with the one or two sub-chains at the secondary amino group being built up according to the definition of the main chain described here; homoaromatic or heteroaromatic or non-aromatic homocyclic or heterocyclic condensed or non-condensed aliphatic hydrocarbon residues having 3 to 10 ring members, and, in the case of heterocyclic moieties, including one or several identical or different hetero atoms selected from O, N, S, and P and, in the case of non-aromatic cyclic systems, having no or one or several carbon-carbon or carbon-heteroatom double bonds or having no, one or several carbon-carbon triple bonds; said $R^4$ and $R^5$ residues optionally bearing one, two or more substituents independently selected from $X_1$, $X_2$, $X_2$, $X_4$ and $X_5$ or optionally bearing, at each possible position, one or more moieties selected from the group consisting of carbonyl, carbonic acid, carbonic acid ester, carbonic acid amide, carbonate and carbamate; with the proviso that substituents defined according to the definition of $R^4$ and $R^5$, which are allowed to occupy positions only that avoid direct -N-N-and -O-O- grouping; and with the further proviso that, if $R^4$ and $R^5$ are bound to the same carbon atom or hetero atom and the valence situation allows, the $R^4$ and $R^5$ substituents may be part of a spiro ring system and form a homocyclic or heterocyclic, condensed or uncondensed ring which is unsubstituted or is substituted by one, two or more substituents selected from the group consisting of $X_1$, $X_2$, $X_3$, $X_4$ and $X_5$;

- $R^1$, $R^2$, $R^6$ and $R^7$ may vary independently and represent residues as defined above for $R^4$ and $R^5$, or all permutatively possible pairs of the substituents $R^1$, $R^2$, $R^6$ and $R^7$ may form, together with the atom(s) of the basic structure (1) to which they are bound, a 5- to 14-membered heterocyclic aromatic (if chemically possible) or non-aromatic ring structure which may be condensed or non-condensed and unsubstituted or substituted with one or more substituent (s) $R^4$ as defined above;

- Sp represents an aliphatic hydrocarbon chain having 2 to 8 carbon atoms in the main chain and bearing no, one or several substituents $R^4$ defined as above, a non-aromatic homocyclic or heterocyclic or homoaromatic or heteroaromatic non-condensed or condensed ring system consisting of 3 to 10 ring atoms and bearing no, one or several substituents $R^4$ defined as above or bearing -O-, -S-, -NH- and $-NR^4$-substituents, wherein $R^4$ is defined as above;

- L represents $-CR^{13}$, >C=O or $>C=NR^{13}$, wherein $R^{13}$ represents a residue having the same meanings as $R^4$ above, with the proviso that, if L represents >C=O or $>C=NR^{13}$ (wherein $R^{13}$ is defined as $R^4$ above), $R^2$ is not existent, or L may be nitrogen or oxygen, respectively, provided that the bond with the respective part of the molecule causes no directly bound -N-N- or -O-O- units;

- $R^3$ represents one of the following substituents of (a), (b), (c) or (d):

(a)

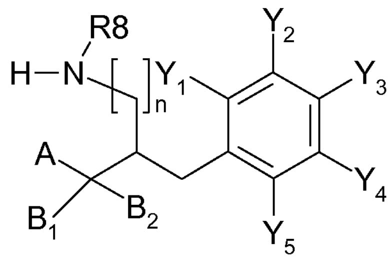

wherein

- A is a structural element directly bound to the substituent L and represents a single bond or a substituent selected from >C=O, $>C=NR^4$, or $>C=CR^4R^5$, an aliphatic straight or once or several times branched hydrocarbon chain having 1 to 6 carbon atoms, having none or one or several carbon-carbon double or triple bonds and being unsubstituted or substituted with one or several $R^4$ substituents, wherein $R^4$ and $R^5$ have the meaning as defined above, or A may be $-NR^4$, -O- or -S-, with the proviso that the bond between A and L forms no -N-N- or -O-O- bond, and n is an integer selected from 0, 1 and 2;

- $B_1$ and $B_2$ are identical to or different from each other and represent a residue selected from the group consisting of -H, $-CH_3$, -halogens, -OH, $-OR^9$, $-NH_2$, $-NHR^9$, $-NR^9R^{10}$ or all meanings of $R^4$ defined above, wherein $R^9$ and $R^{10}$ may be identical to or different from each other and may be selected from the group consisting of all substituents defined above as $R^4$; or $B_1$ and $B_2$ together may be part of or together form a 3-to 10-membered homocyclic or heterocyclic aromatic or non-aromatic saturated or once or several times unsaturated, non-condensed or condensed ring having none or one or several hetero atoms selected from >N-, -O-, -S- and >P<, which ring is unsubstituted or may be substituted with one or several substituent (s) selected from all substituents defined above as $R^4$;

- $R^8$ represents a substituent selected from the group consisting of all substituents represented by $R^4$ above or may be a hydrocarbon chain bridging to the above substituent A or to a carbon or hetero atom contained in the above substituent Sp, said hydrocarbon chain having 1 to 6 carbon atoms in a straight chain, having none or one or several carbon-carbon double or triple bond(s) and being unsubstituted or substituted with one or several $R^4$ substituents, wherein $R^4$ has the meaning defined above, or containing, within said straight hydrocarbon chain, one or several hetero atom(s) selected from the group consisting of -O-, -S-, >NH and $>NR^{12}$ wherein $R^{12}$ may have all meanings as $R^4$ defined above, or represents a homoaromatic or heteroaromatic ring or non-aromatic homocyclic or heterocyclic ring having none, one or multiple double or triple bond(s) and bearing no, one or multiple substituent(s) selected from all meanings of $R^4$; and

- $Y_1$, $Y_2$, $Y_3$, $Y_4$ and $Y_5$ may be identical to or different from each other and may be selected from substituents having the same meaning as the substituents $X_1$, $X_2$, $X_3$, $X_4$ and $X_5$; wherein Y-substituents having consecutive numbers may be may be bound via atoms selected from the group consisting of C, N, O, S or P being part of a condensed or non-condensed, homocyclic or heterocyclic, non-aromatic or homoaromatic or, provided that the chemical situation allows, heteroaromatic ring system having 3 to 10 ring members which may be non-substituted or substituted with one, two or several residues represented by $R^4$ and $R^5$ as defined above so that the phenyl ring is a part of a condensed system;

(b)

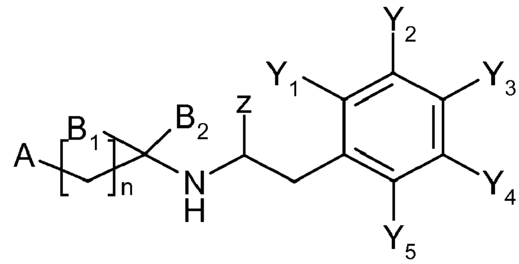

wherein A, $B_1$, $B_2$, and $Y_1$ to $Y_5$ may have the same meaning as the corresponding substituents of the above formula (a), n is an integer selected from the range of between 0 and 3, and Z represents -H, a residue having the meaning selected from all meanings of $R^4$ or may be a hydrocarbon chain selected from those meanings of hydrocarbon chains found above for $R^8$ and bridging to $B_1$, $B_2$, $R^2$ or to a carbon atom or hetero atom of Sp;
(c)

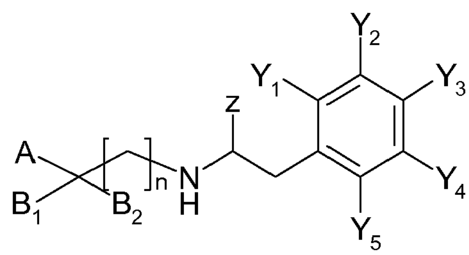

wherein A, $B_1$, $B_2$, $Y_1$ to $Y_5$ and Z may have the same meaning as the corresponding substituents of the formulae (a) and (b), n is an integer selected from the range of between 0 and 3; or
(d)

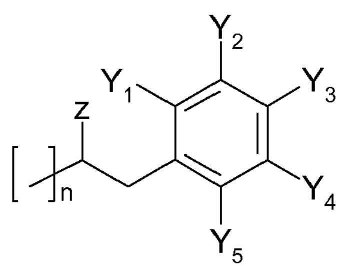

wherein $Y_1$ to $Y_5$ and Z may have the same meaning as the corresponding substituents of the formulae (a), (b) and (c), n is an integer selected from the range of between 0 and 6;

- and for the four representations of $R^3$, (a), (b), (c) and (d), bridgings connecting the structural elements A, $B_1$, $B_2$, $R^8$ and L are allowed between two or more of these elements so that, in the case of more than two moieties connected, bridged condensed and basket-like sub-structures can be formed, respectively; as bridging moieties, unsubstituted and, with substituents according to the definition of $R^4$ and $R^5$, substituted, continuous or interrupted with O, S and $NR^4$, straight and once or multiple branched carbon chains with none, one or several double and triple bond(s), respectively, are possible;

- or the salts thereof with organic and/or inorganic acids.

The chiral carbon atoms of the compounds of the general formula (1) may be in the S or in the R conformation.
Preferred embodiments of the compounds of the general formula (1) result from subclaims 2 to 3.
Compounds of the above general formula (1) may be synthesized by generally known synthetic methods of

the organic chemistry described below in detail exemplarily for single compounds or groups of compounds. Such synthetic methods are well known to a person skilled in organic syntheses. These methods are particularly known to result into compounds of the general formula (1) in high yields and in high purity suitable for medical applications, particularly for the administration to patients.

The invention also relates to the compounds of the general formula (1) mentioned above and described below in detail to be used in medicine.

Furthermore the invention relates to the compounds of the general formula (1) mentioned above and described below in detail, said compounds being inhibitor precursors.

Preferred embodiments result from subclaims 6 to 7.

Furthermore, the invention relates to the use of at least one compound of the general formula (1) mentioned above and described below in detail, said use being for the prophylaxis and therapy of diseases with exceeding immune response and inflammatory genesis including arteriosclerosis, neuronal diseases, cerebral damages, skin diseases, tumour diseases and virus-caused diseases, as well as type I diabetes and SARS.

The invention also relates to the use of at least one compound of the general formula (1) mentioned above and described below in detail, said use being for the preparation of a medicament for the prophylaxis and therapy of diseases with exceeding immune response and inflammatory genesis including arteriosclerosis, neuronal diseases, cerebral damages, skin diseases, tumour diseases and virus-caused diseases, as well as type I diabetes and SARS.

Preferred embodiments of the uses are claimed in claims 10 to 13.

Furthermore, the invention relates to a process to generate at least one dual inhibitor of dipeptidyl peptidase IV (DPIV) and of peptidases with analogous enzymatic effect, as well as of alanyl aminopeptidase N (APN) and of peptidases with analogous enzymatic effect, from at least one of the compounds of general formula (1) according to the above definition and to the following detailed description, wherein at least one compound of the general formula (1) is exposed to conditions as present in cells and tissues, for example is exposed to cellular enzymes, preferably is exposed to deacylases.

Preferred embodiments of the process result from claim 15.

The invention relates also to pharmaceutical or cosmetic preparations comprising at least one of the compounds of the general formula (1) according one of the claims 1 to 7 and according to the following detailed description, optionally in combination with one or more pharmaceutical or cosmetic acceptable carrier(s), auxiliary compound(s) and/or adjuvant (s).

According to the invention the new compounds have the general formula (1):

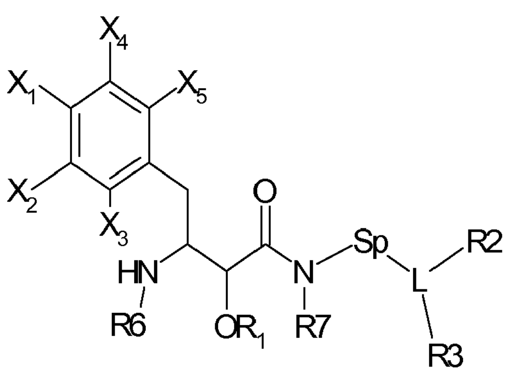

It was surprisingly found that the compounds of said formula themselves have inhibitory effects with regard to the enzymes mentioned below and moreover may be transformed under defined conditions to compounds, which are dual or solitary inhibitors of dipeptidyl peptidase IV (DPIV) and of peptidases with analogous enzymatic effect, and/or of alanyl aminopeptidase N (APN) and of peptidases with analogous enzymatic effect. Hence, compounds of the above general formula (1) may be precursors of such inhibitors.

The term "precursors" of compounds of the general formula (1), as used in the present description and claims means compounds of the general formula (1) bearing acyl residues at N- or O-atoms bearing the residues $R^1$, $R^6$ or $R^8$ (the latter being in the substituent (a) representing $R^3$), said acyl residues replacing -H groups; these acyl precursors of the "active" inhibitors (wherein "active inhibitors" means those compounds of the general formula (1) where the precursor acyl groups are bearing H groups) are converted to the "active" forms of the inhibitors by the action of cellular N-acylases or O-acylases.

The term "precursors" does not exclude that precursors *per se* are able, before being transformed into drugs with a defined pharmacological (for example inhibitory) effect, to develop a pharmacological effect (for example to inhibit

one or two of the two afore-mentioned enzymes). Conditions of the transformation of precursors into drugs in a mammal or in a human, respectively, can be of the type as they are regularly present in the physiological surroundings of a mammal, for example a human, or in the body of a mammal, for example in the body of a human, more particularly in a cell of a body of a mammal or human. Alternatively, such physiological conditions might only be present under defined conditions in a mammal, as for example in a human, more particularly in a cell, as for example a defined physiological condition as present in, for example, a disease pattern, or they can be induced or be invoked by external influences, for example (without restriction) by medical influences, to the organism of a mammal, as for example the organism of a human being, particularly exemplified by a cell in a human body.

Using the term "dipeptidyl peptidase IV" (DPIV, CD26, EC 3.4.14.5) in the following description and in the claims, the serine protease is recognized which catalyzes the hydrolysis of peptide bonds specifically after proline and to a lesser degree alanine and - with restrictions - after other amino acids like serine, threonine, valine, and glycine respectively at the second position of the N-terminus of peptides.

Using the term "peptidases with dipeptidyl peptidase IV-analogous enzymatic effect", peptidases are recognized in the present description and in the claims which catalyze the hydrolysis of peptides specifically after proline or alanine at the second position of the N-terminus. Examples for peptidases with dipeptidyl peptidase IV analogous enzymatic effect are, without restricting the invention to those, DP II, DP 8, DP 9 and FAP/seprase [T. Chen et al., a. a. O.] and attractin (mahagony protein) [J. S. Duke-Cohan et al., a. a. O.].

Using the term "alanyl aminopeptidase N" (APN, CD13, EC 3.4.11.2) in the present description and in the claims, the protease is recognized, which operates metal- (zinc-) dependent and catalyzes the hydrolysis of peptide bonds specifically after N-terminal amino acids of peptides and preferably alanine at the N-terminus.

Using the term "peptidases with alanyl aminopeptidase N-analogous enzymatic effect", peptidases are recognized in the present description and in the claims, which - like APN - operate metal-dependent and catalyze the hydrolysis of peptide bonds specifically after N-terminal amino acids of peptides and preferably after alanine at the N-terminus. Examples of peptidases with alanyl aminopeptidase N-analogous enzymatic effect are, without restricting the invention thereto, the cytosolic soluble alanyl aminopeptidase (EC 3.4.11.14), puromycine-sensitive aminopeptidase, aminopeptidase PS, encephaline-degrading amino-peptidase [A. J. Barret et al., a. a. O.].

Using the term "inhibitor" in the present description and in the claims, such compounds of natural origin, synthetic origin or natural origin with synthetic modification are recognized which have a regulatory, particularly inhibitory effect on an enzyme or a group of enzymes. The regulatory effect can be based on most different effects without limiting the afore-mentioned definition of the term "inhibitor". Preferred inhibitors according to the invention are inhibitors with an inhibitory effect on enzymes, more preferred on groups of enzymes, for example inhibitors with inhibitory effect on dipeptidyl peptidase IV (DPIV) and on peptidases with dipeptidyl peptidase IV analogous enzymatic effect or inhibitors with inhibitory effect on alanyl aminopeptidase N (APN), respectively, and on peptidases with alanyl aminopeptidase N analogous enzymatic effect, as defined above.

Using the term "solitary inhibitor" in the present description and in the claims, a substance or chemical compound is recognized which is capable of inhibiting the enzymes (ia) dipeptidyl peptidase IV (DPIV) as well as (ib) peptidases having an enzymatic effect analogous to dipeptidyl peptidase IV (DPIV) ("peptidases having an analogous enzymatic effect") or the enzymes (iia) alanyl aminopeptidase N (APN) as well as (iib) peptidases having an enzymatic effect analogous to alanyl aminopeptidase N (APN) ("peptidases having an analogous enzymatic effect"). As mentioned above, also while addressing well-known literature in this technical field, the enzymes (ia) dipeptidyl peptidase IV (DPIV) and (ib) peptidases having an analogous enzymatic effect may be inhibited by identical inhibitors. In the same way, the enzymes (iia) alanyl aminopeptidase N (APN) and (iib) peptidases having an analogous enzymatic effect may be inhibited by identical inhibitors.

Using the term "dual inhibitor" in the present description and in the claims, a substance or chemical compound is recognized which is capable of inhibiting the enzymes (ia) dipeptidyl peptidase IV (DPIV) as well as (ib) peptidases having an enzymatic effect analogous to dipeptidyl peptidase IV (DPIV) ("peptidases having an analogous enzymatic effect") and (iia) alanyl aminopeptidase N (APN) as well as (iib) peptidases having an enzymatic effect analogous to alanyl aminopeptidase N (APN) ("peptidases having an analogous enzymatic effect"). Such an inhibitory action is sometimes referred to as "concerted inhibition" in the present description and in the claims.

Using the term "concerted inhibiting" in the present description and in the claims, an inhibiting action is recognized which inhibiting action is effected by the compounds of the present invention, which compounds are multifunctional dual inhibitors for the above two groups of peptidases, i. e. dual inhibitors for (ia) dipeptidyl peptidase IV (DPIV) as well as (ib) peptidases having an enzymatic effect analogous to dipeptidyl peptidase IV (DPIV) ("analogous enzymatic effect") and for (iia) alanyl aminopeptidase N (APN) as well as (iib) peptidases having an enzymatic effect analogous to alanyl aminopeptidase N (APN) ("analogous enzymatic effect"). Compounds having such capability of concerted inhibition of the above enzymes are called "dual inhibitors" in the present description and claims.

In the compounds of the afore-mentioned general formula (1), the substituents $X_1$, $X_2$, $X_3$, $X_4$ and $X_5$, i. e. the substituents at the terminal benzyl residue, may be identical to each other or may be different from each other. In

accordance with the invention, they are selected from the group consisting of -H, -OH, $-NO_2$, -halogens, $-NH_2$, $-OR^4$, $-NHR^4$, $-NR^4R^5$, $-CH_2NHR^4$, $-CH_2NR^4R^5$, -SH, $-SR^4$, $-CH_2(C=O)R^4$, $-P(=O)(OH)_2$, $-P(=O)(OH)(_{OR}{}^4)$, $-P(=O)(OR^4)(OR^5)$, -P(=O)(=O)(OH), $-P(=O)(=O)(OR^4)$, -P(=O)(=O)(H) and $-P(=O)(=O)(R^4)$, homocyclic and heterocyclic, aromatic and non-aromatic, condensed and non-condensed ring systems, in the case of heterocyclic moieties being allowed to have one, two or several heteroatoms selected from the group consisting of N, O, S, P, substituted with substituents $R^4$ and $R^5$. In the afore-mentioned groups, $R_4$ and $R_5$ may be identical to each other or may be different from each other and are selected from the group consisting of -H, -OH, $-NH_2$, $-NO_2$, substituted and unsubstituted straight-chain or once- or multiple-branched aliphatic hydrocarbon, ester, amide, carbonate and carbamate residues having no, one or multiple double or triple carbon-carbon bonds and having from 1 to 29 carbon atoms which may bear O, S, NH or a secondary amino moiety at any chemically possible position of the chain between two chain carbon atoms, with the one or two sub-chains at the secondary amino group being built up according to the definition of the main chain described here; homoaromatic or heteroaromatic or non-aromatic homocyclic or heterocyclic condensed or non-condensed aliphatic hydrocarbon residues having 3 to 10 ring members, and, in the case of heterocyclic moieties, including one or several identical or different hetero atoms selected from O, N, S, and P and, in the case of non-aromatic cyclic systems, having no or one or several carbon-carbon or carbon-heteroatom double bonds or having no, one or several carbon-carbon triple bonds; said $R^4$ and $R^5$ residues optionally bearing one, two or more substituents independently selected from $X_1$, $X_2$, $X_2$, $X_4$ and $X_5$ or optionally bearing, at each possible position, one or more moieties selected from the group consisting of carbonyl, carbonic acid, carbonic acid ester, carbonic acid amide, carbonate and carbamate; with the proviso that substituents defined according to the definition of $R^4$ and $R^5$, which are allowed to occupy positions only that avoid direct -N-N- and -O-O- grouping; and with the further proviso that, if $R^4$ and $R^5$ are bound to the same carbon atom or hetero atom and the valence situation allows, the $R^4$ and $R^5$ substituents may be part of a spiro ring system and form a homocyclic or heterocyclic, condensed or non-condensed ring which is unsubstituted or is substituted by one, two or more substituents selected from the group consisting of $X_1$, $X_2$, $X_3$, $X_4$ and $X_5$.

In accordance with preferred embodiments of the invention, the halogen residues which are represented by $X_1$, $X_2$, $X_3$, $X_4$ and $X_5$ may be selected from halogens as -F, -Cl, -Br and -I. A selection from -Cl and -Br is even more preferred.

Another preferred embodiment of the invention relates to compounds of the general formula (1) wherein $X_1$, $X_2$, $X_3$, $X_4$ and $X_5$ are identical to each other or different from each other, more preferred wherein either all residues $X_1$, $X_2$, $X_3$, $X_4$ and $X_5$ are identical (for example, without restriction, all residues $X_1$, $X_2$, $X_3$, $X_4$ and $X_5$ represent -H) or at least three of them, more preferable four of them are identical (for example, without restriction, represent H), while at least one (preferred) or even two are different from the others and represent a substituent selected from halogens (preferably, without restriction, -Cl and/or -Br), -OH, -C(=O)OH, $-NH_2$ or $-NHR^4$, wherein $R^4$ has the meanings defined above. In alternative, also preferred, embodiments of the invention, a substituent $R^4$ may be bound directly to the terminal benzyl residue. In further preferred embodiments, $R^4$ directly bound to the terminal benzyl residue may be selected from alkyl or alkenyl residues, for example (without restriction) -methyl or -ethyl, may be selected from homoaromatic residues having five or six ring members which, for example (without restriction), may be -phenyl (which may optionally substituted), or may be selected from heteroaromatic residues having five or six ring members and having one or two hetero atoms selected from N, O, S or P, for example (without restriction) residues as -thiophenyl or -pyridinyl or -pyrimidinyl.

In accordance with the present invention, if one or several or all of the residues $X_1$, $X_2$, $X_3$, $X_4$ and $X_5$ represent a residue having the general formula $R^4$ or contain residues bearing one or two of the residues $R^4$ and $R^5$ (e. g. $-NR^4R^5$, $-OR^4$ or $-P(=O)(OR_4)(OR_5)$), each of the residues $R^4$ and $R^5$ may be either identical to the other or may be different from the other.

In accordance with preferred embodiments of the invention, ring systems serving as substituents (e. g. serving as substituents $R^4$ or $R^5$) may be systems consisting of one ring, for example, without restriction, consisting of one phenyl ring (as an example of a homoaromatic 6-membered ring) or consisting of one piperidinyl ring or of one tetrahydrofuranyl ring (as examples of a heterocyclic 6-membered ring and a heterocyclic 5-membered ring), or may be systems consisting of several (optionally condensed) rings (for example, without restriction, consisting of an indolyl ring system (as an example of a benzocondensed heteroaromatic ring system)).

In accordance with the invention, $R^1$, $R^2$, $R^6$ and $R^7$ may vary independently and represent residues as defined above for $R^4$ and $R^5$. Alternatively, all permutatively possible pairs of the substituents $R^1$, $R^2$, $R^6$ and $R^7$ may form, together with the atom(s) of the basic structure (1) to which they are bound, a 5- to 14-membered heterocyclic aromatic (if chemically possible) or non-aromatic ring structure which may be condensed or non-condensed and unsubstituted or substituted with one or more substituent(s) $R^4$ as defined above.

In preferred embodiments of the compounds of the general formula (1), $R^1$ may be a $C_1$- to $C_{29}$-, preferably a $C_6$- to $C_{18}$-, more preferably a $C_8$- to $C_{16}$-alkyl residue or -alkanoyl residue. Preferred are straight chain (i. e. n-alkyl or n-alkanoyl) residues, which may bear one or several residues $R^4$ as substituents, wherein $R^4$ is defined as above.

In another preferred embodiment, $R^1$ may represent an unsubstituted or substituted unit having the sub-formula $-C_{1-14}$-XX-$C_{1-14}$- (wherein XX represents -O-, -S-, -NH- or $-NR^{11}$-, wherein $R^{11}$ may have the same meanings as they

are defined above for $R^4$). There may be present one or several independent groups -XX- in the main chain of $R^1$.

In another preferred embodiment of the compounds of the general formula (1), $R^1$ may be connected to $R^4$, for all described meanings, via atoms selected from C, O, N, S and P.

In further preferred embodiments of the compounds of the general formula (1), $R^1$ may be a 3-membered to 10-membered non-aromatic homocyclic or heterocyclic ring or ring system or a 5-membered to 8-membered homoaromatic or heteroaromatic ring or ring system which may be unsubstituted or optionally be substituted by one or several substituents $R^4$. The rings may be single rings, non-condensed plural rings or condensed plural rings.

In accordance with the invention, Sp represents an aliphatic hydrocarbon chain having 2 to 8 carbon atoms in the main chain and bearing none, one or several substituents $R^4$ defined as above, a homoaromatic or heteroaromatic 5- to 8-membered ring bearing none, one or several substituents $R^4$ defined as above; a homocyclic or heterocyclic aliphatic 3- to 8-membered ring having none, one or several carbon-carbon or carbon-heteroatom double or carbon-carbon triple bonds and bearing none, one or several substituents $R^4$ defined as above. Examples of the hydrocarbon chain are methylene, bismethylene (ethylene), trismethylene (propylene) and butylene groups which are preferably straight in their chain. Sp may also represent branched alkylene groups or cycloalkylene groups. Examples of homoaromatic ring systems of Sp are phenylene groups, while examples of homocyclic rings are cyclopentylene and cyclohexylene groups. The saturated or unsaturated homocyclic or heterocyclic rings or homoaromatic or heteroaromatic rings for which Sp stands may be single rings or may be non-condensed rings or may be condensed ring systems.

In preferred embodiments of the invention, Sp may be as defined above, with the proviso that Sp is part of the ring formed by a common hydrocarbon chain of the connected residues $R^2$ and $R^7$ (see the above definition of the residues $R^2$ and $R^7$). As a non-restricting example, $R^2$ and $R^7$ may form together a unit of $-CH_2-CH_2-$, L may simultaneously be N, and Sp represents $-CH_2-CH_2-$ resulting into a piperazinyl residue for the combination of the residues $R^2$, $R^7$, L and Sp.

In accordance with the invention, L represents $-CR^{13}$, >C=O, >C=NH or $>C=NR^{13}$, wherein $R^{13}$ represents -H or a residue having the same meanings as $R^4$ above, with the proviso that, if L represents >C=O, >C=NH or $>C=NR^{13}$, $R^2$ is not existent. Alternatively, L may be nitrogen, provided that the bond with the respective part of the molecule causes no directly bound -N-N- or -O-O-units.

In accordance with the invention, $R^3$ has the meaning of one of the formulae (a), (b), (c) or (d):

(a)

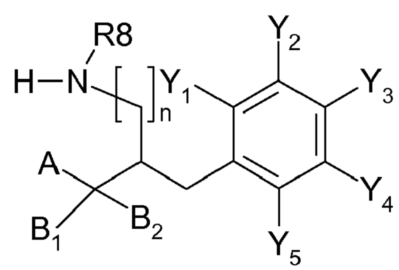

wherein

- A is a structural element directly bound to the substituent L and represents a single bond or a substituent selected from >C=O, $>C=NR^4$, or $>C=CR^4R^5$, an aliphatic once or several times branched hydrocarbon chain having 1 to 6 carbon atoms, having none or one or several carbon-carbon double or triple bonds and being unsubstituted or substituted with one or several $R^4$ substituents, wherein $R^4$ and $R^5$ have the meaning defined above, or A may be $-NR^4$, -O- or -S- with the proviso that the bond between A and L forms no -N-N- or -O-O- bond, and n is an integer selected from the group consisting of 0, 1 and 2;

- $B_1$ and $B_2$ are identical to or different from each other and represent a residue selected from the group consisting of -H, $-CH_3$, -halogens, -OH, $-OR^9$, $-NH_2$, $-NHR^9$, $-NR^9R^{10}$ or all meanings of $R^4$ defined as above, wherein $R^9$ and $R^{10}$ may be identical to or different from each other and may be selected from the group consisting of all substituents defined above as $R^4$; or $B_1$ and $B_2$ together may be part of or form together a 3-to 10-membered homocyclic or heterocyclic aromatic or non-aromatic saturated or once or several times unsaturated non-condensed or condensed ring having none or one or several hetero atom(s) selected from >N-, -O-, -S- and >P<, which ring is unsubstituted or may be substituted with one or several substituent(s) selected from all substituents

defined above as $R^4$;

- $R^8$ represents a substituent selected from the group consisting of all substituents represented by $R^4$ above or may be a hydrocarbon chain bridging to the above substituent A or to a carbon or hetero atom contained in the above substituent Sp, said hydrocarbon chain having 1 to 6 carbon atoms in a straight chain, having none or one or several carbon-carbon double or triple bond(s) and being unsubstituted or substituted with one or several $R^4$ substituents, wherein $R^4$ has the meaning defined above, or containing, within said straight hydrocarbon chain, one or several hetero atom(s) selected from the group consisting of -O-, -S-, >NH and >$NR^{12}$ wherein $R^{12}$ may have all meanings as $R^4$ defined above, or represents a homoaromatic or heteroaromatic ring or non-aromatic homocyclic or heterocyclic ring having none, one or multiple double or triple bond(s) and bearing no, one or multiple substituent(s) selected from all meanings of $R^4$; and

- $Y_{13}$ $Y_2$, $Y_3$, $Y_4$ and $Y_5$ may be identical to or different from each other and may be selected from substituents having the same meaning as the substituents $X_1$, $X_2$, $X_3$, $X_4$ and $X_5$; wherein Y-substituents having consecutive numbers may be may be bound via atoms selected from the group consisting of C, N, O, S or P being part of a condensed or non-condensed, homocyclic or heterocyclic, non-aromatic or homoaromatic or, provided that the chemical situation allows, heteroaromatic ring system having 3 to 10 ring members which may be non-substituted or substituted with one, two or several residues represented by $R^4$ and $R^5$ as defined above so that the phenyl ring is a part of a condensed system;

(b)

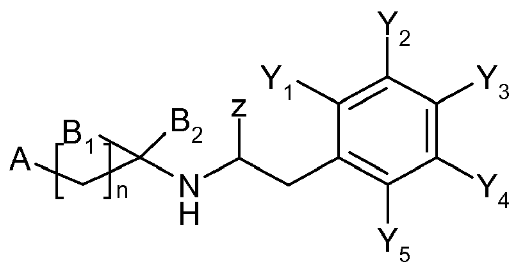

wherein

- A, $B_1$, $B_2$, and $Y_1$ to $Y_5$ may have the same meaning as the corresponding substituents of the above formula (a), n is an integer selected from the range of between 0 and 3, and Z represents -H, a residue having the meaning selected from all meanings of $R^4$ or may be a hydrocarbon chain selected from those meanings of hydrocarbon chains found above for $R^8$ and bridging to $B_1$, $B_2$, $R^2$ or to a carbon atom or hetero atom of Sp;

(c)

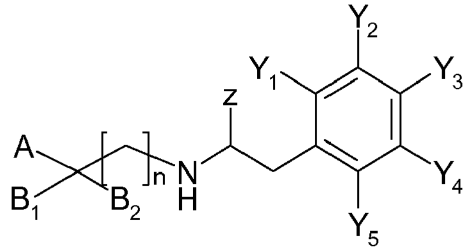

wherein A, $B_1$, $B_2$, $Y_1$ to $Y_5$ and Z may have the same meaning as the corresponding substituents of the formulae (a) and (b), n is an integer selected from the range of between 0 and 3; or

(d)

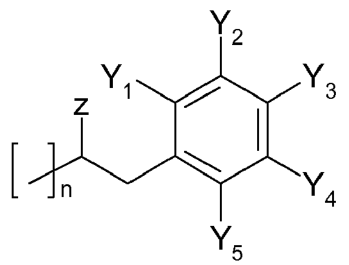

wherein

$Y_1$ to $Y_5$ and Z may have the same meaning as the corresponding substituents of the formulae (a), (b) and (c), n is an integer selected from the range of between 0 and 6;

- for the four representations of $R^3$, (a), (b), (c) and (d), bridgings connecting the structural elements A, $B_1$, $B_2$, $R^8$ and L are allowed between two or more of these elements, so that, in the case of more than two moieties connected, bridged condensed and basket-like sub-structures can be formed, respectively; as bridging moieties, unsubstituted and, with substituents according to the definition of $R^4$ and $R^5$, substituted, contunuous or interrupted with O, S and $NR^4$, straight and once or multiple branched carbon chains with none, one or several double and triple bond(s), respectively, are possible.

In the residue $R^3$ having the above formula (a), the hydrocarbon chain of A is a straight chain and is exemplified by methylene, bis-methylene (ethylene), trismethylene (propylene), n-butylene, penta-methylene (n-pentylene) and hexamethylene (hexylene) groups. Said hydrocarbon chain may comprise none, one or plural double and/or triple bond(s). A may also be a single bond (in which case the residue (a) is directly, i. e. via said single bond, bound to the unit L-$R^2$. A may also be >C=O or -C(=O)NH- or may be -NH- in case that L represents either >C=O or >C=NH.

In preferred embodiments of the compounds of the general formula (1), the residues $B_1$ and $B_2$ in the residue (a) of $R_3$ may represent -F, -Cl, -Br or -I as the halogens.

The corresponding definitions are also applicable for compounds of the general formula (1) according to the invention, wherein $R^3$ represents one of the general formulae (b), (c) and (d).

All heterocyclic or heteroaromatic compounds present in the compounds of the formula (1) may contain one or plural of the heteroatoms N, O, S or P; said heteroatoms may have the any of the oxidation stages commonly allowed for the respective atom.

All substituents $R^1$ to $R^{13}$, L, A, $B_1$, $B_2$, XX, $X^1$ to $X^4$, $Y^1$ to $Y^5$ and Z may vary independently of each other, as far as possible in accordance with the rules of chemical binding (Erlenmeyer Rule). The selection of all structural elements presented above and furtheron in the specification and claims avoids direct -N-N- and -O-O-bonds.

Wherever condensed ring systems are allowed in the compounds of the general formula (1), the degree of condensation may be from 1 to 3. Homoaromatic, heteroaromatic, non-aromatic homocyclic and heterocyclic systems may be combined as desired.

Using the term "alkyl residue" in the present description and in the claims, a monovalent straight-chained ("unbranched") or branched residue made of carbon atoms linked by single bonds to each other with hydrogen atoms bound to the carbon atoms is recognized. Hence, alkyl-residues are according to the present invention saturated monovalent hydrocarboned residues. Preferably the alkyl-residues in the compounds of the general formula (1) comprise 1 to 18 carbon atoms and are thus selected from the residues methyl, ethyl, n-propyl, i-propyl and the numerous different straight-chained and branched isomers of the residues butyl, pentyl, hexyl, heptyl, octyl, nonyl, decyl, undecyl, dodecyl, tridecyl, tetradecyl, pentadecyl, hexadecyl, heptadecyl and octadecyl. Particularly preferred are straight-chained and branched alkyl-residues having 1 to 12 carbon atoms; straight-chained and branched alkyl-residues having 1 to 6 carbon atoms are even more preferred. Most preferred are residues methyl, ethyl, n-propyl, i-propyl, n-butyl, i-butyl, sec-butyl and tert-butyl.

Accordingly in the present description and in the claims the terms "alkenyl-residue" and "alkinyl-residue" monovalent straight-chained ("unbranched") or branched residues of carbon atoms linked to each other by single bonds and at least one double bond or triple bond, respectively at an arbitrary but defined position in the molecule with hydrogen atoms bound to the remaining bonds of the carbon atoms are recognized having at least 2 carbon atoms and up to 18 carbon atoms. Such residues are for example preferably vinyl-residues or allyl-residues; however, carbon-carbon multiple bond-containing residues are not restricted to said residues.

In the present descriptions and in the claims the term "alkylene-residue" is recognized to be a divalent straight-chained ("unbranched") or branched residue of carbon atoms linked to each other by single bonds with hydrogen atoms

bound to the carbon atoms. Hence, alkylene-residues are according to the present invention saturated divalent hydrocarbon-residues. Preferably alkylene-residues in the compounds of the general formulae (1) and (2) comprise 1 to 18 carbon atoms and are therefore selected from the residues methylene, ethylene, n-propylene, 2,2-propylene, 1,2-propylene and numerous different straight-chained and branched isomers of the residues butylene, pentylene, hexylene, heptylene, octylene, nonylene, decylene, undecylene, dodecylene, tridecylene, tetradecylene, pentadecylene, hexadecylene, heptadecylene and octadecylene. Particularly preferred are straight-chained and branched alkylene residues having 1 to 12 carbon atoms and straight-chained and branched alkylene residues having 1 to 6 carbon atoms are more preferred. Most preferred are the residues methylene, ethylene, n-propylene, 2,2-propylene, 1,2-propylene and the numerous different butylene-position isomers.

In the alkyl-residues and/or the alkylene-residues which, according to the invention, may be part of the compounds of the general formula (1), the chains of carbon atoms might be interrupted by O-atoms, N-atoms or S-atoms; Hence, in the course of the chain, there might exist instead of one or more $-CH_2$-group(s) one or more group(s) of the group -O-, -NH- and -S-, whereas usually not two of the groups -O-, -NH- and/or -S- follow each other in the chain. Said one or more group(s) -O-, -NH- or -S- can be inserted at arbitrary positions in the molecule. Preferably, if a hetero group of that ilk is present, a group of that ilk is present in the molecule.

Straight-chained as well as branched alkyl- or alkylene-residues might be substituted, according to the invention in the compounds of the general formula (1) in a further embodiment, with one or more substituents, preferably with one substituent. Even more preferably, the substituent is selected from the residues selected from $R^4$. The substituent(s) can be located at arbitrary positions of the backbone, formed by the carbon atoms and can preferably, without restricting the invention hereto, be selected from the group consisting of halogen atoms like fluorine, chlorine, bromine and iodine, particularly preferred chlorine and bromine, alkyl groups having 1 to 6 carbon atoms each, particularly preferred alkyl groups having 1 to 4 carbon atoms, alkoxy groups having 1 to 6 carbon atoms in the alkyl residue, preferably having 1 to 3 carbon atoms in the alkyl residue, unsubstituted or - with one or two alkyl residue(s) containing 1 to 6 carbon atoms independently from each other, preferably 1 to 3 carbon atoms - substituted amino groups, carbonyl groups and carboxyl groups. The latter can also be present in form of salts or esters with alcohols having 1 to 6 carbon atoms in the alkyl residue; hence the term "carboxyl-groups" includes groups of the general structure $-COO^- M^+$ (with M = monovalent metal atom such as an alkali metal-atom or an accordant equivalent of a polyvalent metal atom such as half an equivalent of a divalent metal atom like an alkaline earth metal atom) or of the general structure $-COOR_x$ (with $R_x$ = alkyl groups having 1 to 6 carbon atoms). The substituting alkyl groups are selected from alkyl groups mentioned above in detail and are particularly preferred methyl groups, ethyl groups, n-propyl groups, i-propyl groups, n-butyl groups, i-butyl groups, sec-butyl groups or tert-butyl groups.

Alkoxy groups are alkyl groups in the above-defined sense which are bound via an O-atom to the backbone formed by the carbon atoms. They are preferably selected from the group consisting of the residues methoxy, ethoxy, n-propoxy, i-propoxy; n-butoxy, i-butoxy, sec-butoxy and tert-butoxy.

Amino groups are groups of the general structure $-NR_x R_y$ in which the residues $R_x$ and $R_y$ might independently from each other designate: hydrogen or alkyl groups (according to the afore-mentioned definition) having 1 to 6 carbon atoms particularly preferred having 1 to 3 carbon atoms in which the residues $R_x$ and $R_y$ might be identical to or different from each other. Such amino groups being particularly preferred as substituents are $-NH_2$, $-NH(CH_3)$, $-N(CH_3)_2$, $-NH(C_2H_5)$ and $-N(C_2H_5)_2$. The term "amino groups" contains also groups of the above-defined structure which are present as quaternary ammonium ions, either because of salt formation with organic acids or inorganic acids (e.g. residues of the structure $R_x R_y R_z N^+ Q^-$, in which $R_x$, $R_y$ and $R_z$ might be identical or different, preferably identical, and $R_x$ and $R_y$ might have the above-defined meanings, and at least one of the residues is hydrogen from the quaternation with the organic or inorganic acid, and Q is an acid residue from an acid of the organic or inorganic acid) or because of salt formation with suitable quaternation reagents which are known to a person skilled in the field such as (without restriction hereto) with alkyl halogenids.

In the present description and in the claims the term "cycloalkyl" is used for unsubstituted or substituted monovalent residues of $-CH_2$ groups linked to each other in form of closed rings. According to the invention said rings might contain preferably 3 to 8 atoms forming the ring and might either contain exclusively carbon atoms or contain one or more hetero atom(s) which is/are selected from -O-, -S- and $-NR_x-$ in which $R_x$ is hydrogen or a alkyl residue (as defined above) having 1 to 6 carbon atoms. In case hetero atoms are inserted in the rings said hetero atoms can be - in case of more than one hetero atom - identical or different. Preferably in case hetero atoms are present one hetero atom is inserted into the ring. Particularly preferred among purely carbocyclic rings are the residues cyclopentyl, cyclopentenyl, cyclopentadienyl, cyclohexyl, cyclohexenyl, cyclohexadienyl, cycloheptyl, cycloheptenyl, cycloheptadienyl and cycloheptatrienyl. Examples for heteroatom-containing cycloalkyl-residues, which are often referred to as heterocycloalkyl residues in further embodiments of the invention, are the residues tetrahydrofuranyl, pyrrolidinyl, pyrazolidinyl, imidazolidinyl, piperidinyl, piperazinyl and morpholinyl.

Possible substituents at the carbocyclic or heterocyclic cyloalkyl residues might be preferably, without restricting the invention hereto, selected from the afore-mentioned group of substituents for linear alkyl-groups. Particularly preferred

substituents for cycloalkyl-groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy and -tert-butoxy, $-NH_2$, $-NH(CH_3)$, $-N(CH_3)_2$, $-NH(C_2H_5)$ and $-N(C_2H_5)_2$, -carbonyl and -carboxyl.

In the present description and in the claims the term "cycloalkylene" is used for unsubstituted or substituted divalent residues of $-CH_2$ groups linked to closed rings. According to the invention these can preferably contain three to eight atoms in the ring and can consist either exclusively of carbon atoms or contain one or more hetero atom(s) which is/are selected from -O-, -S- and $-NR_x-$, in which $R_x$ is hydrogen or a alkyl-residue (as defined above) having 1 to 6 carbon atoms. Particularly preferred among the purely carbocyclic rings are the residues cyclopentylen, cyclopentenylen, cyclopentadienylen, cyclohexylen, cyclohexenylen, cyclohexadienylen, cycloheptylen, cycloheptenylen, cycloheptadienylen and cycloheptatrienylen. Also, the heterocyclic groups defined above with regard to the cycloalkyl residues can appear in compounds of the general formula (1) as groups "B" in form of divalent residues and particularly preferred are such cyclic divalent residues in which one group -O- or $-NR_x-$ is inserted into the ring. In those cases, both valences are localized at arbitrary C atoms in the ring. Preferably one hetero atom or two hetero atom(s) is/are inserted into the ring and in particularly preferred embodiments of such groups the divalent residues are derived from tetrahydrofuran, pyrrolidin, pyrazolidin, imidazolidin, piperidin, piperazin and morpholin.

Possible substituents at these carbocyclic or heterocyclic cycloalkylene residues can be preferably, without restricting the invention hereto, selected from the afore-mentioned group of substituents for linear alkyl-groups. Particularly preferred substituents for cycloalkylene-groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy and- tert-butoxy, $-NH_2$, $-NH(CH_3)$, $-N(CH_3)_2$, $-NH(C_2H_5)$ and $-N(C_2H_5)_2$, -carbonyl and -carboxyl.

Using the term "aryl residue" in the present description and in the claims, a monovalent hydrocarbon residue is recognized, which is derived from a cyclic molecule with aromatic character ($4n + 2$ $\pi$-electrons delocalized in ring-shaped orbitals) which might be unsubstituted or substituted. The ring structure of such an aryl residue can be a five-, six- or seven-membered ring structure with one ring or a structure formed by two or more ("annelated") rings bound to each other where the annelated rings have identical or different numbers of ring members, particularly of C-atoms. In case of systems consisting of at least two rings condensated to each other, benzo-condensated rings are particularly preferred, i.e. a ring system in which at least one of the rings is an aromatic six-membered ring exclusively containing C-atoms (e. g. a phenyl ring). Typical but not limiting examples of aryl rings are cyclopentadienyl-residues ($C_5H_5^-$) (being a five-membered ring), phenyl-residues (being a six-membered ring), cycloheptatrienyl-residues ($C_7H_7^+$) (being an seven-membered ring) naphthyl-residues (being a ring system comprising two annelated six-membered rings) as well as monovalent residues being derived from anthracen and phenanthren (being three annelated six-membered rings). According to the invention most preferred aryl-residues are phenyl- and naphthyl-residues.

Possible substituents of carbocyclic aryl-residues can be selected preferably from the groups of substituents mentioned above for linear alkyl-groups, without restricting the invention to these substituents. Particularly preferred substituents for aryl-groups are substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy and -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituent(s) of this group, which might be identical to or different from each other, can be bound to one aryl residue according to the present invention. The substituted position(s) at the aryl ring (system) can be chosen arbitrarily.

A comparable definition as in case of the aryl residues applies to the present description and the claims with regard to the definition of the term "arylene residue": In this regard, a divalent residue is recognized the elementary composition of which, the selection of which and the substituent(s) of which are comparable to the afore-mentioned definitions of the aryl residues, with the exception that it is a divalent residue the insertion of which can be carried out at two arbitrary carbon atoms.

In the present description and in the claims, by the term "heteroaryl residue" an aryl residue is recognized (in accordance with the afore-mentioned definition) the ring structure of which contains one or more hetero atom(s) preferably from the group O, N or S, without losing the aromatic character of the molecule. The ring structure of such a heteroaryl residue may either be a five-membered, a six-membered or a seven-membered ring structure with one ring or may be a structure formed by two or more ("annelated") rings bound to each other, wherein the annelated rings might have an identical or a different number of ring members. The hetero atom(s) can occur in one ring alone or in more than one ring of the ring system.

The heteroaryl residues preferably consist of one or two rings. In case of systems consisting of more than one ring, e. g. two rings condensed to each other, benzo-condensed rings are especially preferred, i.e. ring systems in which at least one of the rings is an aromatic carbocyclic (i.e. containing only C atoms) six-membered ring. Particularly preferred heteroaryl residues are selected from furanyl, thiophenyl, pyridyl, indolyl, cumaronyl, thionaphthenyl, chinolinyl (benzopyridyl), chinazolinyl (bezopyrimidinyl) and chinoxylinyl (benzopyrazinyl). Heteroaryl residues can be unsubstituted or substituted according to the invention. Possible substituents at these heteroaryl residues can be preferably selected from the afore-mentioned group of substituents for linear alkyl groups without restricting the invention to these substituents. Particularly preferred substituents for heteroaryl-groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy, -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituents of that group, which might be identical to or different from each other, might be bound to one heteroaryl residue according to the present invention. The substituted position(s) at the heteroaryl ring (-system) can be selected arbitrarily.

A comparable definition as in the case of the heteroaryl residues applies to the present description and the claims with regard to the definition of the term "heteroarylene residue": In this regard a divalent residue is recognized the general composition of which and the selection of which and the substituents of which are comparable to the afore-mentioned definition of "heteroaryl residues", with the exception that it is a divalent residue the insertion of which can be carried out at two arbitrary carbon atoms of the ring or the ring system, respectively, or at a nitrogen atom as well.

In the context of the present description and in the claims the terms "aralkyl residue", "heteroarylalkyl residue", "heterocycloalkyl residue", "arylamidoalkyl residue" and heteroarylamidoalkyl residue", mean alkyl residues (or - more specifically - alkylene residues) according to the afore-mentioned general and specific definition which are substituted at one of their bonds with an aryl-residue (according to the afore-mentioned general and specific definition), heteroaryl residue (according to the afore-mentioned general and specific definition), heterocyclyl residue (according to the afore-mentioned general and specific definition of the cycloalkyl residues substituted with hetero atoms), arylamido residues (according to the following general and specific definition) or heteroarylamido residues (according to the following general and specific definition). These residues can be unsubstituted or substituted.

In preferred embodiments of the invention aralkyl residues are residues of that group, in which the aryl residue is a phenyl residue, substituted phenyl residue, naphthyl residue or substituted naphthyl residue and the alkyl(ene) group is straight-chained or branched and may have 1 to 6 carbon atoms. In a very particular and advantageous way, the residues benzyl, phenethyl, naphthylmethyl and naphthylethyl can be used as aralkyl residues, of which benzyl residues are particularly preferred.

Possible substituents at the aryl groups of the aralkyl residues can be preferably selected from the afore-mentioned group of substituents for linear alkyl groups without restricting the invention to those substituents. Particularly preferred substituents for aryl groups of the aralkyl residues are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy, -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One ore more substituents of that group which might be identical or different from each other can be bound to one aryl group of an aralkyl residue according to the present invention. The substituted position(s) at the aryl ring (-system) can be chosen arbitrarily.

In preferred embodiments of the invention the heteroalkyl residues are such residues in which the heteroaryl residue of the heteroarylalkyl residue according to the invention is substituted and the alkylene group is straight-chained or branched and may have 1 to 6 carbon atoms. The ring structure of such a heteroaryl residue can be a ring structure with one ring or a structure formed by two or more than two ("annelated") rings bound to each other wherein the annelated rings might have an identical or different number of ring members. The hetero atom(s) can occur in one or more ring(s) of the ring system. The heteroaryl residues of the heteroarylalkyl residue consist preferably of one or two rings. In case of heteroarylalkyl systems composed of at least two rings condensated to each other, benzo-condensated rings are especially preferred, i.e. ring systems in which at least one of the rings is an aromatic carbocyclic six-membered ring. Particularly preferred heteroaralalkyl residues are selected from furanylmethyl and -ethyl, thiophenylmethyl and -ethyl, pyridylmethyl and -ethyl, indolylmethyl and -ethyl, cumaronylmethyl and -ethyl, thionaphthenylmethyl and -ethyl, chinolinyl-(bezopyridyl-)methyl and -ethyl, chinazolinyl-(benzopyrimidinyl-) and chinoxylinyl-(benzopyrazinyl-)methyl and -ethyl.

Possible substituents at these heteroaryl-groups of heteroarylalkyl-residues can be preferably selected from the afore-mentioned group of substituents for linear alkyl groups without restricting the invention to thereto. Particularly preferred substituents for heteroaryl groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituent(s) of that group which can be identical to or different from each other can be bound to a heteroarylalkyl residue according to the present invention. The substituted position(s) at the heteroaryl ring (-system) can be chosen arbitrarily.

In preferred embodiments of the invention heterocycloalkyl residues are cycloalkyl residues according to the afore-mentioned general and specific definition, which contain one or more hetero atom(s) which is/are selected from -O--S- and $-NR_x-$, in which $R_x$ is hydrogen or an alkyl residue having 1 to 6 carbon atoms (as defined above) and the alkyl(ene) groups of the heterocycloalkyl residues are straight-chained or branched and may have 1 to 6 carbon atoms. In case of at least two hetero atoms inserted into the ring(s), these can be identical or different. Preferably one hetero atom is incorporated in the ring. Preferred examples for hetero atoms containing cycloalkyl residues which are also referred to as heterocycloalkyl residues are, in further embodiments of the invention, the residues tetrahydrofuranyl, pyrrolinidyl, pyrazolidinyl, imidazolidinyl, piperidinyl, piperazinyl and morpholinyl.

Possible substituents at these heterocycloalkyl residues can preferably be selected from the afore-mentioned group of substituents for linear alkyl groups, without restricting the invention to those substituents. Particularly preferred substituents for heteroaryl groups are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy; -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituent(s) of that group, which might be identical to or different from each other, can be bound to one heterocycloalkyl residue according to the present invention. The substituted position(s) at the heterocacloalkyl ring (-system) can be chosen arbitrarily.

Using the terms "arylamidoalkyl-residue" and "heteroarylamidoalkyl-residue" in the present description and in the claims, alkyl residues (more precisely: alkylene residues) according to the afore-mentioned general and specific definition are recognized which are substituted at one of their bonds by an arylamido residue or heteroarylamido residue of the general formula $Ar-NR_x-C(=O)-$ or the general formula $Ar-C(=O)-NR_x-$ in which $R_x$ is hydrogen or an alkyl having 1 to 6 carbon atoms and Ar is an arbitrary aryl residue or heteroaryl residue according to the afore-mentioned general or specific definition. These aryl or heteroaryl residues can be unsubstituted or substituted. Preferred examples for an arylamidoalkyl residue - without restricting the invention - are 2-, 3- or 4-benzoic acid-amino-n-butyl residues or 2-nitro-3-, -4-, -5- or -6-benzoic acid-amido-n-butyl residues; preferred but not limiting examples for heteroarylamidoalkyl residues are 2-, 4-, 5- or 6-pyridin-3-carbonic acid-amido-n-butyl residues.

Possible substituents at these arylamidoalkyl residues and heteroarylamidoalkyl residues can preferably be selected from the afore-mentioned group of substituents for linear alkyl groups, without restricting the invention to those substituents. Particularly preferred substituents for aryl groups or heteroaryl groups of the arylamidoalkyl residues and heteroarylamidoalkyl residues are the substituents -Cl, -Br, -methyl, -ethyl, -n-propyl, -i-propyl, -n-butyl, -i-butyl, -sec-butyl or -tert-butyl, -methoxy, -ethoxy, -n-propoxy, -i-propoxy, -n-butoxy, -i-butoxy, -sec-butoxy, -tert-butoxy, $-NH_2$, $-NH(CH_3)$, $-N(CH_3)_2$, $-NH(C_2H_5)$ and $-N(C_2H_5)_2$, -carbonyl and -carboxyl. One or more substituent(s) of that group which can be identical to or different from each other can be bound to an aryl or heteroaryl group of the arylamidoalkyl residues or heteroarylamidoalkyl residues according to the present invention. The substituted position(s) at the aromatic ring (system) can be chosen arbitrarily.

A comparable definition as for the aralkyl residues, heteroarylalkyl residues, heterocycloalkyl residues, arylamidoalkyl residues and heteroarylamidoalkyl residues applies in the context of the present description and the claims with regard to the definition of the terms "aralkylene residue", "heteroarylalkylene residue", "heterocycloalkylene residue", "arylamidoalkylene residue" and "heteroarylamidoalkylene residue": These are understood to be divalent residues which general composition and the selection thereof and the substituent(s) thereof are comparable to the afore-mentioned definition of "aralkyl residue", "heteroarylalkyl residue", "heterocycloalkyl residue", "arylamidoalkyl residue" and "heteroarylamidoalky residue", with the exception that it is in either case a divalent residue the insertion of which can be carried out at two arbitrary carbon atoms of the ring or the ring system of the alkylene group respectively, or also at a nitrogen atom of the heteroaryl or heterocyclyl ring system.

According to the invention, the compounds of the general formula (1) are present in form of neutral molecules and are according to the invention used as neutral molecules. Alternatively, the compounds of the general formula (1) can also be present in form of their acid addition salts with inorganic and/or organic acids. Because of the presence of basic atoms (mostly of alkaline nitrogen atoms) in the molecule, such acid addition salts are formed by the addition of one or more molecules of H-acid compounds (Brönstedt acids), preferably one molecule of an H-acid compound, and provide an improved solubility of the molecules on polar media like for example in water. The latter characteristic is of particular impact for such compounds which develop pharmacological effect.

In preferred embodiments of the invention, acid addition salts are salts of pharmaceutically acceptable acids and are advantageously chosen (but without limiting the present invention) from the group consisting of hydrochlorides, trifluoroacetates, tartrates, succinates, formiates and/or citrates of the compounds of the general formula (1).

There were prepared, in particularly preferred embodiments of the invention, a number of exemplary (non-restricting) compounds of the general formula (1)

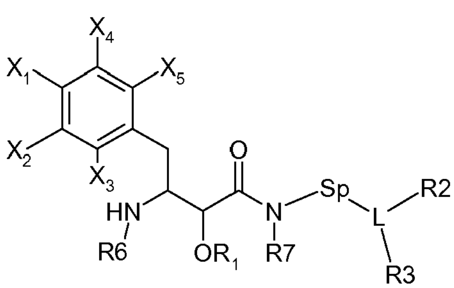

which are shown and summarized in the subsequent Table 1.

**Table 1: Examples for specific compounds of the general formula (1)**

| Structure | DPIV-$IC_{50}$ / µM | APN-$IC_{50}$ / µM |
|---|---|---|
| 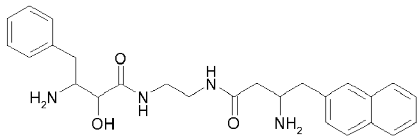 | 28.0 | 1.5 |
| 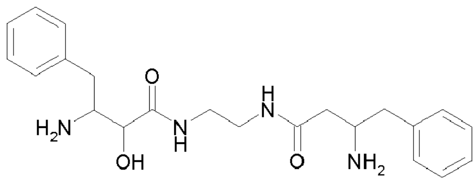 | 0.8 | 2.9 |
| 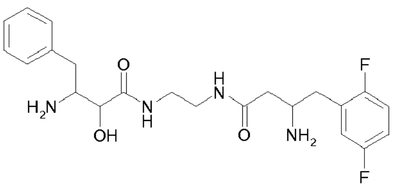 | 0.16 | 5.6 |
| 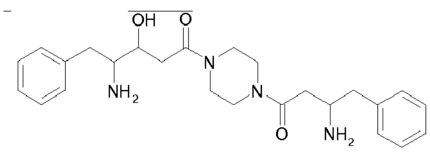 | 0.9 | >50 |
| 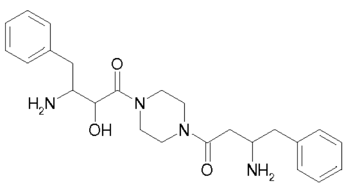 | 0.8 | 27.0 |
| 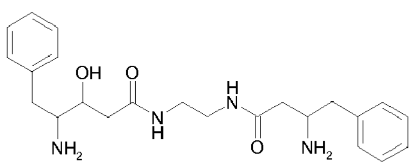 | 1.0 | >50 |
| 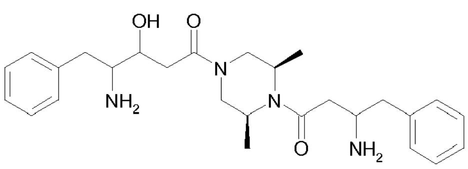 | 0.28 | 22.0 |
| 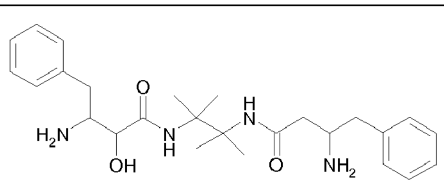 | 13.8 | 16.7 |

(continued)

| Structure | DPIV-$IC_{50}$ / μM | APN-$IC_{50}$ / μM |
|---|---|---|
| 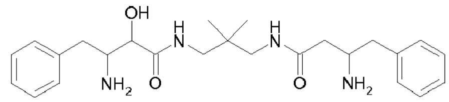 | 2.0 | 0.18 |
| 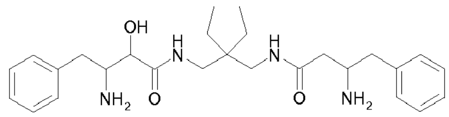 | 2.6 | 2.8 |
| 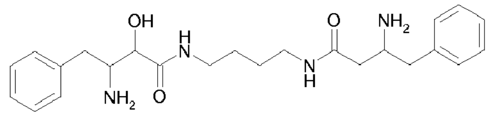 | 2.7 | 0.18 |
| 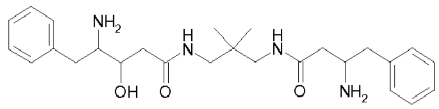 | 2.2 | >25 |
| 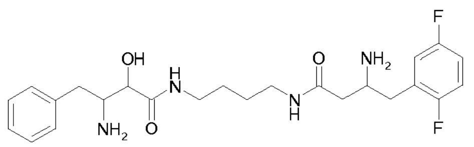 | 0.08 | 0.14 |
| 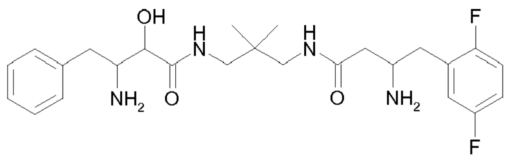 | 0.10 | 0.06 |
| 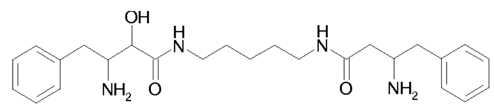 | 2.67 | 0.12 |
| 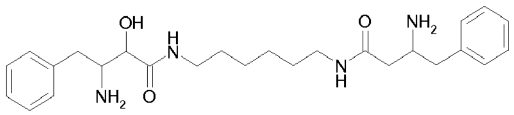 | 1.20 | 0.41 |
| 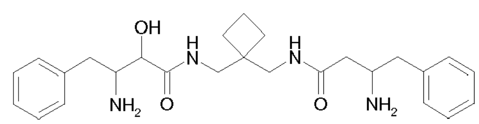 | 1.67 | 0.191 |
| 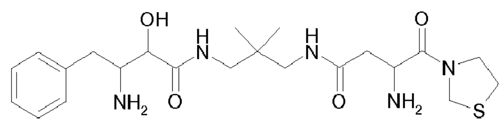 | 2.12 | 2.00 |
| 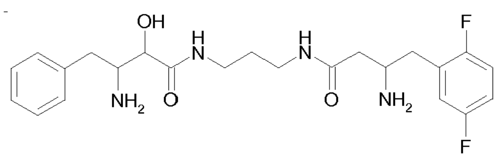 | 0.5 | 0.02 |

(continued)

| Structure | DPIV-$IC_{50}$ / μM | APN-$IC_{50}$ / μM |
|---|---|---|
| 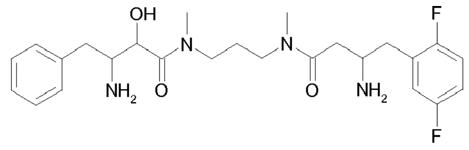 | 0.178 | 8.5 |
| 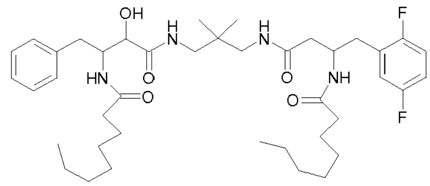 | > 50 | > 50 |
| 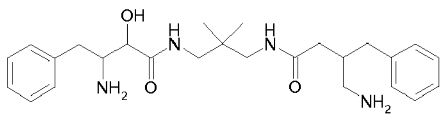 | > 50 | 0.15 |
| 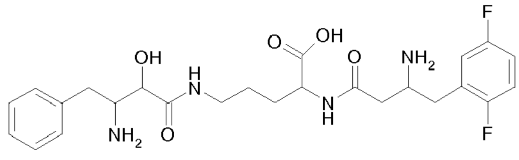 | 0.2 | 6.2 |
| 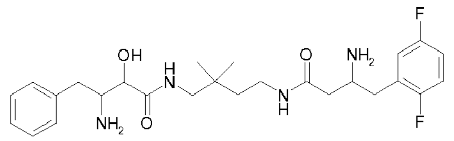 | 0.1 | > 50 |
| 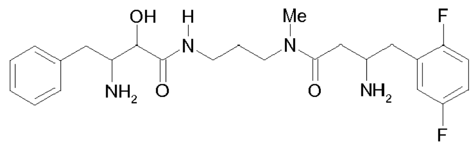 | 0.072 | 0.7 |
| 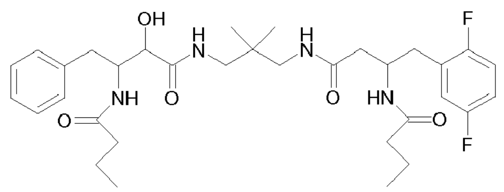 | > 50 | > 50 |
| 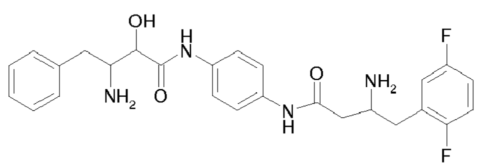 | 2.5 | 0.02 |
| 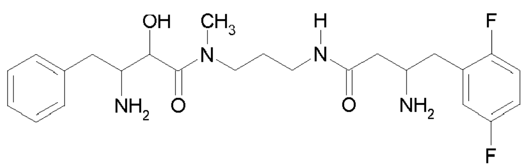 | 0.2 | 15.4 |

(continued)

| Structure | DPIV-$IC_{50}$ / $\mu M$ | APN-$IC_{50}$ / $\mu M$ |
|---|---|---|
| 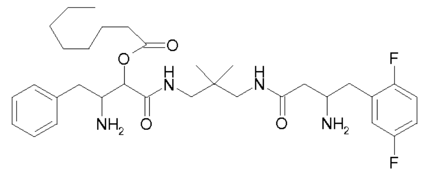 | 0.3 | 0.2 |
| 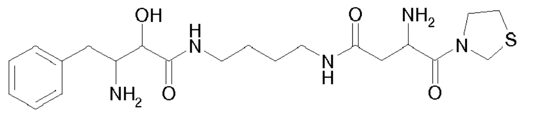 | 1.7 | > 50 |
| 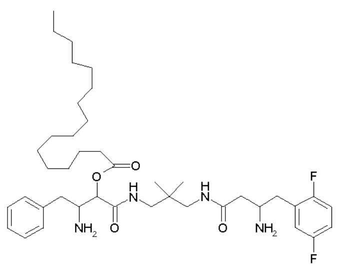 | 0.06 | 0.03 |
| 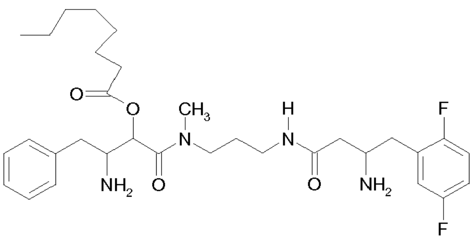 | 0.02 | >50 |
| 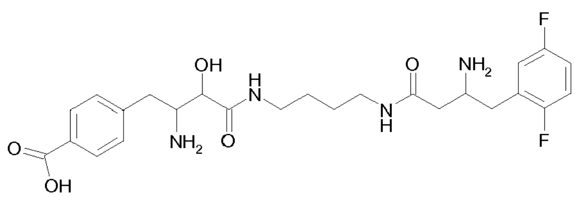 | 0.47 | >50 |
| 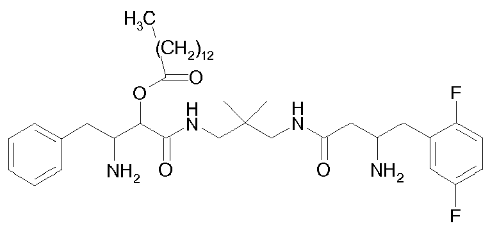 | 1.0 | 21.0 |
| 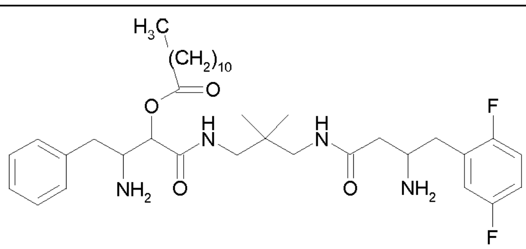 | 0.6 | 18.0 |

(continued)

| Structure | DPIV-$IC_{50}$ / μM | APN-$IC_{50}$ / μM |
|---|---|---|
| 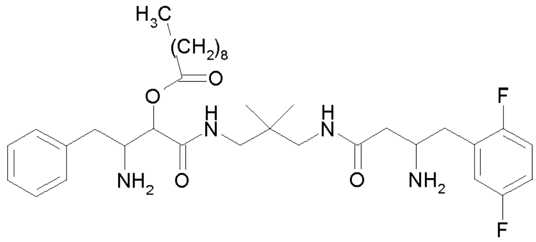 | 0.2 | >50 |
| 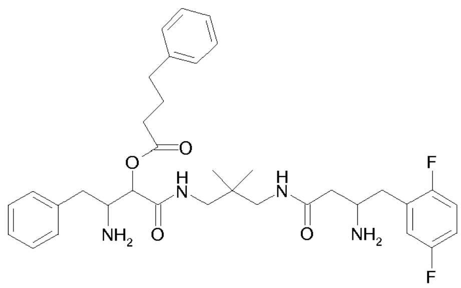 | 0.3 | 15.0 |
| 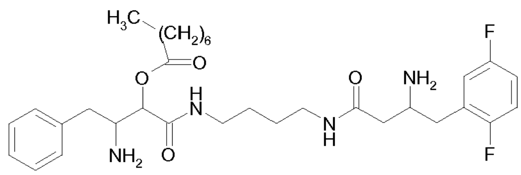 | 0.12 | >50 |
| 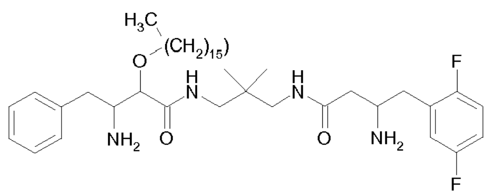 | 25.0 | >50 |
| 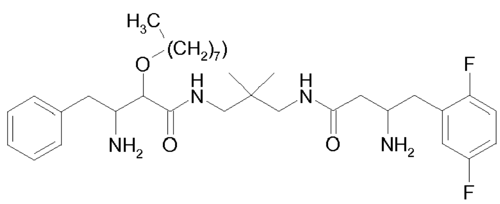 | 14.0 | >50 |
| 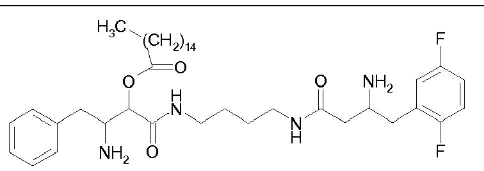 | 8.0 | 25.0 |
| 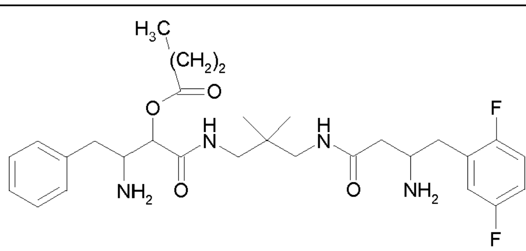 | 0.08 | 25.0 |

(continued)

| Structure | DPIV-$IC_{50}$ / μM | APN-$IC_{50}$ / μM |
|---|---|---|
| 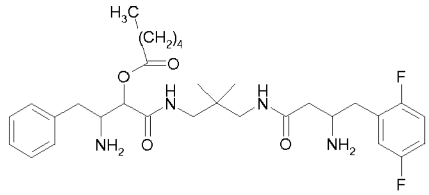 | 0.03 | 1.9 |
| 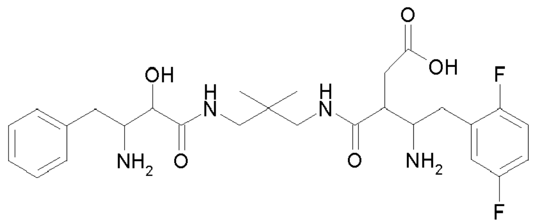 | 3.7 | 1.4 |
| 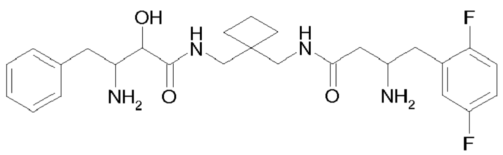 | 0.1 | 0.07 |
| 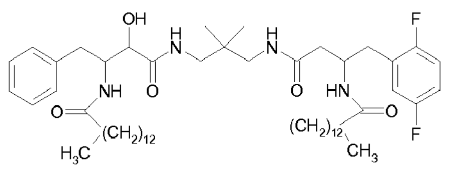 | 5.5 | 32.0 |
| 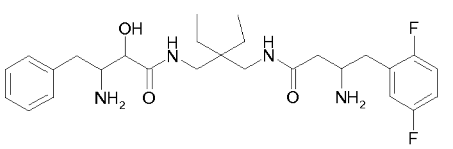 | 0.1 | 0.3 |
| 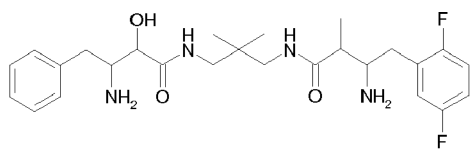 | 0.45 | 0.87 |
| 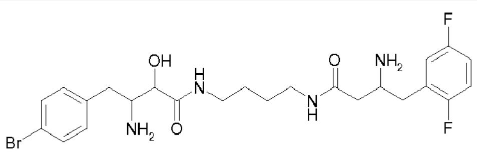 | 0.1 | 0.06 |
| 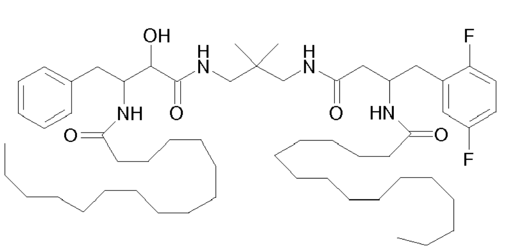 | >50 | >50 |

(continued)

| Structure | DPIV-$IC_{50}$ / $\mu M$ | APN-$IC_{50}$ / $\mu M$ |
|---|---|---|
| 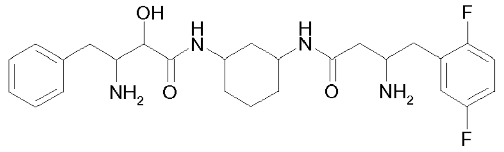 | 0.12 | 0.03 |
| 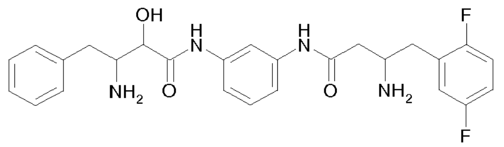 | 0.5 | 0.35 |
| 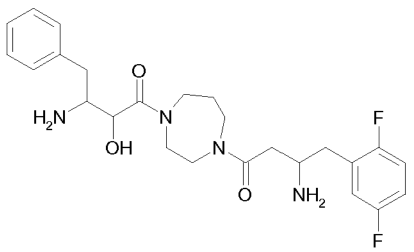 | 0.07 | 10.01 |
| 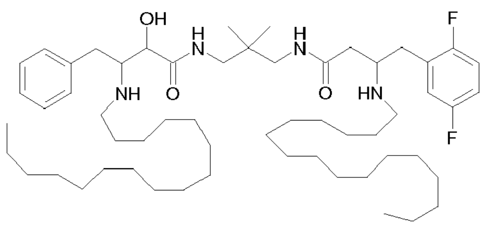 | >50 | >50 |
| 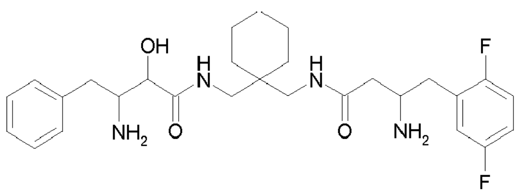 | 0.19 | 0.85 |
| 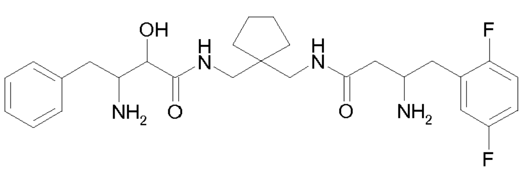 | 0.16 | 0.08 |
| 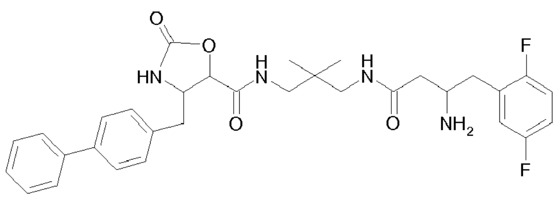 | 40 | 1.9 |

(continued)

| Structure | DPIV-$IC_{50}$ / $\mu$M | APN-$IC_{50}$ / $\mu$M |
|---|---|---|
| 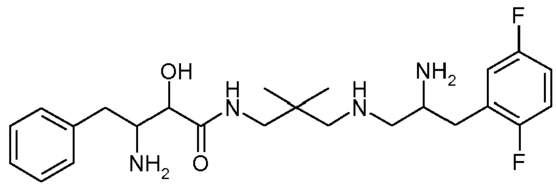 | 30 | 0.33 |
| 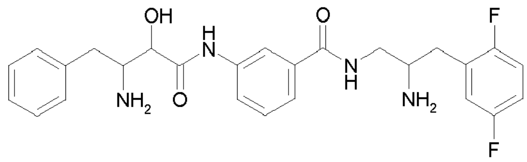 | 1.7 | 0.009 |
| 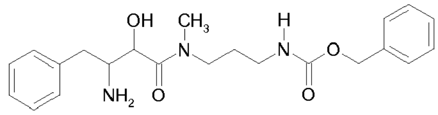 | > 50 | 11.5 |
| 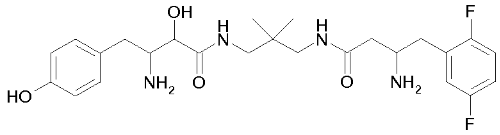 | 4.6 | >50 |
| 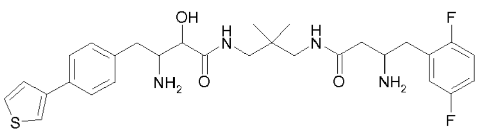 | 0.1 | 0.001 |
| 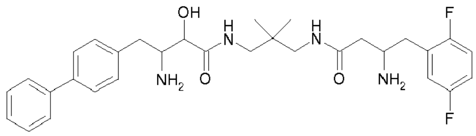 | 0.1 | 0.346 |
| 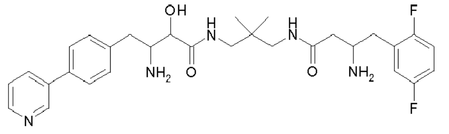 | 0.096 | 0.0008 |
| 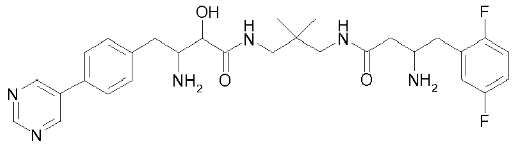 | 0.1 | 0.01 |
| 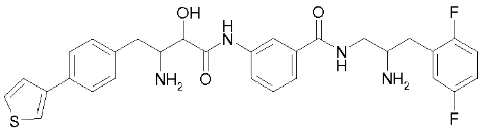 | 0.46 | 0.0005 |

(continued)

| Structure | DPIV-$IC_{50}$ / $\mu M$ | APN-$IC_{50}$ / $\mu M$ |
|---|---|---|
| 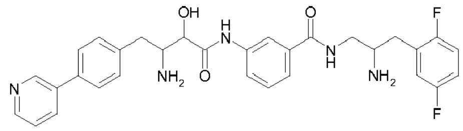 | 0.775 | 0.0008 |

## Measurement of DPIV activity

The inhibition of DPIV activity was measured by using purified, recombinant human DPIV (final enzyme concentration approx. 1 nM). The assay was performed in 0.05 M TRIS/HCl buffer pH 7.5, supplemented with 0.05 % Triton (v/v), 0.05 % BSA (w/v), 2 mM $MgCl_2$.

The enzymatic activity of DPIV was assessed by the hydrolysis of the fluorogenic substrate bis-(L-alanyl-prolyl)-Rhodamin-110 (bis-trifluoracetate, abbreviation $(Ala\text{-}Pro)_2$-R110). The final substrate concentration was 0.5 $\mu M$.

The assay was performed in white microtitre plates for fluorescence measurements. The test items, substrate and enzyme were diluted in assay buffer. The highest test item concentrations used were 25 $\mu M$. For the calculation of $IC_{50}$ values, at least 16 log2-dilutions of each test item were analyzed. As controls, the DPIV activity in the absence of test items as well as the spontaneous hydrolysis of the substrate was determined.

The release of the fluorescent hydrolysis product Rhodamine 110 was measured at an excitation wavelength of 485 nm and an emission wavelength of 530 nm by using a microtitre fluorescence reader immediately after substrate addition as well as after 30, 60 and 120 min.

## Measurement of APN activity

Inhibitory effects of test items on the enzymatic activity of human recombinant Aminopeptidase N (final concentration approx. 5 nM) were assessed by using the fluorogenic substrate bis-(L-alanyl)-Rhodamine-110 (bis-trifluoracetate, abbreviation $(Ala)_2$-R110) at a final concentration of 0.5 $\mu M$. The assay was performed in 0.05 M TRIS/HCl buffer pH 7.5, supplemented with 0.05 % Triton (v/v), 0.05 % BSA (w/v), 2 mM $MgCl_2$.

The assay was performed in white microtitre plates for fluorescence measurements.

For the calculation of $IC_{50}$ values, at least 16 log2-dilutions of each test item - starting at a concentration of 25 $\mu M$ - were analyzed. As controls, $(Ala)_2$-R110 hydrolyzing activity in the absence of any test item as well as the spontaneous substrate hydrolysis was determined.

The release of the fluorescent hydrolysis product Rhodamine 110 was measured at an excitation wavelength of 485 nm and an emission wavelength of 530 nm by using a microtitre fluorescence reader immediately after substrate addition as well as after 30, 60 and 120 min.

## Synthesis of inhibitors

Methods for synthesizing the compounds of the above general formula (1) follow usual routes of synthesis or organic compounds. A skilled person confronted with the task of synthesizing specific compounds of the general formula (1) can apply, based on his/her knowledge of synthesis routes for organic compounds, usual procedures which are well known and documented in the literature and usual handbooks of organic synthesis. Starting compounds for such syntheses are readily available commercially.

Synthesis routes resulting into specific compounds of the general formula (1), specifically those depicted in the above Table 1 are explained in detail below in the experimental part.

According to the invention the compounds of the general formula (1) are obtained during their synthesis in form of acid addition salts according to one of the processes described in detail below, particularly when their precise characterization and subsequent use in an aqueous milieu is to be carried out. Preferably, acid addition salts with certain physiologically (i.e. pharmacologically and/or cosmetically) acceptable inorganic or organic acids are prepared for the characterization and for the subsequent use of the compounds of the general formula (1). In preferred embodiments of the invention, salts of pharmaceutically acceptable acids from the group consisting of hydrochlorides, trifluoroacetates, tartrates, succinates, formiates and/or citrates of the compounds of the general formula (1) are prepared as acid addition salts.

The compounds mentioned above in general and in detail (referring to formula (1)) which can be prepared for example using one of the previously mentioned processes without restricting the preparation of the compounds to one of the said processes can be used for numerous purposes.

Surprisingly it was found by the invention that the compounds can be used in the field of medicine. Particularly it was found that the new compounds according to the present invention themselves are inhibitors of the dipeptidyl peptidase IV or of enzymes with analogous enzymatic effect and of alanyl aminopeptidase N or of enzymes with analogous enzymatic effect.

In preferred embodiments of the invention the compounds can be successfully used as inhibitor precursors. With regard to the definitions the terms "inhibitor" and "precursor", it can be referred to the afore-mentioned definitions.

Further preferred compounds according to the invention serve for the use as precursors for inhibitors of dipeptidyl peptidase IV (DPIV) and peptidases with analogous enzymatic effect as well as of alanyl aminopeptidases N (APN) and peptidases with analogous enzymatic effect. Namely it was surprisingly found that compounds according to the invention can react under physiological or pathological conditions to such compounds which are highly effective inhibitors of dipeptidyl peptidase IV (DPIV) and peptidases with analogous enzymatic effect, as well as highly effective inhibitors of alanyl aminopeptidase N (APN) and peptidases with analogous enzymatic effect. According to the invention one of the new compounds according to formula (1) mentioned above can be used according to the general or special description or more than one of the compounds mentioned above can be used in combination. Said combinations of more than one compounds can comprise more than one compound of the general formula (1), i.e. at least two compounds of the general formula (1) in combination.

The invention also relates to the use of one or more compound(s), e. g. at least one compound, particularly preferred exactly one compound, of the general formula (1) according to the afore-mentioned general and detailed description for the prophylaxis and therapy of diseases with exceeding immune response and inflammatory genesis, including arteriosclerosis, neuronal disease, cerebral damages, skin diseases, tumour diseases and virus-caused diseases as well as type I diabetes.

Furthermore the invention relates to the use of one or more compound(s), e.g. at least of one compound, particularly preferred exactly one compound, of the general formula (1) according to the afore-mentioned general and detailed description for the preparation of a medicament or a cosmetic preparation for the prophylaxis and therapy of diseases with exceeding immune response and inflammatory genesis including arteriosclerosis, neuronal diseases, cerebral damages, skin diseases, tumour diseases and virus-caused diseases as well as type I diabetes.

Furthermore the invention relates to the use of one or more compound(s), e.g. at least of one compound, particularly preferred exactly one compound, of the general formula (1) according to the afore-mentioned general and detailed description for the preparation of a cosmetic preparation.

In preferred embodiments of the invention compounds of the general formula (1) are used in general, and preferably compounds according to Table 1 are used, solely or in combination or in the form of pharmaceutical or cosmetic preparations. Such preparations comprise one or more than one, i.e. at least two, of said compounds. Pharmaceutical preparations are for the prophylaxis and therapy of diseases such as, for example, multiple sclerosis, Morbus Crohn, colitis ulcerosa, and other autoimmune diseases as well as inflammatory diseases, asthma bronchiale and other allergic diseases, skin- and mucosa-related diseases, for example psoriasis, acne as well as dermatological diseases with hyper-proliferation and modified conditions of differentiation of fibroblasts, benign fibrosing and sclerosing skin diseases and malign fibroblastic conditions of hyper-proliferation, acute neuronal diseases such as for example ischemia-caused cerebral damages after an ischemia - or haemorrhagic apoplexia, cranio-cerebral injury, cardiac arrest, heart attack or as a consequence of cardio surgical intervention, of chronic neuronal diseases for example of Morbus Alzheimer, of the Pick-disease, a progressive supra-nuclear palsy, the corticobasal degeneration, the frontotemporal dementia, of Morbus Parkinson, especially parkinsonism coupled to chromosome number 17, of Morbus Huntington, of prion-caused conditions or diseases and amyotrophic lateral sclerosis, of arteriosclerosis, arterial inflammation, stent-restenosis, of chronic obstructive pulmonary disease (COPD), of tumours, metastases, of prostate carcinoma, of severe acute respiratory syndrome (SARS), and of sepsis and sepsis-like conditions as well as diabetes I.

In a further preferred embodiment of the invention the compounds of the general formula (1) are used in general, and preferably compounds according to Table 1, solely or in combination or in the form of pharmaceutical or cosmetic preparations. Such preparations comprise one or more of said compounds. Pharmaceutical preparations are used for the prophylaxis and therapy of the rejection of transplanted tissues and cells. As an example of such a use, the use of one or more of the afore-mentioned compounds or of a pharmaceutical preparation containing one or more of the afore-mentioned compounds can be mentioned with regard to allogene or xenogene transplanted organs, tissues and cells such as bone marrow, kidney-, heart-, liver- pancreas-, skin- or stem cell transplantation, as well as graft versus host diseases (GvHD).

In a further preferred embodiment of the invention the compounds of the general formula (1) in general, and preferably the compounds according to the afore-mentioned table, are used solely or in combination or in the form of pharmaceutical or cosmetic preparations. Such preparations contain one or more of said compounds. Pharmaceutical preparations are used for the prophylaxis and therapy of reactions concerning rejection or inflammation at - or caused by - medical devices implanted into an organism. These can be for examples stents, vessel balloons, joint implants (knee joint implants, hip joint implants), bone implants, cardiac pace makers or other implants.

In a further preferred embodiment of the invention the compounds of the general formula (1) are used in general, and preferably the compounds according to the above table 1 are used, solely or in combination or the form of pharmaceutical or cosmetic preparations. Such preparations are containing one or more of said compound(s) in that way that the compound(s) or preparation(s) are applied in form of a coating or a wetting onto the item(s), or at least one of the compounds or preparations is materially admixed to the item(s). Also in this case it is certainly possible to apply one of the compounds or preparations - if applicable subsequently or parallelly - locally or systemically.

In the same way as mentioned above - and for comparable purposes or for prophylaxis and therapy of the above exemplary but not completely mentioned diseases and conditions - the compounds of the general formula (1) in general, and the compounds according to the above table in preferred embodiments, as well as the following pharmaceutical and cosmetic preparations containing said compounds can be used solely or in combination of more than one of them to prepare medicaments for the treatment of the afore-mentioned diseases and conditions or cosmetic preparations. These can comprise the afore-mentioned compounds in amounts mentioned in the following, optionally in combination with *per se* known carrier substances, auxiliary substances and/or additives.

In the context of the inventive use an application of at least one of said compounds of general formula (1) can be achieved on any pathway *per se* known which a person normally skilled in this technical field knows. The application of compounds of the general formula (1) in general, and further preferred the compound according to the afore-mentioned table, or pharmaceutical or cosmetic preparations, respectively, which comprise one or more of the afore-mentioned compounds in combination with *per se* known usual carrier substances, auxiliary substances and/or additives is either carried out as topic application, on the one hand, in form of for example cremes, ointments, pastes, gels, solutions, sprays, liposomes and nanosomes, shake mixtures, "pegylated" formulations degradable (e.g. degradable under physiological conditions) depot-matrices, hydrocolloid bandages, plasters, micro-sponges, prepolymers and similar new carrier substrates, jet-injection or other dermatological principles/vehicles including instillative application, and, on the other hand, as systemic application for oral, transdermal, intravenous, subcutane, intracutane, intramuscular, intrathecal application in suitable formulations or suitable galenic forms, thus in form of tablets, dragees, lozenges, capsules, aerosols, sprays, solutions, emulsions and suspensions.

The invention also relates to a process for the inhibition of the alanyl aminopeptidase N activity or of the activity of peptidases with analogous enzymatic effect as well as the dipeptidyl peptidase IV activity or of the activity of peptidases with analogous enzymatic effect, either solely or in combination with other inhibitors of alanyl aminopeptidase N or inhibitors of peptidases with analogous enzymatic effect and/or other inhibitors of DPIV or inhibitors of peptidases with analogous enzymatic effect, by the application of at least one compound of the general formula (1) or of a pharmaceutical or cosmetic preparation, which comprises at least one of the compounds of the general formula (1) according to the afore-mentioned detailed description, in an amount necessary for the inhibition for the enzyme activity. The amounts of the compounds of the general formula (1) in general, or of the compounds according to the above table, respectively, significantly vary for the diseases or conditions for which they are used, and for the severity of such diseases and conditions, as well as for the route of administration applied. Only to give selected examples, which do not restrict the invention presently, amounts are in the range - as mentioned above - of 0.01 to 10,000 mg of at least one compound per application unit, preferably in the range of 0.1 to 1,000 mg per application unit.

Further the invention relates to a process for topically influencing the alanyl aminopeptidase N activity or the activity of peptidases with analogous enzymatic effect as well as for topically influencing the dipeptidyl peptidase IV activity or the activity of peptidases with analogous enzymatic effect, either solely or in combination with other alanyl aminopeptidase N inhibitors or inhibitors of peptidases with analogous enzymatic effect and/or with other dipeptidyl peptidase IV inhibitors or inhibitors of peptidases with analogous enzymatic effect, by the application of at least one compound of the general formula (1) or of pharmaceutical or cosmetic preparations according to the following detailed description in an amount necessary for influencing or manipulating the enzyme activity. Also in these cases, the amounts of the compound(s) of the general formula (1) are in the range mentioned above.

Furthermore the invention relates to a process to generate at least one inhibitor of dipeptidyl peptidase IV (DPIV) and of peptidases with analogous enzymatic effect as well as of alanyl aminopeptidase N (APN) and of peptidases with analogous enzymatic effect from at least one of the compounds of the general formula (1). The process according to the invention comprises the step that at least one of the compounds of the general formula (1) is exposed to suitable conditions according to the afore-mentioned description. As previously described a skilled person is not restricted with regard to suitable conditions for the transformation of at least one compound of the general formula (1) which can insofar as well be regarded as intermediate in the synthesis of inhibitors, according to the invention, of dipeptidyl peptidase IV (DPIV) and of inhibitors of peptidases with analogous enzymatic effect as well as of inhibitors of the alanyl aminopeptidase N (APN) and of inhibitors of peptidases with analogous enzymatic effect into the actual inhibitors of a dipeptidyl peptidase IV (DPIV) and inhibitors of peptidases with analogous enzymatic effect as well as into the actual inhibitors of alanyl aminopeptidase N (APN) and inhibitors of peptidases with analogous enzymatic effect.

Furthermore the invention relates to a process for the prophylaxis and therapy of numerous diseases, for example of diseases with exceeding immune response (autoimmune diseases, allergies and transplant rejections including Graft-versus-Host Diseases (GvHD), of other chronic inflammatory diseases, neuronal diseases and cerebral damages, skin diseases (*inter alia* acne and psoriasis), of tumour diseases and of particular virus infections (*inter alia* SARS) as well as type I diabetes and particularly the diseases mentioned above in detail. This includes: processes for the prophylaxis and therapy of diseases such as for example multiple sclerosis, Morbus Crohn, colitis ulcerosa, and other autoimmune diseases as well as inflammatory diseases, asthma bronchiale and other allergic diseases, skin- and mucosa diseases for example psoriasis, acne and atopic dermatitis, as well as dermatological diseases with a hyper-proliferation and modified conditions of differentiation of fibroblasts, benigne fibrosing and sclerosing skin diseases and malign fibroblastic hyper-proliferation conditions, acute neuronal diseases, such as for example ischemia-caused cerebral damages after an ischemia- or haemorrhagic apoplexia, cranio-cerebral injury, cardiac arrest, heart attack or as a consequence of cardio-surgical interventions, of chronic neuronal diseases such as for example Morbus Alzheimer, the Pick-disease, the progressive supra-nuclear palsy, the corticobasal denegeration, the fronto-temporal dementia, of Morbus Parkinson, particularly parkinsonism coupled to chromosome 17, of Morbus Huntington, or disease conditions caused by prions and of amyotrophic lateral sclerosis, of arteriosclerosis, arterial inflammations, stent-restenosis, of chronic obstructive pulmonary diseases (COPD), of tumours, metastases, of prostate carcinoma, of severe acute respiratory syndrome (SARS) and of sepsis and sepsis-like conditions. The process comprises an application of at least one compound or pharmaceutical preparation according to the following detailed description in an amount necessary for the prophylaxis or therapy of the accordant disease. Also in these cases, exemplary (non-restricting) amounts of compound(s) is/are in the afore-mentioned range of 0.01 to 10,000 mg of a compound per application unit preferably in a range of 0.1 to 1000 mg per application unit.

The invention also relates to pharmaceutical preparations, which comprise at least one compound of at least one of the general formula (1) of the afore-mentioned general and detailed description, optionally in combination with one or more pharmaceutically acceptable carrier substance(s), auxiliary substance(s) and/or adjuvant(s).

Furthermore, the invention also relates to cosmetic preparations, which comprise at least one of the compounds of at least one of the general formula (1) of the afore-mentioned general and detailed description if applicable in combination with one or more cosmetically acceptable carrier substance(s), auxiliary substance(s) and/or adjuvant(s).

With regard to these preparations the pharmaceutically or cosmetically acceptable carrier substances, auxiliary substances and/or adjuvants are sufficiently known to a person skilled in the pharmaceutical or cosmetic field and do not require any further detailed mentioning.

The mentioned pharmaceutical or cosmetic preparations, might contain at least one compound of the general formula (1), preferably one or two compounds of the general formula (1), in such amount(s) which is/are necessary for the desired effect in the pharmaceutical or cosmetic field. The amount(s) is/are not particularly restricted and is/are dependent on a number of parameters such as for example the application pathway, the specific disease pattern or the cosmetic status, of the constitution of the addressee who can be a mammal such as for example a human, of the bioavailablility of the used compound(s) etc.. In particularly preferred embodiments not restricting the invention, a pharmaceutical application unit or a cosmetic application unit, respectively, contains an amount of at least one compound of the general formula (1) which is in the range of 0.01 to 2,000 mg of a compound per application unit, preferably in the range of 0.1 to 500 mg per application unit. Usually the application units can be of that type (and contain such concentrations of at least one compound of the general formula (1)) that the application of one or less further preferred two or three application units per day is sufficient to apply an amount necessary for a systematic pharmaceutical or cosmetic treatment with regard to at least one of the compounds (1) to the patient or receiver such as for example a mammal, particularly a human.

The invention is explained in the following by examples of particularly preferred embodiments. The following examples of embodiments are not to restrict the invention but only to give an exemplary illustration.

## Examples

### Example 1

### Preparation of compounds of the general formula (1)

Compounds of the general formula (1) were prepared using the following processes:

Scheme 1: α-Hydroxy-β-homophenylalanine fragment 5

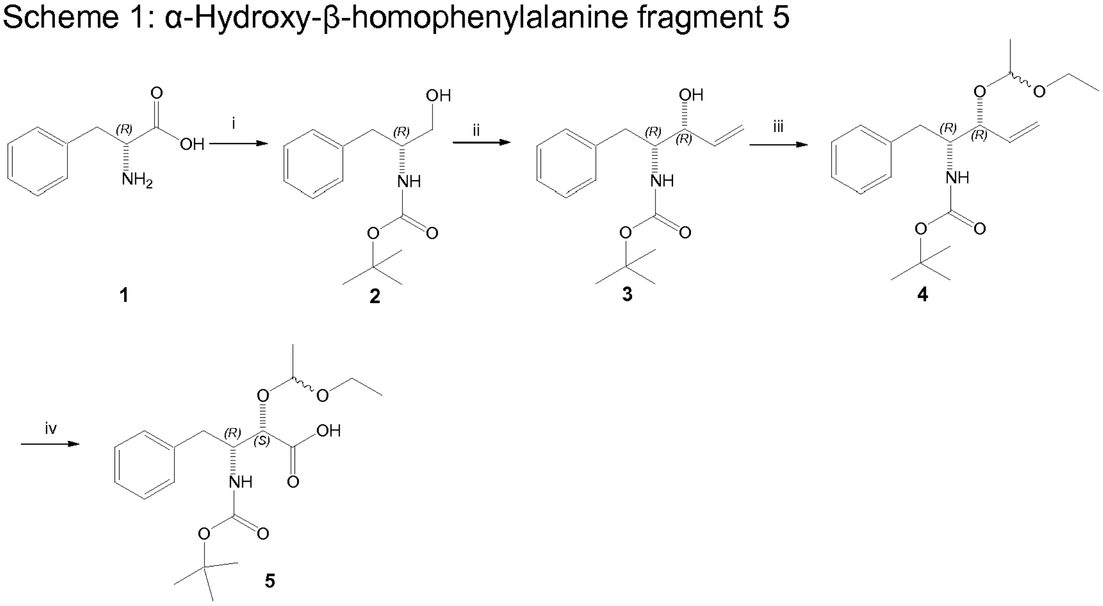


1 2 3 4 5

Reagents and conditions: i) 1. $LiAlH_4$, THF, reflux; 2. $NaOH/H_2O$, $Boc_2O$/DCM, RT. ii) 1. $(COCl)_2$, DMSO, DIPEA, DCM, -78˚C => -10˚C; 2. Vinylmagnesium bromide, THF/DCM, RT. iii) Vinylethylether, PPTS, DCM, RT. iv) $NaIO_4$, $NaHCO_3$, $RuCl_3$, $CH_3CN/CCl_4/H_2O$, RT.

Scheme 2: β-homophenylalanine fragment 10

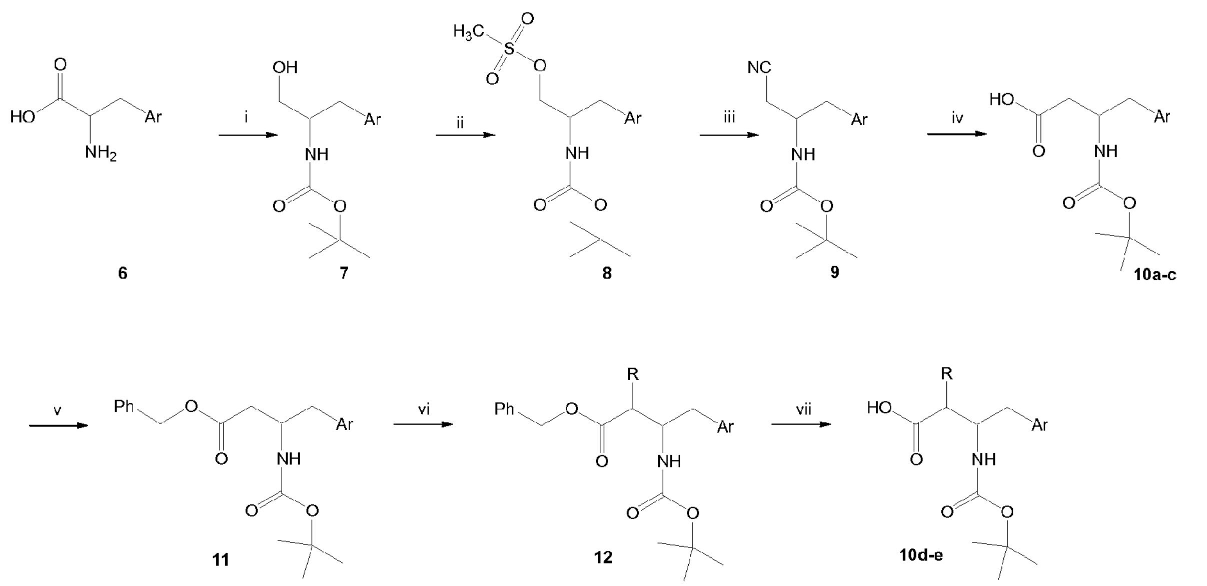


6 7 8 9 10a-c

11 12 10d-e

Reagents and conditions: i) 1. $LiAlH_4$, THF, reflux.; 2. $NaOH/H_2O$, $Boc_2O$/DCM, RT. ii) $CH_3SO_2Cl$, TEA, DCM, RT. iii) NaCN, DMF, 60˚C. iv) 1. NaOH, $H_2O$, 100˚C; 2. $Boc_2O$/1,4-Dioxane, RT. v) BnBr, $K_2CO_3$, DMF. vi) LiHMDS, THF, - 78˚C then $BrCH_2COO$-*t*-Bu or MeI. vii) $H_2$, Pd/C, MeOH.

**Table 2:**

| Compound | Configuration | Ar | R |
|---|---|---|---|
| 10a | *R* | Phenyl | H |

(continued)

| Compound | Configuration | Ar | R |
|---|---|---|---|
| 10b* | *R* | 2-Naphtyl | H |
| 10c | *R*/*S* | 2,5-Difluorophenyl | H |
| 10d | *R*/*S* | 2,5-Difluorophenyl | $CH_2COO$-*t*-Bu |
| 10e | *R*/*S* | 2,5-Difluorophenyl | Me |
| * Compound commercially available | | | |

Scheme 3:

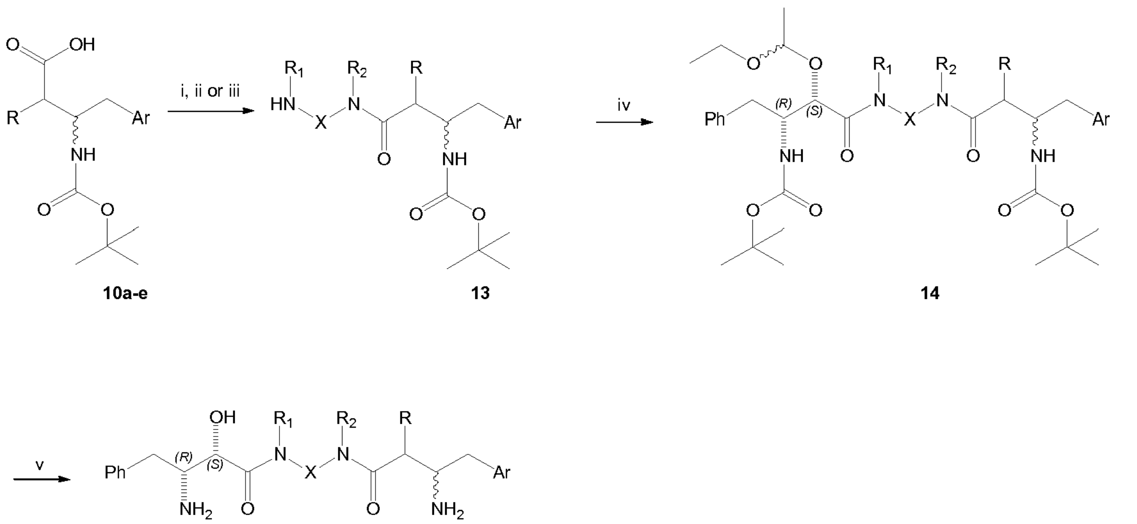


10a-e

13

14

15

Reagents and conditions: i) HOBt, DCC, DCM, diamino compound, 0˚C => RT. ii) a) HOBt, DCC, DCM, mono-Cbz-protected diamino compound, 0˚C => RT; b) $H_2$, Pd/C, MeOH. iii) a) HOBt, DCC, DCM, nitro amino compound, 0˚C => RT; b) $HCOONH_4$, Pd/C, MeOH. iv) 5, HOBt, DCC, DCM, 0˚C => RT. v) aq. HCl (37%), EtOH, RT.

**Table 3:**

| Starting material (Ar) | X | $R_1$ | $R_2$ | Method | R |
|---|---|---|---|---|---|
| 10a (Phenyl) | $(CH_2)_2$ | H | H | ii | H |
| 10b (2-Naphtyl) | $(CH_2)_2$ | H | H | ii | H |
| 10c (2,5-Difluorophenyl) | $(CH_2)_2$ | H | H | ii | H |
| 10a (Phenyl) | $CH_2CH(CH_3)$ | $CH_2CH(CH_3)$ | | ii | H |
| 10a (Phenyl) | $(CH_2)_4$ | H | H | i | H |
| 10a (Phenyl) | $C(CH_3)_2C(CH_3)_2$ | H | H | i | H |
| 10a (Phenyl) | $CH_2C(CH_2CH_3)_2CH_2$ | H | H | i | H |
| 10a (Phenyl) | $CH_2C(CH_3)_2CH_2$ | H | H | i | H |
| 10a (Phenyl) | $CH_2C((CH_2)_3)CH_2$ | H | H | i | H |
| 10c (2,5-Difluorophenyl) | $CH_2C(CH_3)_2CH_2$ | H | H | i | H |
| 10c (2,5-Difluorophenyl) | $(CH_2)_4$ | H | H | i | H |
| 10a (Phenyl) | $(CH_2)_5$ | H | H | i | H |

(continued)

| Starting material (Ar) | X | $R_1$ | $R_2$ | Method | R |
|---|---|---|---|---|---|
| 10a (Phenyl) | $(CH_2)_6$ | H | H | i | H |
| 10c (2,5-Difluorophenyl) | $(CH_2)_3$ | H | H | i | H |
| 10c (2,5-Difluorophenyl) | $CH_2C(CH_3)_2(CH_2)_2$ | H | H | iii | H |
| 10c (2,5-Difluorophenyl) | $(CH_2)_3$ | $CH_3$ | $CH_3$ | i | H |
| 10c (2,5-Difluorophenyl) | $(CH_2)_3C(COOH)$ | H | H | ii | H |
| 10c (2,5-Difluorophenyl) | $(CH_2)_3$ | H | $CH_3$ | i | H |
| 10c (2,5-Difluorophenyl) | 1,4-$C_6H_4$ | H | H | i | H |
| 10d (2,5-Difluorophenyl) | $CH_2C(CH_3)_2CH_2$ | H | H | i | $CH_2COOH$ |
| 10c (2,5-Difluorophenyl) | $(CH_2)_3$ | $CH_3$ | H | i | H |
| 10c (2,5-Difluorophenyl) | $CH_2C((CH_2)_3)CH_2$ | H | H | i | H |
| 10c (2,5-Difluorophenyl) | $CH_2C(CH_2CH_3)_2CH_2$ | H | H | i | H |
| 10e (2,5-Difluorophenyl) | $CH_2C(CH_3)_2CH_2$ | H | H | i | Me |
| 10c (2,5-Difluorophenyl) | 1,3-$C_6H_4$ | H | H | i | H |
| 10c (2,5-Difluorophenyl) | $(CH_2)_3$ | $(CH_2)_2$ | | i | H |
| 10c (2,5-Difluorophenyl) | $CH_2C((CH_2)_4)CH_2$ | H | H | i | H |
| 10c (2,5-Difluorophenyl) | $CH_2C((CH_2)_5)CH_2$ | H | H | i | H |
| 10c (2,5-Difluorophenyl) | 1,3-Cyclohexane | H | H | i | H |
| 10c (2,5-Difluorophenyl) | $(CH_2)_2$ | $(CH_2)_2$ | | i | H |

Scheme 4:

5
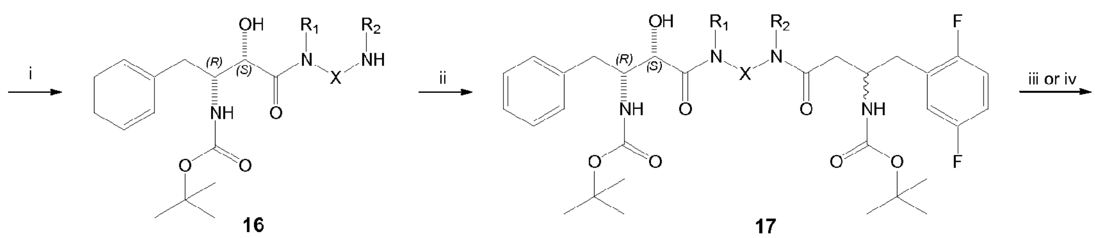


16 17

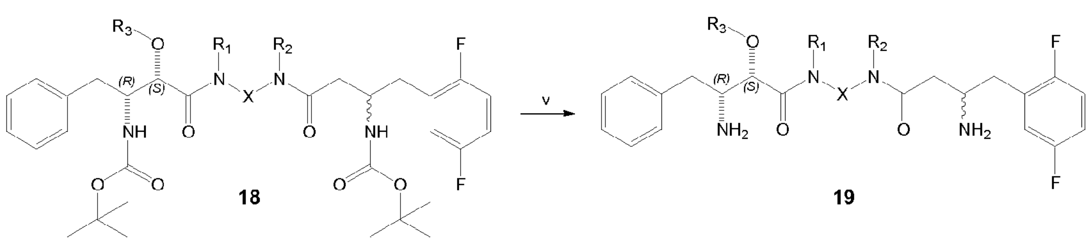


18 19

Reagents and conditions: i) HOBt, DCC, DCM, diaminocompound, 0˚C => RT. ii)l, HOBt, DCC, DCM, 0˚C => RT. iii) Alkanoyl chloride, TEA, DMAP, DCM, RT. iv) Alkyl bromide, NaH, KI, DMF. v) TFA, DCM, RT.

**Table 4:**

| X | $R_1$ | $R_2$ | $R_4$ |
|---|---|---|---|
| $CH_2C(CH_3)_2CH_2$ | H | H | $CO(CH_2)_{14}CH_3$ |
| $CH_2C(CH_3)_2CH_2$ | H | H | $CO(CH_2)_{12}CH_3$ |
| $CH_2C(CH_3)_2CH_2$ | H | H | $CO(CH_2)_{10}CH_3$ |
| $CH_2C(CH_3)_2CH_2$ | H | H | $CO(CH_2)_8CH_3$ |
| $CH_2C(CH_3)_2CH_2$ | H | H | $CO(CH_2)_6CH_3$ |
| $CH_2C(CH_3)_2CH_2$ | H | H | $CO(CH_2)_4CH_3$ |
| $CH_2C(CH_3)_2CH_2$ | H | H | $CO(CH_2)_2CH_3$ |
| $CH_2C(CH_3)_2CH_2$ | H | H | $CO(CH_2)_3Ph$ |
| $CH_2C(CH_3)_2CH_2$ | H | H | $(CH_2)_{15}CH_3$ |
| $CH_2C(CH_3)_2CH_2$ | H | H | $(CH_2)_7CH_3$ |
| $(CH_2)_4$ | H | H | $CO(CH_2)_{14}CH_3$ |
| $(CH_2)_4$ | H | H | $CO(CH_2)_6CH_3$ |
| $(CH_2)_3$ | Me | H | $CO(CH_2)_6CH_3$ |

Scheme 5:

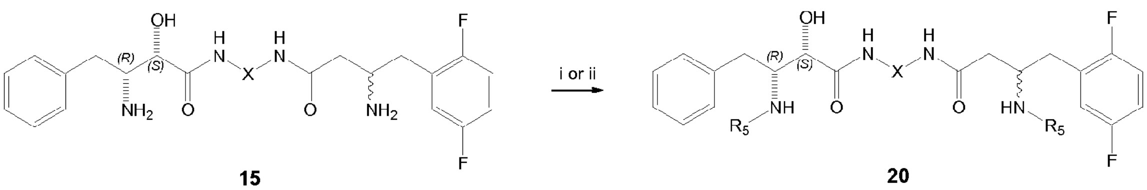


15

20

Reagents and conditions: i) MgO, THF/$H_2O$, Alkanoyl chloride. ii) Aldehyde, THF, MS 3A then $NaCNBH_3$.

**Table 5:**

| X | $R_5$ | Method |
|---|---|---|
| $CH_2C(CH_3)_2CH_2$ | $CO(CH_2)_6CH_3$ | i |
| $CH_2C(CH_3)_2CH_2$ | $CO(CH_2)_2CH_3$ | I |
| $CH_2C(CH_3)_2CH_2$ | $CO(CH_2)_{12}CH_3$ | I |
| $CH_2C(CH_3)_2CH_2$ | $CO(CH_2)_{14}CH_3$ | I |
| $CH_2C(CH_3)_2CH_2$ | $(CH_2)_{15}CH_3$ | Ii |

Scheme 6:

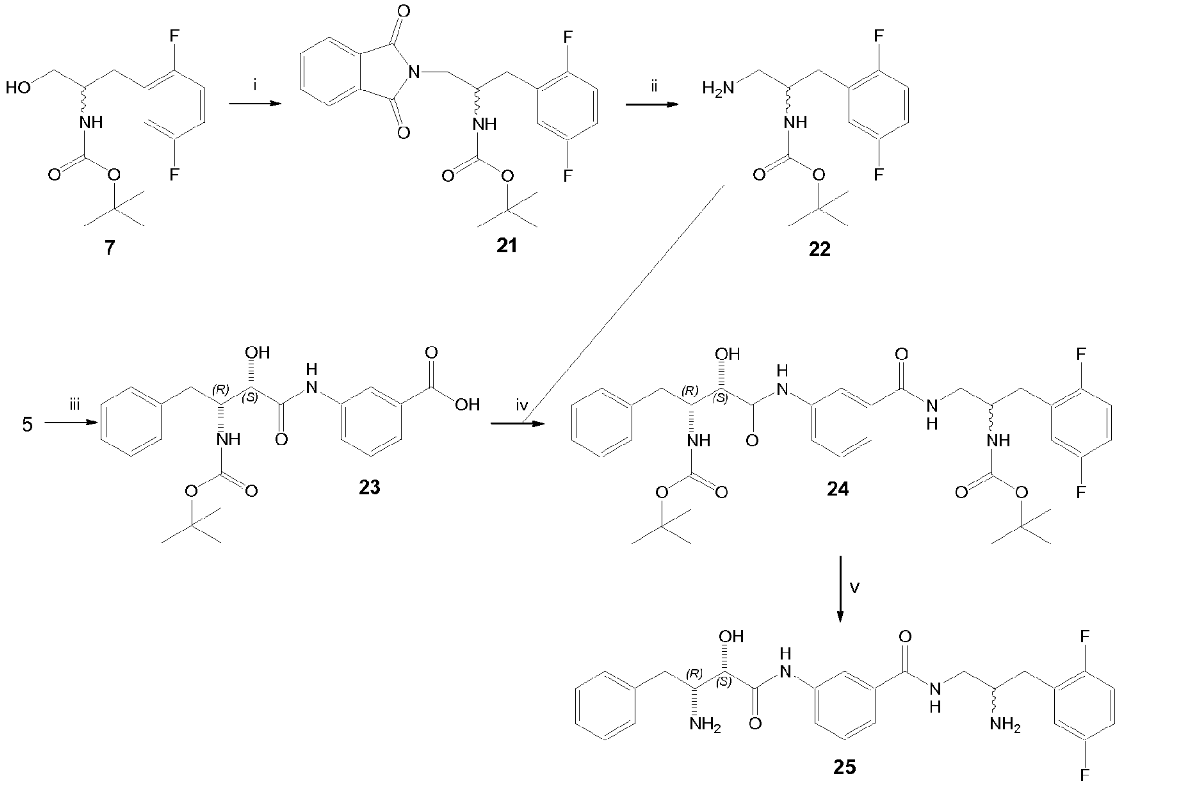


7, 21, 22, 5, 23, 24, 25

Reagents and conditions: i) $PPh_3$, DEAD, phtalimide, THF, 0˚C => RT. ii) $H_2NNH_2$, EtOH, reflux. iii) HOBt, DCC, DCM, 3-aminobenzoic acid, 0˚C => RT. iv) HOBt, DCC, DCM, 0˚C => RT. v) TFA, DCM, RT.

Scheme 7:

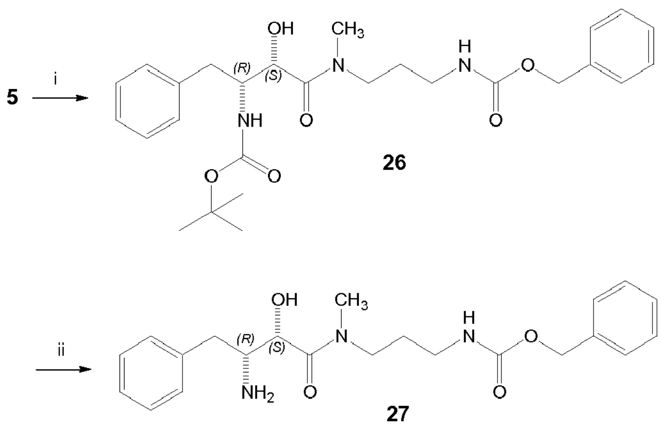


5, 26, 27

Reagents and conditions: i) HOBt, DCC, DCM, mono-Cbz-protected diamino compound, 0˚C => RT. ii) TFA, DCM, RT.

Scheme 8:

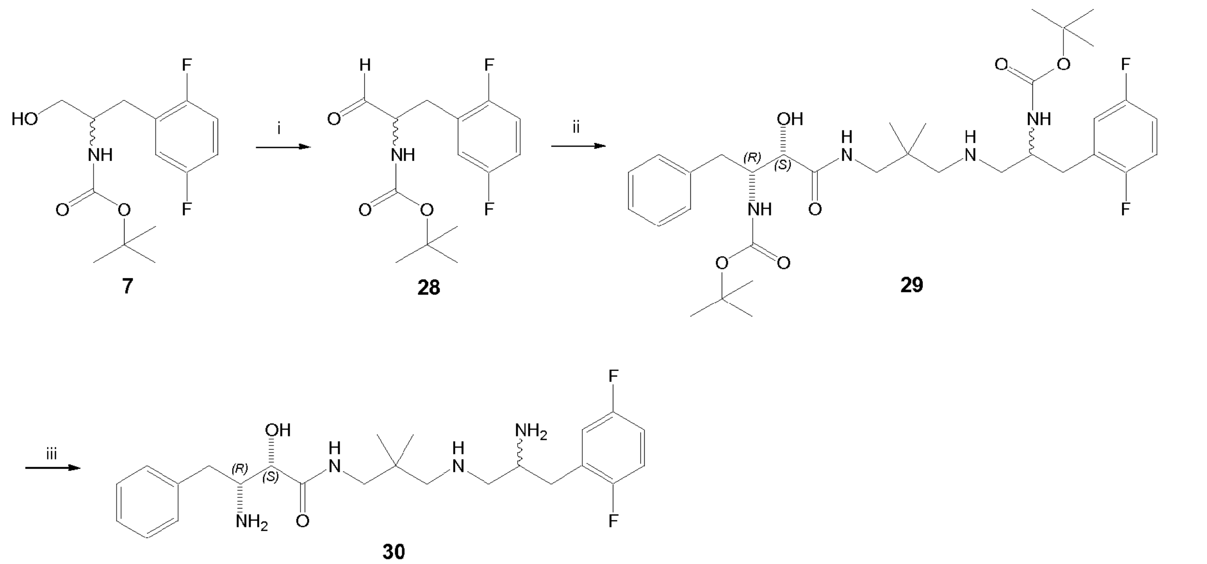


7 28 29

30

Reagents and conditions: i) Dess-Martin-periodoindane, DCM, 0˚C -> RT. ii) 16, THF, MS 3A then $NaCNBH_3$, RT. iii) aq. HCl (37%), EtOH, RT.

Scheme 9:

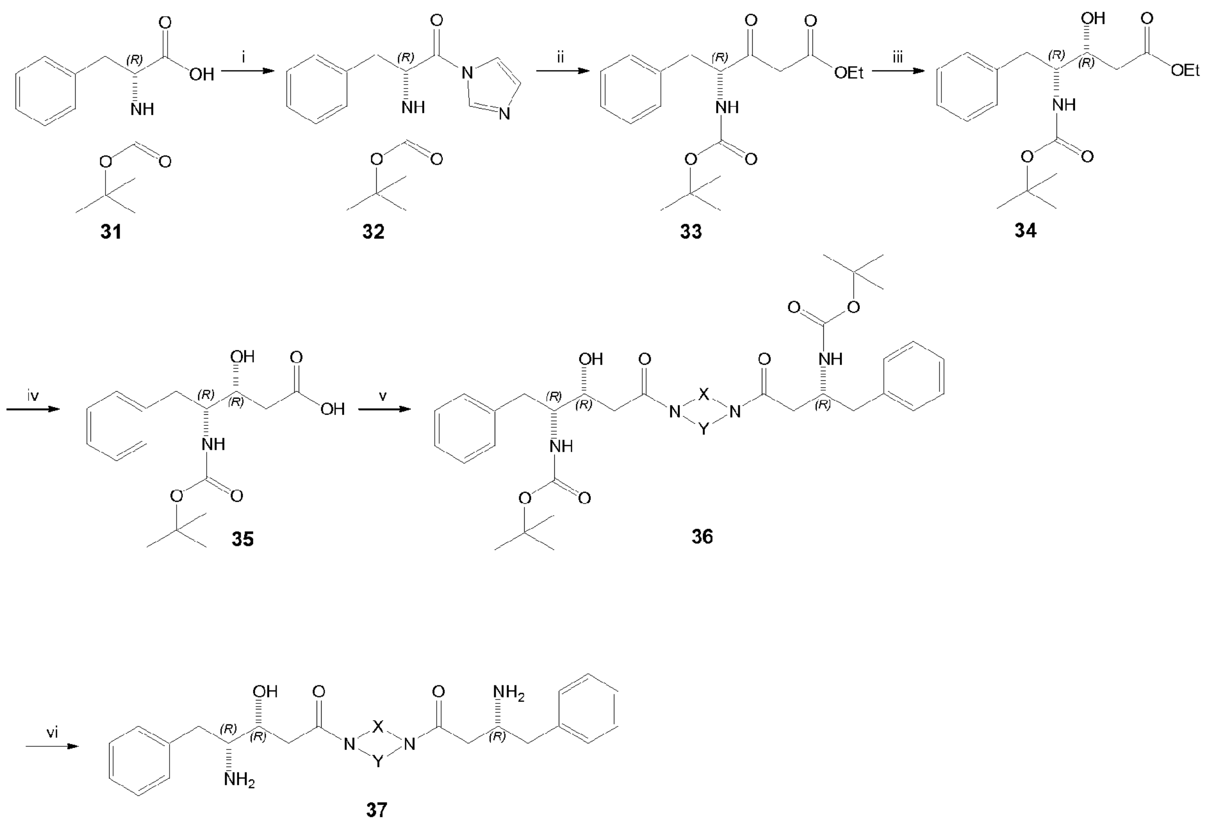


31 32 33 34

35 36

37

Reagents and conditions: i) CDI, THF, RT. ii) LDA, EtOAc, THF, -78˚C. iii) $TiCl_4$, $PyBH_3$, DCM, -78˚C. iv) LiOH*$H_2O$, MeOH, RT. v) DCC, HOBt, 13, DCM, 0˚C -> RT. vi) aq. HCl (37%), EtOH, RT.

**Table 6:**

| X | Y |
|---|---|
| $(CH_2)_2$ | $H_2$ |
| $CH_2(CMe_2)CH_2$ | $H_2$ |
| $(CH_2)_2$ | $(CH_2)_2$ |

Scheme 10:

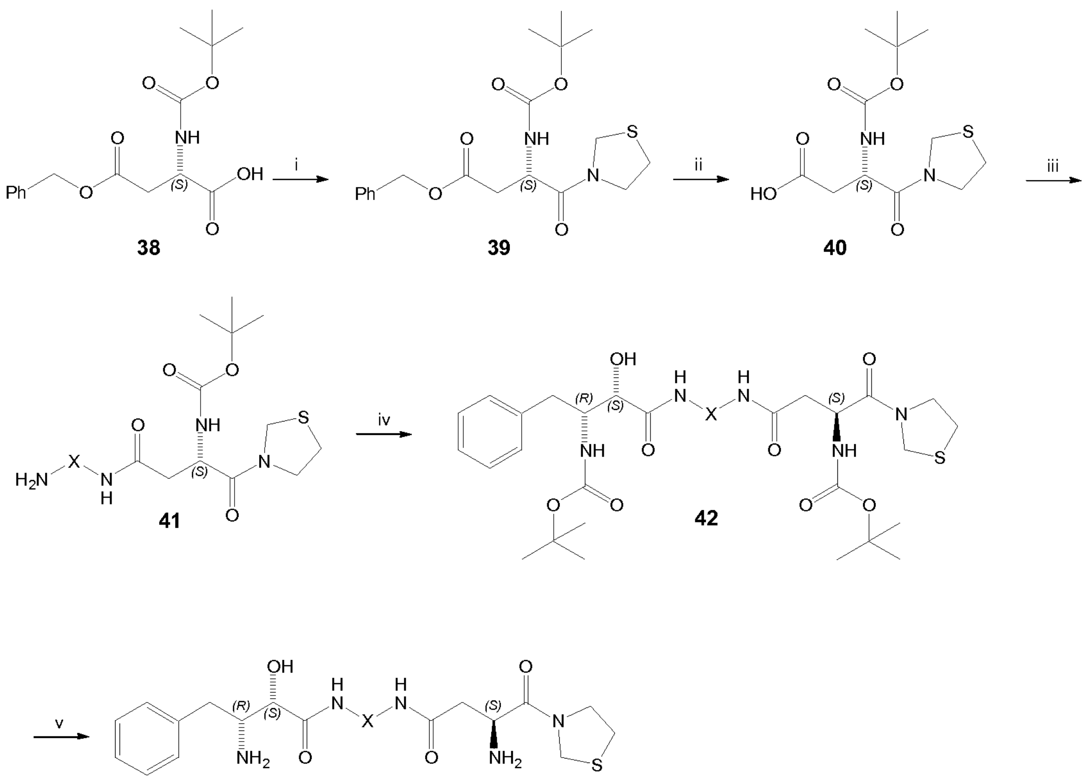


38 39 40 41 42 43

Reagents and conditions: i) CDI, thiazolidine, DCM, RT. ii) LiOH*$H_2O$, MeOH, RT. iii) DCC, HOBt, diamine, 0˚C -> RT. iv) 16, DCC, HOBt, 0˚C -> RT. v) aq. HCl (37%), EtOH, RT.

Table 7:

| X |
|---|
| $CH_2C(CH_3)_2CH_2$ |
| $(CH_2)_4$ |

Scheme 11:

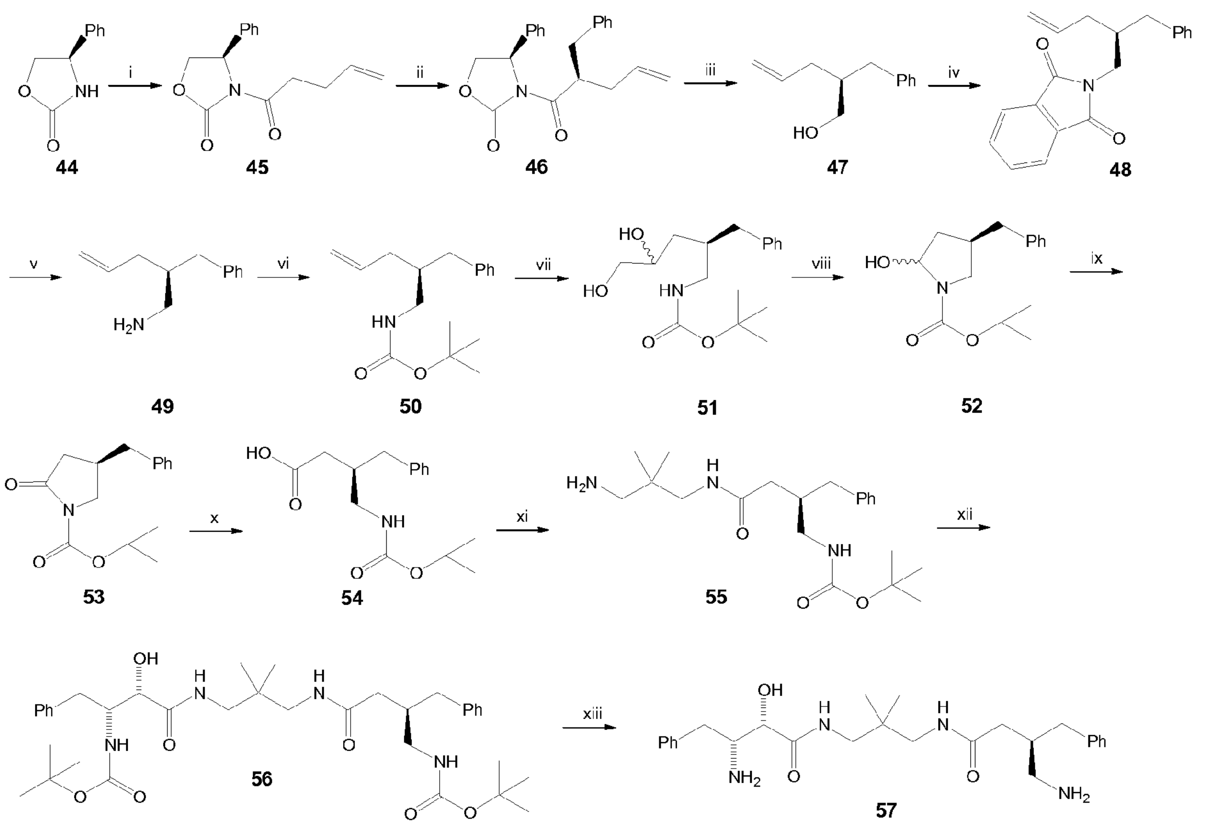


44 45 46 47 48 49 50 51 52 53 54 55 56 57

Reagents and conditions: i) n-BuLi, pent-4-enoylchloride, THF, -78˚C -> RT. ii) LiHMDS, benzylic bromide, THF, -78˚C -> RT. iii) $LiAlH_4$, THF, -78˚C -> RT. iv) phthalimde, $PPh_3$, DEAD, THF, -0˚C -> RT. v) hydrazine hydrate, EtOH, reflux. vi) $Boc_2O$, $NaHCO_3$, water/1,4-dioxane. vii) $OsO_4$, NMMO, water / THF, 0˚C -> RT. viii) $NaIO_4$, water / THF, 0˚C. ix) PCC, MS 3A, dichloromethane, RT. x) LiOH, water / THF, RT. xi) 1,3-diamino-2,2-dimethylpropane, HOBt, DCC, dichloromethane, 0˚C -> RT. xii) 5, HOBt, DCC, dichloromethane, 0˚C -> RT. xiii) HCl, EtOH, RT.

Scheme 12:

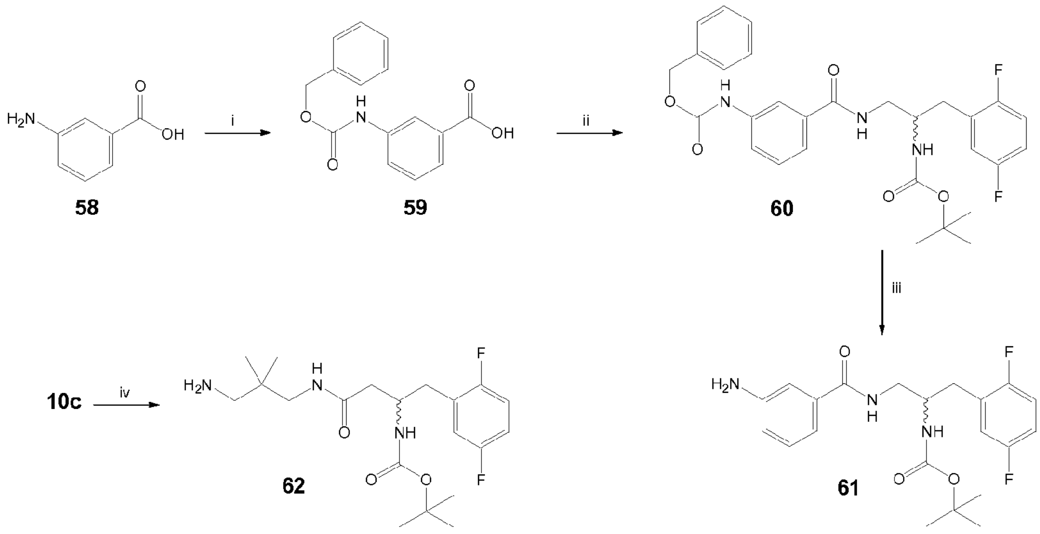


58 59 60 10c 62 61

Reagents and conditions: i) CBz-Cl, $NaHCO_3$, H2O / 1,4-dioxane. ii) 22, DCC, HOBt, DCM, 0˚C -> RT. iii) $H_2$, cat. Pd / C, MeOH, RT, 1 atm. iv) 10c, DCC, HOBt, DCM, 0˚C -> RT.

Scheme 13:

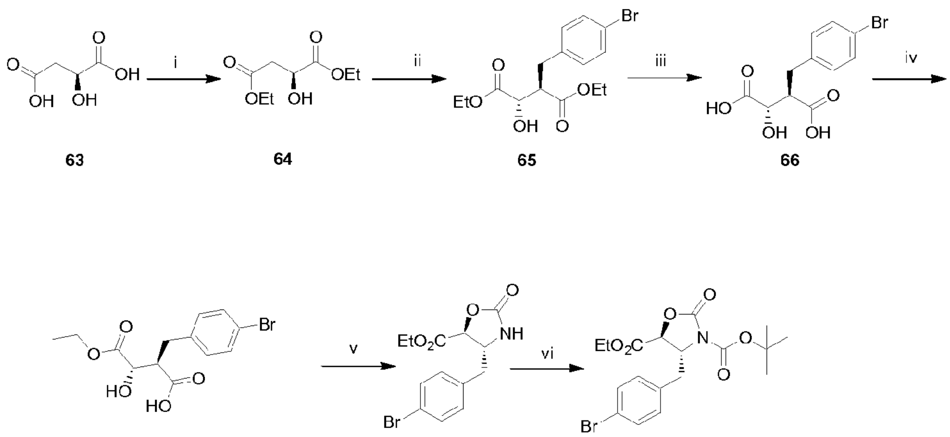


63 64 65 66 67 68 69

Reagents and conditions: i) EtOH, Conc.HCl, reflux, 17 h. ii) LiHMDS, THF, 4-Br-B*n*Br, -78 ˚C, 22 h. iii) 1M aq.NaOH, Dioxane, RT to reflux. iv) TFAA, 0 ˚C, EtOH. v) Diphenyl phosphonic azide, $Et_3N$, Benzene, reflux. vi) $Boc_2O$, 4-DMAP, $Et_3N$, $CH_2Cl_2$, RT, 4 h.

Scheme 14:

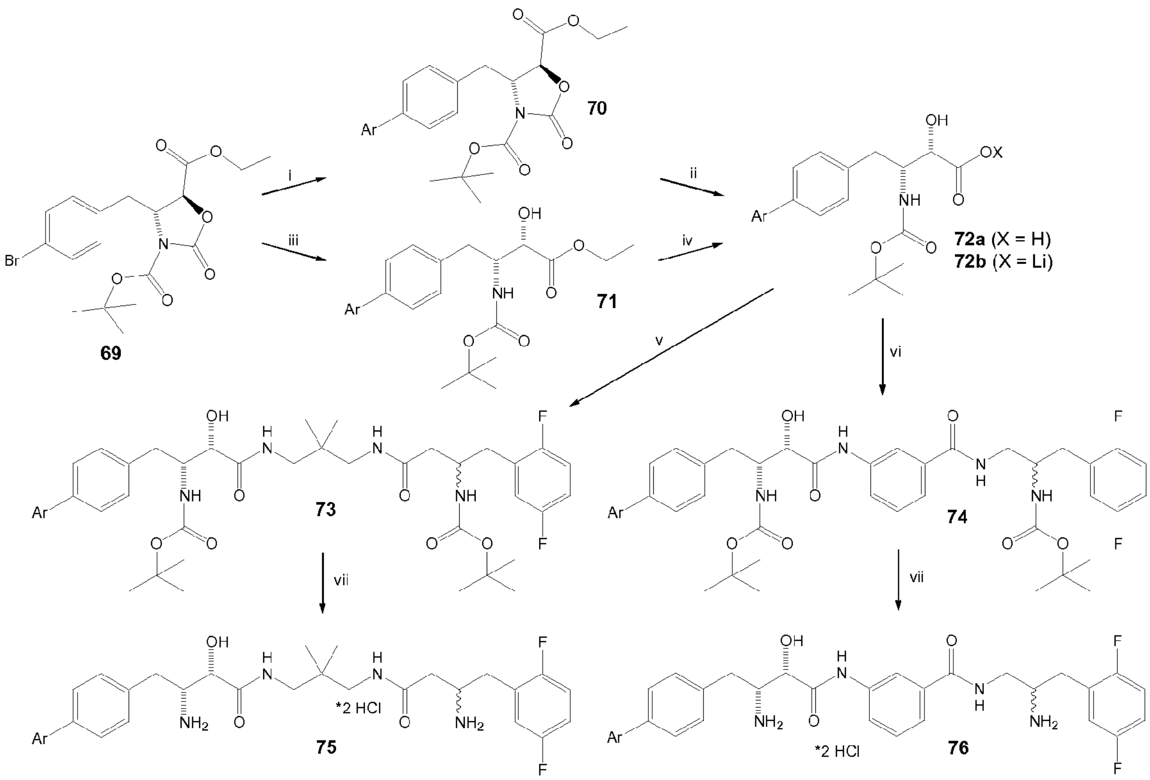


69 70 71 73 74 75 76

Reagents and conditions: i) arylboronic acid, cat. Pd($PPh_3$)$_4$, $K_2CO_3$, toluene, 90˚C. ii) LiOH, MeOH / $H_2O$. iii) arylboronic acid, cat. Pd($PPh_3$)$_4$, $K_2CO_3$, toluene / EtOH, 90˚C. iv) LiOH, MeOH / $H_2O$. v) 62, DCC, HOBt, DCM, 0˚C -> RT. vi) 61, DCC, HOBt, DCM, 0˚C -> RT. vii) HCl / EtOH, RT.

**Table 8:**

| Compound | Ar | X |
|---|---|---|
| 75a | phenyl | H |
| 75b | thiophen-3-yl | H |
| 75c | pyridin-3-yl | Li |
| 75d | pyrmindin-5-yl | Li |
| 76a | thiophen-3-yl | H |

Scheme 15:

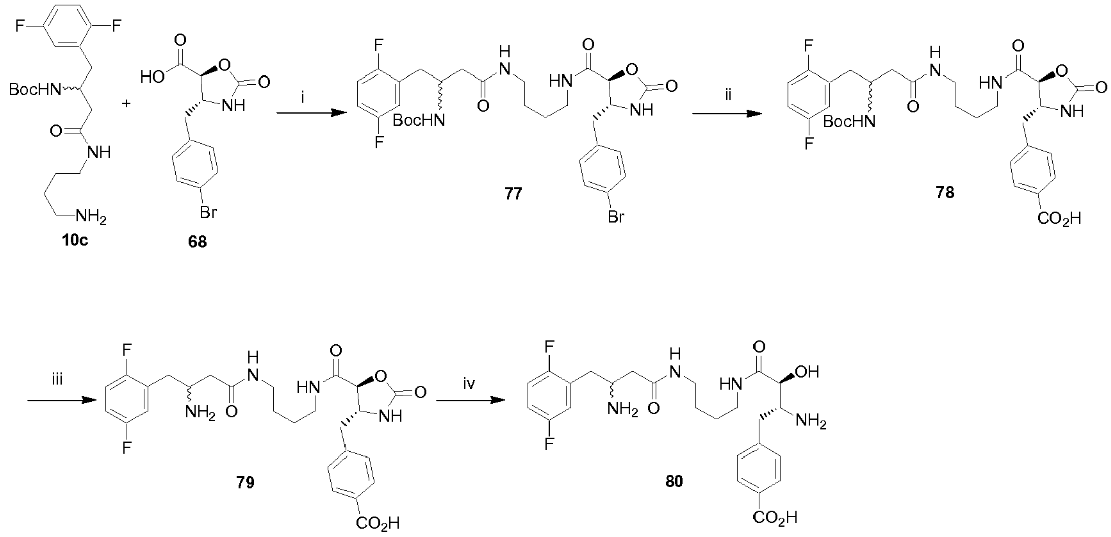


Reagents and conditions: i) DCC, HOBt, $CH_2Cl_2$, 0 ˚C to RT. ii) *t*-BuLi, -78 ˚C, $CO_2$. iii) TFA, $CH_2Cl_2$, 0 ˚C to RT. iv) $Cs_2CO_3$, MeOH/$H_2O$.

Scheme 16:

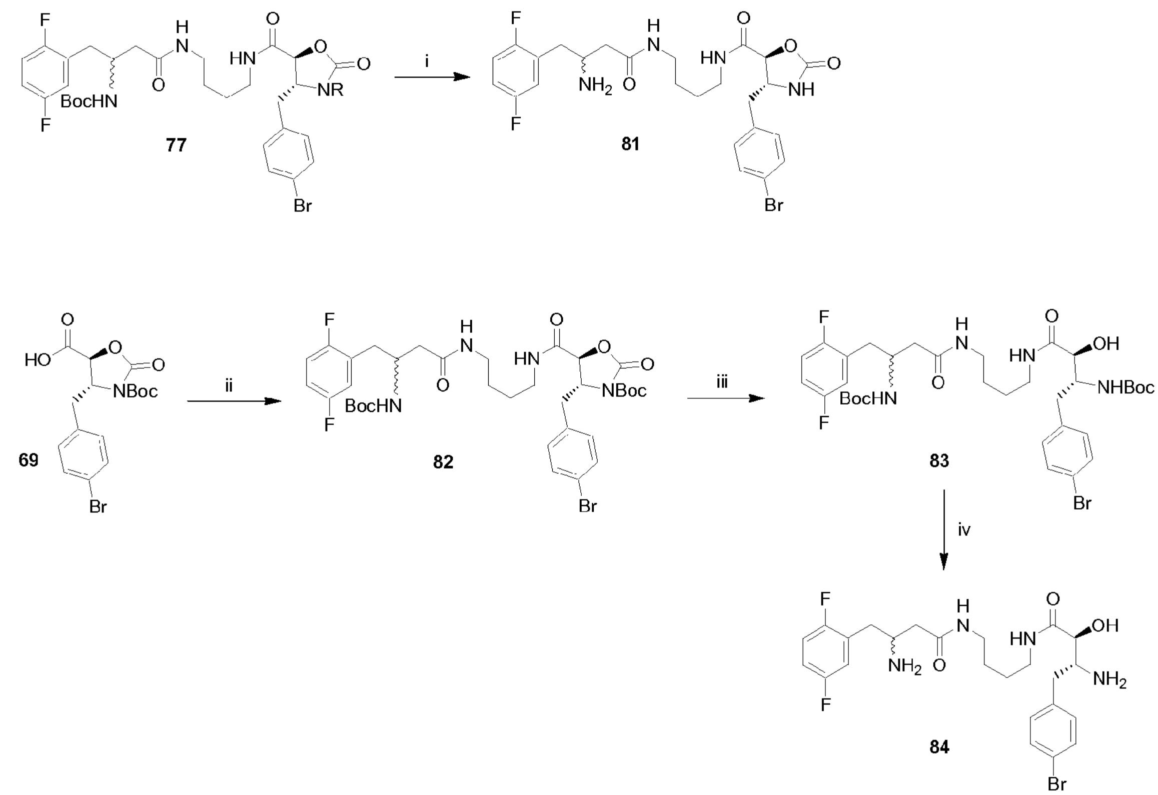

Reagents and conditions: i)TFA, $CH_2Cl_2$, 0 ˚C to RT 20 h. ii) Amine 10c, DCC, HOBt, $CH_2Cl_2$, 0˚C to RT iii) $Cs_2CO_3$, MeOH/$H_2O$, RT, 2 h. iv)TFA, $CH_2Cl_2$, 0 ˚C to RT 20 h.

Scheme 17:

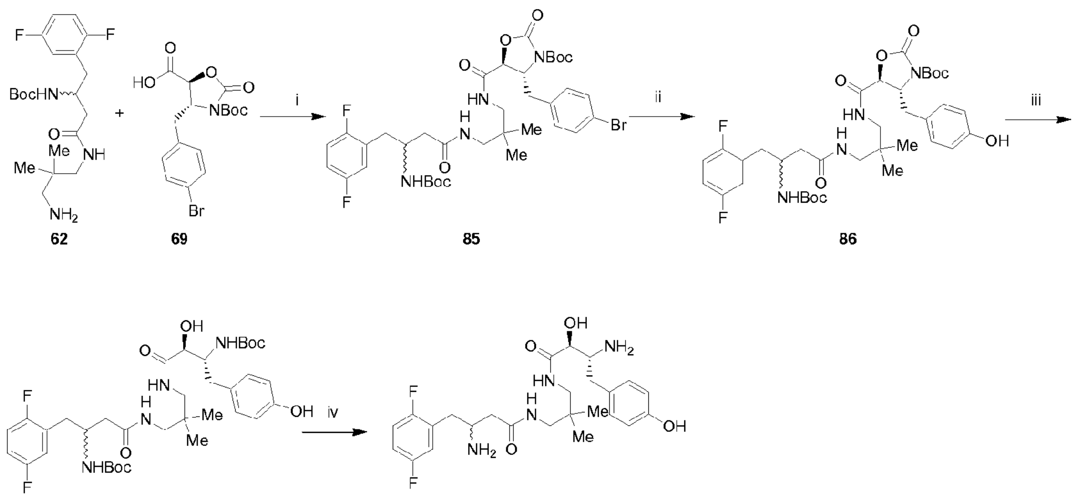


62 69 85 86

87 88

Reagents and conditions: i) DCC, HOBt, $CH_2Cl_2$, 0 ˚C to RT. ii) *t*-BuLi, $B(OMe)_3$, THF, -78 ˚C, $H_2O_2$, 0 ˚C to RT. iii) $Cs_2CO_3$, MeOH/$H_2O$, RT. iv) TFA, $CH_2Cl_2$, 0 ˚C to RT.

**Experimental procedures**

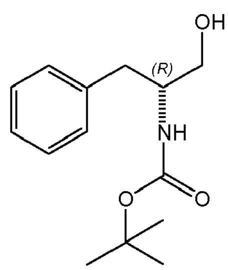

2 (Scheme 1): D-Phenylalanine 1 (5,00 g, 30,3 mmol) was added slowly to a suspension of $LiAlH_4$ (2,30 g, 60,5 mmol) in THF (100 mL). The resulting mixture was heated under reflux for 6 hours and then cooled to 0˚C. Excess $LiAlH_4$ was subsequently quenched with 10% NaOH aq. (5 mL) and water (5mL). The slurry was stirred at ambient temperature for 30 minutes and $Boc_2O$ (6,93 g, 31,8 mmol) in dichloromethane (30 mL) was added. The rection mixture was stirred over night and filtered over a short path of silica. Evaporation of the solvents provided 1 (7,53 g).

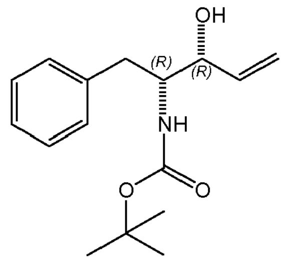

3 (Scheme 1): A solution of oxalylchloride (1,61 mL, 16,9 mmol) in dichloromethane (50 mL) was cooled with dry ice / acetone to -78˚C and a solution of dimethylsulfoxide (2,74 mL, 16,9 mmol) in dichloromethane (10 mL) was added over a period of 10 minutes. The mixture was stirred for 15 minutes before a solution of 2 (3,87 g, 15,4 mmol) in dichloromethane (20 mL) was added over a period of 20 minutes. The mixture was stirred for 30 minutes before ethyldiisopropyl-amine (10,6 mL, 61,6 mmol) was added. The temperature was slowly raised to -10˚C before the reaction mixture was cooled again to -78˚C. The cold mixture was transferred by the use of a double-ended needle to a mixture of vinylmagnesiumbromide (100 mL of a 1M solution in tetrahydrofurane, 100 mmol) and dichloromethane (100 mL). The rusulting mixture was stirred for 1 hours at ambient temperature and then quenched with $KHSO_4$ (200 mL, 1M solution in water). The phases were separated and the the aquaous phase was extracted with dichloromethane ( 3 x 50 mL). The combined organic phases were dried ($MgSO_4$) and evaporated. 3 (2,28 g) was isolated by flash-chromatography on silica (eluent: pentane / diethylether).

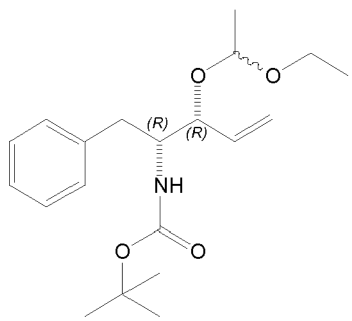

4 (Scheme 1): A solution of 3 (2,51 g, 9,05 mmol) in dichloromethane (20 mL) was stirred with ethylvinylether (8,7 mL, 90,5 mmol) and pyridinium-para-toluenesulfonate (229 mg, 0,905 mmol) for 210 minutes. The solvents were evaorated, the residue subjected to a flash-chromatography on silica (eluent: pentane / diethylether) and 4 (3,01 g) were obtained.

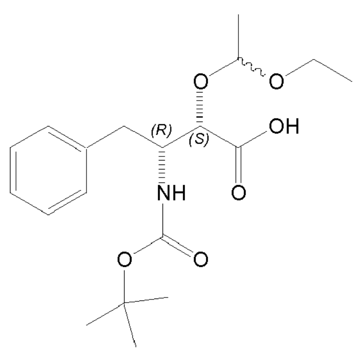

5 (Scheme 1): A solution of 4 (6,48 g, 18,5 mmol) in acetonitrile (55 mL), carbontetrachloride (55 mL) and water

(84 mL) was treated subsequently with Na-$HCO_3$ (10,1 g, 121 mmol) and $NaIO_4$ (21,8 g, 102 mmol). The slurry was stirred for 30 minutes and $RuCl_3$-hydrate (583 mg, 2,96 mmol) was added. After 3 days at ambient temperature, the mixture was diluted with water (400 mL) and washed with diethylether (2 x 200 mL). The aqueous phase was brought to pH < 1 with concentrated hydrochloric acid and extracted with dichloromethane (3 x 150 mL). The combined organic phases were dried ($MgSO_4$) and evaporated. 5 (4,09 g) was isolated by flash-chromatography on silica (eluent: pentane / diethylether).

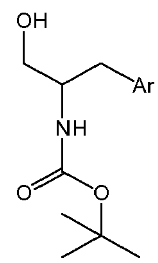

7 (Scheme 2): Amino acid 6 (1,0 eq.) was added slowly to a suspension of Li-$AlH_4$ (2,0 eq.) in THF (3 mL per mmol). The resulting mixture was heated under reflux for 6hoursand then cooled to 0˚C. Excess $LiAlH_4$ was subsequently quenched with 10% NaOH aq. (0,17 mL per mmol) and water (0,17 mL per mmol). The slurry was stirred at ambient temperature for 30 minutes and $Boc_2O$ (1,1 eq.) in dichloromethane (1 mL per mmol) was added. The rection mixture was stirred over night and filtered over a short path of silica. Evaporation of the solvents provided 7.

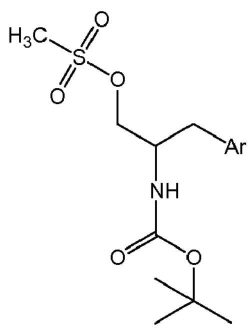

8 (Scheme 2): A solution of 7 (1,0 eq.) in dichloromethane (2 mL per mmol) was cooled to 0˚C and triethylamine (1,2 eq.) and methane sulfonylchloride (1,1 eq.) were added. The resulting mixture was stirred over night and washed with 1M $KHSO_4$. Evaporation of the solvent afforded 8 which was used without further purifaction.

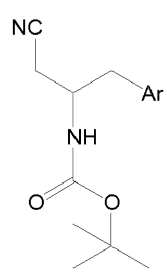

9 (Scheme 2): A mixture of 8 (1,0 eq.), sodium cyanide (2,0 eq.) and dimethylformamide (1,5 ml per mmol) were heated to 60˚C. After 18hoursthe solvent was evaporated and the residue was distributed between water and dichloromethane. The combined organic phases were dried ($MgSO_4$) and evaporated. 9 was isolated by flash-chromatography on silica (eluent: pentane / diethylether).

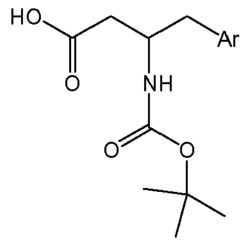

10a-c (Scheme 2): 9 (1,0 eq.) was heated with a solution of sodium hydroxide (1 g per mmol) in water (2 ml per mmol) under reflux for 6 h. After cooling to ambient temperature, the mixture was neutralized with concentrated hydrochloric acid. $NaHCO_3$ (2,4 eq.) and a solution of $Boc_2O$ (1,2 eq.) in 1,4-dioxane (3 ml per mmol) was added. After stirring for 18 h, the dioxane was evaporated and the residue was distributed between 1M hydrochloric acid and dichloromethane. The combined organic phases were dried ($MgSO_4$) and evaporated. 10a-c were purified by flash-chromatography on silica (eluent: pentane / diethylether).

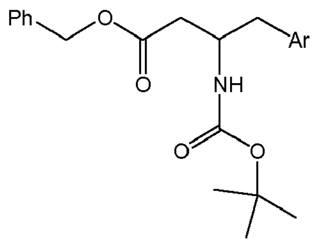

11 (Scheme 2): A solution of 10c (1,0 eq.), benzylic bromide (2,0 eq.) and $K_2CO_3$ (2 eq.) in dimethylformamide (3 mL per mmol) was stirred for 18 hours at ambient temperature. The solvent was evaporated and the residue subjected to flashchromatography on silica (eluent: pentane / diethylether) to afford 11.

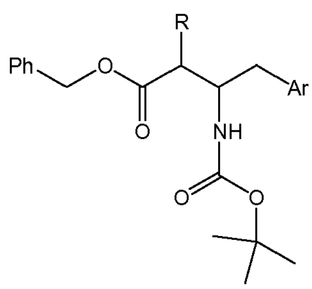

12 (Scheme 2): A solution of 11 (1,0 eq.) in tetrahydrofurane (10 mL per mmol) was cooled to -78˚C. LiHMDS (2,2 eq., 1M solution in tetrahydrofurane) was added and the resulting mixture was stirred at -78˚C for 2 h. Neat alkyl halide (1,5 eq.) was added and stirring was continued for additional 4 h. The reaction mixture was quenched by the addition of saturated aq. $NH_4Cl$. The phases were separated and the the aquaous phase was extracted with ethyl acetate. The combined organic phases were dried ($MgSO_4$) and evaporated. 12 was isolated by flash-chromatography on silica (eluent: pentane / diethylether).

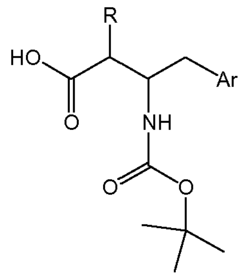

10d-e (Scheme 2): A solution of 12 (1,0 eq.) in methanol (6 mL per mmol) was treated with 10% palladium on charcoal (0,10 eq.). The suspension was stirred for 2 hours under an atmosphere of hydrogen at ambient pressure and temperature. Filtration and evaporation afforded 10d-e without further purification.

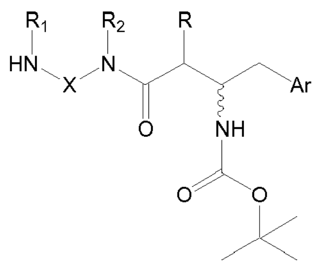

13: Method i (Scheme 3, symmetrical non-protected diamines ($HR^1N$-X-$NR^2H$): A solution of 10 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, $HR^1N$-X-$NR^2H$ (5 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases was dried ($MgSO_4$) and evaporated. 13 was isolated by flash-chromatography on silica (eluent: dichloromethane / methanol / triethylamine).

13: Method ii (Scheme 3, mono-benzyloxycarbonyl-protected diamines (BnO-$COR^1N$-X-$NR^2H$): A solution of 10 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, $BnOCOR^1N$-X-$NR^2H$ (1 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases was dried ($MgSO_4$) and evaporated. The residue was disolved in methanol (3 ml per mmol) and 10% palladium on charcoal (0,05 eq.) was added. The suspension was stirred for 16 hours under an atmosphere of hydrogen at ambient pressure. The crude product obtained by filtration and evaporation was subjected to flash-chromatography on silica (eluent: dichloromethane / methanol / triethylamine).

13: Method iii (Scheme 3, nitro amino compound ($O_2N$-X-$NR^2H$): A solution of 10 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, $O_2N$-X-$NR^2H$ (1,0 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. Na-$HCO_3$ and saturated aq. NaCl. The organic phases was dried ($MgSO_4$) and evaporated. The residue was disolved in methanol (5 ml per mmol), 10% palladium on charcoal (0,1 eq.) and ammonium formiate (4,6 eq.) were added. After 3 hours at ambient temperature the solvent was evaporated and the residue distributed between water and dichloromethane. The combined organic phases were dried ($MgSO_4$) and evaporated. 13 was purified by flash-chromatography on silica (eluent: dichloromethane / methanol / triethylamine).

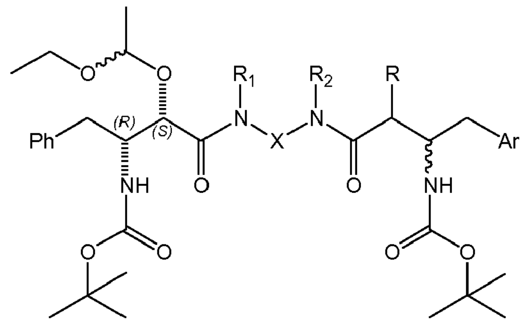

14 (Scheme 3): A solution of 5 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, 13 (1,0 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases was dried ($MgSO_4$) and evaporated. the crude 14 was used without further purification.

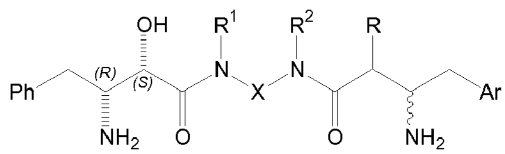

15 (Scheme 3): 14 (1,0 eq.) was dissolved in a solution of concentrated hydrochloric acid (37%, 0,1 ml per mmol) in ethanol (0,9 ml per mmol) and stirred for 6 hours at ambient temperature. The solvents were evaporated and the residue suspended in dichloromethane (10 ml per mmol). Sodium carbonate decahydrate (1 g per mmol) was added and the suspension was vigorously stirred for 30 minutes. After addition of $MgSO_4$ and filtration, the solvent was evaporated and 15 was isolated by flash-chromatography on silica (eluent: dichloromethane / methanol / triethylamine).

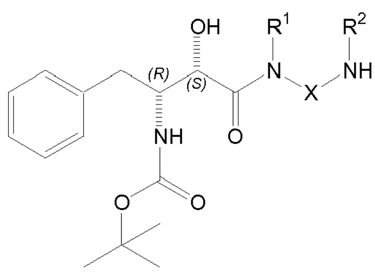

16 (Scheme 4): A solution of 5 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, $HR^1N$-X-$NR^2H$ (5 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phase was dried ($MgSO_4$) and evaporated. 16 was isolated by flash-chromatography on silica (eluent: dichloromethane / methanol / triethylamine).

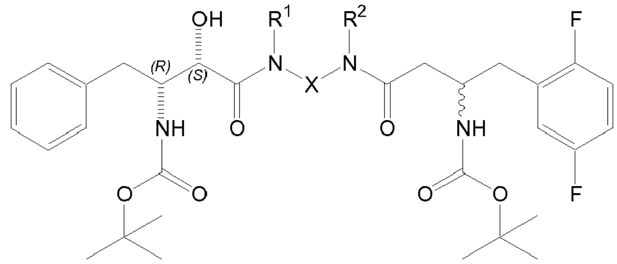

17 (Scheme 4): A solution of 10 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, 16 (1,0 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phase was dried ($MgSO_4$) and evaporated. 17 was isolated by flash-chromatography on silica (eluent: dichloromethane / diethylether).

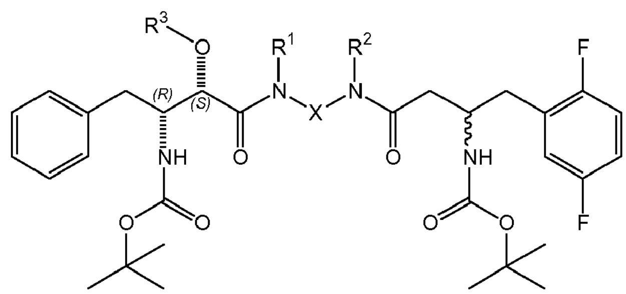

18: Method iii (Scheme 4, $R^3$ = alkanoyl): A solution of 17 (1,0eq.) in dichloromethane (10 mL per mmol) was treated with triethylamine (4 eq.), alkanoyl chloride (3eq.) and N,N'-dimethylaminopyridine (0,2 eq.). The mixture was stirred for 18 hours and was afterwards directly subjected to flash-chromatography on silica (eluent: dichloromethane / diethylether) to yield 18.

Method iv (scheme 4, $R^3$ = alkyl): A solution of 17 (1,0eq.) in N,N'-dimethylformamide (20 mL per mmol) was treated with potassium iodide (2 eq.), alkyl bromide (5 eq.) and sodium hydride (1,5 eq.). The reaction mixture was stirred for 2 hours at ambient temperature and subsequently distributed between water and diethylether. The organic phase was dried ($MgSO_4$) and evaporated. 18 was isolated by flash-chromatography on silica (eluent: dichloromethane/ diethylether).

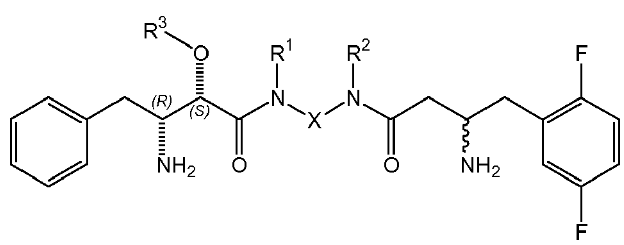

19 (Scheme 4): 18 (1,0 eq.) was dissolved in a mixture of dichloromethane (0,5 ml per mmol) and trifluoroacetic acid (0,5 ml per mmol) and stirred for 6 hours at ambient temperature. The solvents were evaporated and the residue suspended in dichloromethane (10 ml per mmol). Sodium carbonate decahydrate (1g per mmol) was added and the suspension was vigorously stirred for 30 minutes. After addition of $MgSO_4$ and filtration, the solvent was evaporated and 15 was isolated by flash-chromatography on silica (eluent: dichloromethane / methanol / triethylamine).

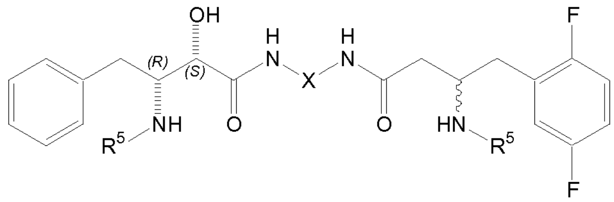

20: Method i (Scheme 5, $R^5$ = alkanoyl): To a solution of 15 (1,0 eq.) in tetrahydrofurane (20 mL per mmol) and water (5 ml per mmol) magnesium oxide (5 eq.) was added and the resulting suspension was stirred for 45 minutes prior to the addition of alkanoyl chloride (2 eq.). The mixture was stirred for 15 hours and subsequently distributed between water and ethyl acetate. The organic phase was dried ($MgSO_4$) and evaporated. 20 was isolated by flash-chromatography on silica (eluent: dichloromethane / diethylether).

Method ii (Scheme 5, $R^5$ = alkyl): To a solution of 15 (1,0 eq.) in tetrahydrofurane (20 mL per mmol) molecular sieve 3A (1g per mmol) and aldehyde (2,0 eq.) were added and the resulting suspension was stirred for 2 hours prior to the addition of sodium cyanoborohydride (4,0 eq.). The mixture was stirred for 15 hours and subsequently distributed between water and ethyl acetate. The organic phase was dried ($MgSO_4$) and evaporated. 20 was isolated by flash-chromatography on silica (eluent: dichloromethane / diethylether).

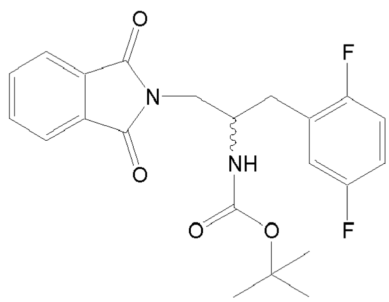

21 (Scheme 6): To a solution of 7 (1,0 eq.) in tetrahydrofurane (10 ml per mmol) triphenyphosphine (3,0 eq.) and phthalimide (1,5 eq.) were added and the mixture was cooled to 0˚C. Diethylazodicarboxylate (2,5 eq.) was added dropwise. Subsequently the cooling bath was removed and the reaction mixture was stirred for 16 h. Removal of the solvent and recrystallization from methanol afforded 21.

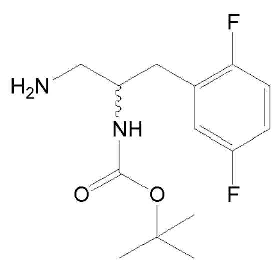

22 (Scheme 6): A solution of 21 (1,0 eq.) and hydrazine hydrat (1mL per mmol) in ethanol (20 mL per mmol) was heated under reflux for 2 hours. The precipitate was filtered off and the filtrate was diluted with ethyl acetate (160 ml per mmol). The solution was washed twice with water and once with saturated aq. NaCl. Drying ($MgSO_4$) and evaporation afforded 22, which was used without further purification.

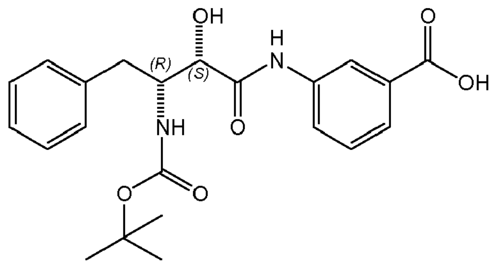

23 (Scheme 6): A solution of 5 (1,0 eq.) in dichloromethane (10 ml per mmol) was cooled to 0˚C and 1-hydroxybenzotriazole (1,05 eq.) and N,N'-dicyclohexylcarbodiimid (1,05 eq.) were added. After 1 hours at this temperature, 3-aminobenzoic acid (1,3 eq.) was added and the temperature was raised to ambient temperatur. The suspension was stirred for 18 hours and prior to filtration diluted with ethyl acetate (5 ml per ml dichloromethane). The filtrate was subsequently washed with 1M aq. $KHSO_4$ and saturated aq. NaCl. The organic phase was dried ($MgSO_4$) and evaporated. he obtained crude 23 was used without further purification.

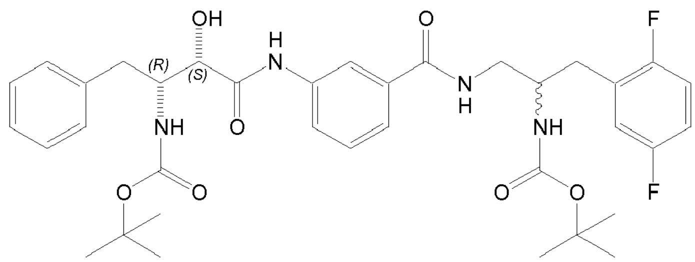

24 (Scheme 6): A solution of 23 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hour at this temperature, 22 (1,1 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phase was dried ($MgSO_4$) and evaporated. he obtained crude 24 was used without further purification.

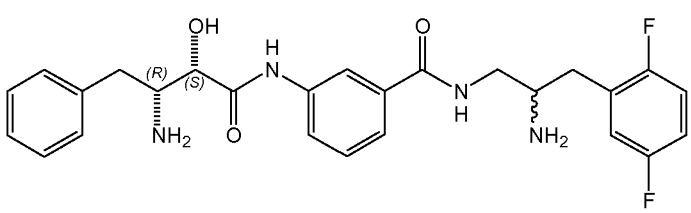

25 (Scheme 6): 24 (1,0 eq.) was dissolved in a mixture of dichloromethane (0,5 ml per mmol) and trifluoroacetic acid (0,5 ml per mmol) and stirred for 6 hours at ambient temperature. The solvents were evaporated and the residue suspended in dichloromethane (10 ml per mmol). Sodium carbonate decahydrate (1g per mmol) was added and the suspension was vigorously stirred for 30 minutes. After addition of $MgSO_4$ and filtration, the solvent was evaporated and 25 was isolated by flash-chromatography on silica (eluent: dichloromethane / methanol / triethylamine).

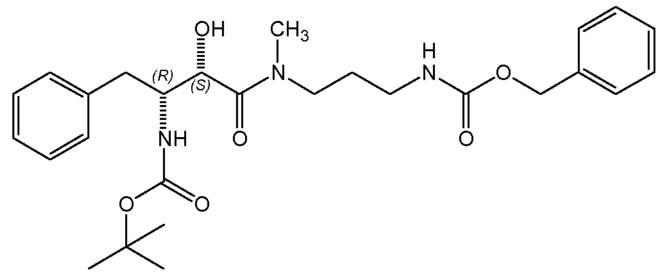

26: (Scheme 7): A solution of 5 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, BnOCOHN-$(CH_2)_3$-NHMe (1 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases was dried ($MgSO_4$) and evaporated. The residue was subjected to flash-chromatography on silica (eluent: dichloromethane / diethylether).

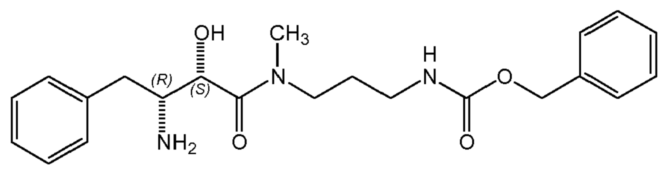

27 (Scheme 7): 26 (1,0 eq.) was dissolved in a mixture of dichloromethane (0,5 ml per mmol) and trifluoroacetic acid (0,5 ml per mmol) and stirred for 6 hours at ambient temperature. The solvents were evaporated and the residue suspended in dichloromethane (10 ml per mmol). Sodium carbonate decahydrate (1g per mmol) was added and the suspension was vigorously stirred for 30 minutes. After addition of $MgSO_4$ and filtration, the solvent was evaporated and 27 was isolated by flash-chromatography on silica (eluent: dichloromethane / methanol / triethylamine).

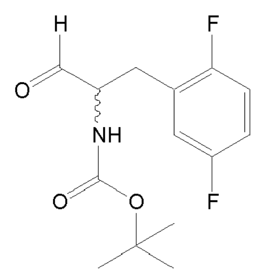

28 (Scheme 8): A solution of 7 (267 mg) in dichloromethane (8 ml) was treated at 0˚C with a solution of Dess-Martin-periodoindan (2,06 ml, 15% in dichloromethane) and subsequently stirred for 15 minutes at this temperature and for additional 10 minutes at ambient temperature. The reaction mixture was directly subjected to flash-chromatography on silica (eluent: pentane / diethylether) to yield 28 (213 mg).

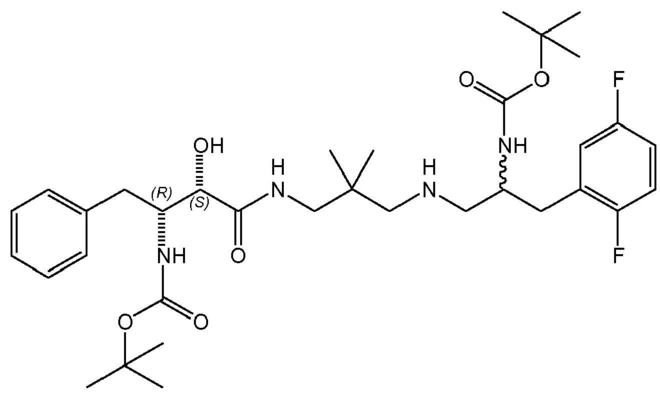

29 (Scheme 8): To a solution of 16 (77,1 mg) in tetrahydrofurane (3 mL) molecular sieve 3A (100 mg) and 28 (44,6 mg) were added and the resulting suspension was stirred for one hour prior to the addition of sodium cyanoborohydride (29,5 mg). The mixture was stirred for 24 hours and subsequently distributed between water and ethyl acetate. The organic phase was dried ($MgSO_4$) and the crude 20 (84 mg) was used without further purification.

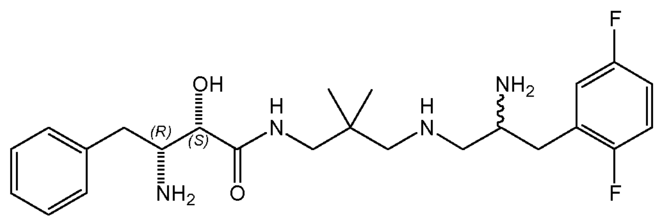

30 (Scheme 8): 29 (84 mg) was dissolved in a solution of concentrated hydrochloric acid (0,5 ml) in ethanol (4,5 ml) and stirred for 16 hours at ambient temperature. The solvents were evaporated and the residue suspended in dichloromethane (10 ml per mmol). Sodium carbonate decahydrate (1 g) was added and the suspension was vigorously stirred for 30 minutes. After addition of $MgSO_4$ and filtration, the solvent was evaporated and 30 was isolated by flash-chromatography on silica (eluent: dichloromethane / methanol / triethylamine).

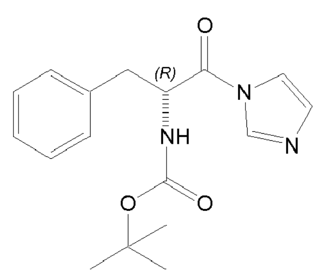

32 (Scheme 9): Boc-D-phenylalanine 31 (1,55 g) in tetrahydrofurane (10 ml) was treated at ambient temperature with N,N'-carbonyldiimidazole (1,04 g) and stirred for 3 hours. The mixture was diluted with diethylether (100 ml) and washed with water (2 x 50 ml) and brine (50 ml). Driying ($MgSO_4$) and evaporation of the solvents gave 32 (1,65 g).

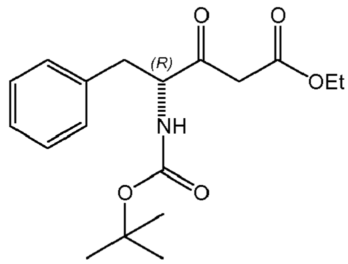

33 (Scheme 9): A solution of diisopropylamine (2,20 ml) in tetrahydrofurane (10 ml) was cooled to 0˚C, treated with n-butyllithium (6,28 ml, 2,5 M solution in tetrahydrofurane) and stirred at this temperature for 20 minutes. The solution was cooled to -78˚C and a solution of ethylacetate (1,54 ml) in tetrahydrofurane (3,5 ml) was added slowly. After one hour a solution of 32 (1,65 g) in tetrahydrofurane (10 ml) was added slowly. The resulting mixture was stirred for 2 hours at - 78˚C and then quenched with $KHSO_4$ (50 mL, 1M solution in water). The phases were separated and the the aquaous phase was extracted with diethylether ( 3 x 50 mL). The combined organic phases were dried ($MgSO_4$) and evaporated to yield 33 (1,75 g) without further purification.

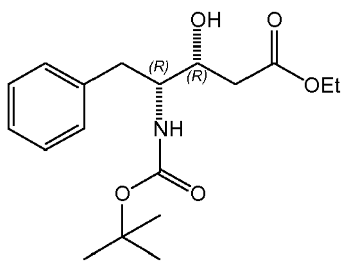

34 (Scheme 9): A solution of 33 (565 mg) in dichloromethane (17 ml) was cooled (-78˚C) and treated with $TiCl_4$ (0,222 ml). After 3 minutes pyridineborane-complex (0,187 ml) was added dropwise and the mixture was stirred for 30 minutes. Hydrochloric acid (20 ml, 1M) was added and the temperature was raised slowly to ambient temperature. The phases were separated and the the aquaous phase was extracted with dichloromethane ( 3 x 50 mL). The combined organic phases were dried ($MgSO_4$) and the solvent was evaporated. 34 (351 mg) was isolated by flash-chromatography on silica (eluent: pentane / diethylether).

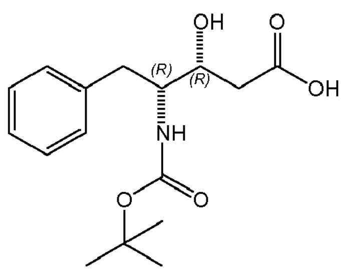

35 (Scheme 9): 34 (341 mg) was dissolved in methanol (8 ml) and $LiOH*H_2O$ (212 mg) was added. After 4 hours at room temperature the methanol was distilled off at reduced pressure and the residue was distributed between $KHSO_4$ (20 ml) and ethylacetate (20 ml). The phases were separated and the the aquaous phase was extracted with ethylacetate ( 2 x 50 mL). The combined organic phases were dried ($MgSO_4$) and the solvent was evaporated to yield 35 (299 mg) which was used without further purification.

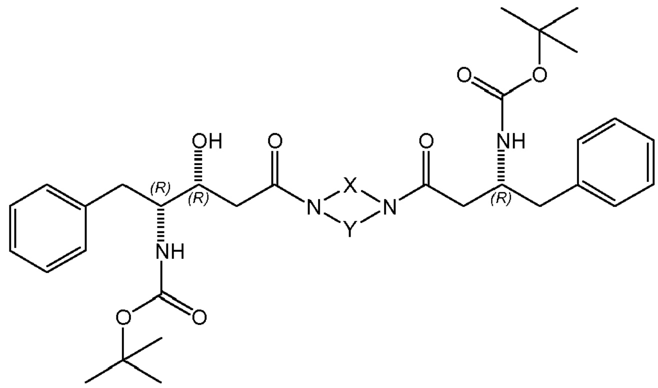

36 (Scheme 9): A solution of 35 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, 13 (1,0 eq.) was added and the temperature was raised to ambient temperatur. The suspension was stirred for 18 hours and was prior to filtration diluted with ethyl acetate (5 ml per ml dichloromethane). The filtrate was subsequently washed with saturated aq. $NaHCO_3$ and saturated aq. NaCl. The organic phases were dried ($MgSO_4$) and evaporated. The crude 14 was used without further purification.

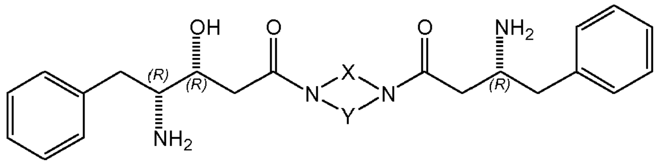

37 (Scheme 9): 36 (1,0 eq.) was dissolved in a solution of concentrated hydrochloric acid (37%, 0,1 ml per mmol) in ethanol (0,9 ml per mmol) and stirred for 6 hours at ambient temperature. The solvents were evaporated and the residue suspended in dichloromethane (10 ml per mmol). Sodium carbonate decahydrate (1 g per mmol) was added and the suspension was vigorously stirred for 30 minutes. After addition of $MgSO_4$ and filtration, the solvent was evaporated and 37 was isolated by flash-chromatography on silica (eluent: dichloromethane / methanol / triethylamine).

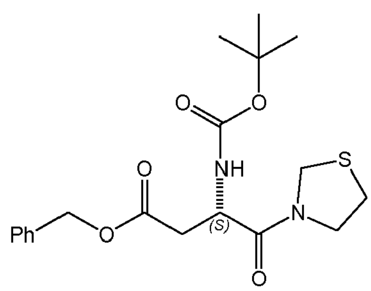

39 (Scheme 10): 38 (3,02 g) in dichloromethane (25 ml) was treated at ambient temperature with N,N'-carbonyldiimidazole (1,51 g) and stirred for 2 hours. Thiazolidine (0,736 ml) was added and the resulting mixture was stirred overnight. The solution was washed subsequently with $KHSO_4$ (20 ml, 1M) and saturated $NaHCO_3$, dried ($MgSO_4$) and evaporated. 39 (2,70 g) was isolated by flash-chromatography on silica (eluent: pentane / diethylether).

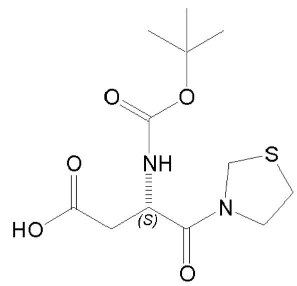

40 (Scheme 10): 39 (525 mg) was dissolved in methanol (10 ml) and LiOH*$H_2O$ (69,8 mg) was added. After 15 minutes at room temperature the methanol was distilled off at reduced pressure and the residue was partitioned between $KHSO_4$ (20 ml) and dichloromethane (20 ml). The phases were separated and the the aqueous phase was extracted with dichloromethane ( 2 x 20 mL). The combined organic phases were dried ($MgSO_4$) and the solvent was evaporated. 40 (257 mg) was purified by flash-chromatography on silica (eluent: pentane / diethylether).

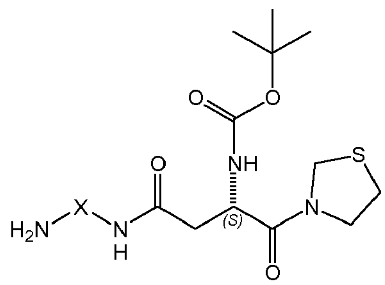

41 (Scheme 10): A solution of 10 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, $H_2N$-X-$NH_2$ (5 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases was dried ($MgSO_4$) and evaporated. 41 was isolated by flash-chromatography on silica (eluent: dichloromethane / methanol / triethylamine).

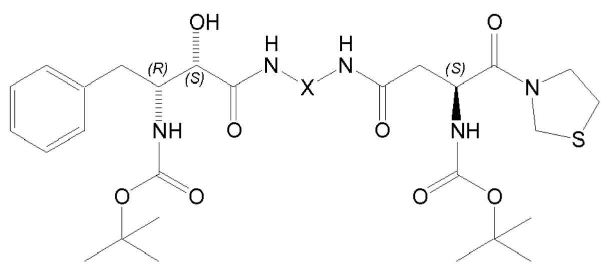

42 (Scheme 10): A solution of 16 (1,0 eq.) in dichloromethane (10 ml per mmol) was cooled to 0˚C and 1-hydroxybenzotriazole (1,3 eq.) and N,N'-dicyclohexylcarbodiimid (1,5 eq.) were added. After 1 hours at this temperature, 41 (1,0 eq.) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases were dried ($MgSO_4$) and evaporated. the crude 42 was used without further purification.

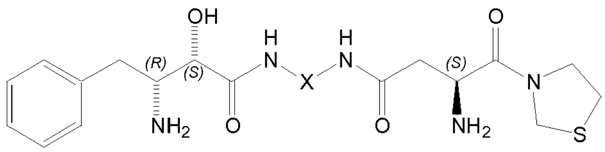

43 (Scheme 10): 42 (1,0 eq.) was dissolved in a solution of concentrated hydrochloric acid (37%, 1 ml per mmol) in ethanol (9 ml per mmol) and stirred for 18 hours at ambient temperature. The solvents were evaporated and the residue suspended in dichloromethane (10 ml per mmol). Sodium carbonate decahydrate (1g per mmol) was added and the suspension was stirred vigorously for 30 minutes. After addition of $MgSO_4$ and filtration, the solvent was evaporated and 43 was isolated by flash-chromatography on silica (eluent: dichloromethane / methanol / triethylamine).

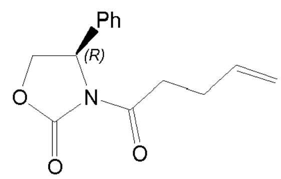

45 (Scheme 11): A solution of 44 (4,96 g) in tetrahydrofurane (200 ml) was cooled to -78˚C and n-butyllithium (12,2 ml, 2,5 M solution in tetrahydrofurane) was added slowly. The mixture was stirred for 45 minutes at this temperature followed by addition of pent-4-ene acid chloride (4,03 ml). After one hour, the mixture was warmed slowly to ambient temperature and and stirred for additional 15 hours. The reaction was quenched with water (100 ml) and the phases were separated. The aqueous phase was extracted twice with ethylacetate (100 ml) and the combined organic phases were subsequently washed with saturated aq. $NaHCO_3$ and brine. After drying ($MgSO_4$) and evaporation of the solvents, 45 (7,12 g) was purified by flash-chromatography on silica (eluent: pentane / diethylether).

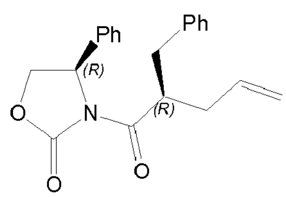

46 (Scheme 11): A solution of 45 (638 mg) in tetrahydrofurane (10 ml) was cooled to -78˚C and lithium bis (trimethylsilyl)amide (2,73 ml, 1,0 M solution in tetrahydrofurane) was added slowly. The mixture was stirred for two hours at this temperature followed by addition of a solution of benzylic bromide (0,62 ml) in tetrahydrofurane (3 ml). After two hours, the mixture was warmed slowly to ambient temperature and and stirred for additional 15 hours. The reaction was quenched with water (30 ml) and the phases were separated. The aqueous phase was extracted twice with ethylacetate (30 ml) and the combined organic phases were dried ($MgSO_4$) and the solvents were evaporated. 46 (654 mg) was isolated by flash-chromatography on silica (eluent: pentane / diethylether).

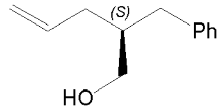

47 (Scheme 11): A suspension of $LiAlH_4$ (70,5 mg) in tetrahydrofurane (8 ml) was cooled to -78˚C and a solution of 46 (623 mg) in tetrahydrofurane (8 ml) was slowly added. After 30 minutes, the mixture was warmed slowly to ambient temperature and and stirred for one hour. The reaction was quenched with water (0,8 ml) and the filtered through a short

path of celite®. The solvents were evaporated and 47 (158 mg) was isolated by flash-chromatography on silica (eluent: pentane / diethylether).

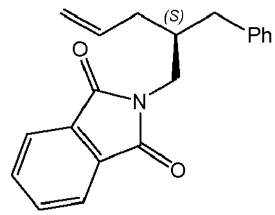

48 (Scheme 11):A solution of 47 (158 mg), phthalimide (145 mg) and triphenyl phosphine (295 mg) in tetrahydrofurane (9 ml) was cooled to 0˚C and treated with diethyl azodicarboxylate (0,155 ml). The mixture was warmed to ambient temperature and stirred for 16 hours. The solvent was evaporated and the residue was subjected to flash-chromatography on silica (eluent: pentane / diethylether) to yield 48 (276 mg).

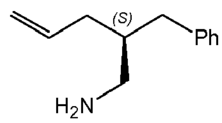

49 (Scheme 11): A solution of 48 (276 mg) in ethanol (10 ml) was treated with hydrazine hydrate (0,28 ml) an the resulting mixture was heated under reflux for three hours. After cooling to ambient temperature, the precipitate was filtered off. The filtrate was partitioned between saturated $NaHCO_3$ (20 ml) and dichloromethane (20 ml). The phases were separated and the the aqueous phase was extracted with dichloromethane (2 x 20 ml). The combined organic phases were dried ($MgSO_4$) and evaporation of the solvents gave 49 (142 mg) which was used without further purification.

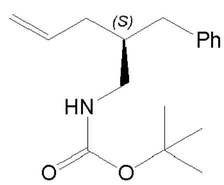

50 (scheme 11): 49 (1,07 g) was treated with $NaHCO_3$ (1,03 g) in water (12 ml) and di-*tert*-butyl dicarbonate (1,60 g) in 1,4-dioxane (12 ml). The mixture was stirred for five hours and subsequently partitioned between water (50 ml) and diethylether (50 ml). The phases were separated and the the aqueous phase was extracted with diethylether ( 2 x 50 mL). The combined organic phases were dried ($MgSO_4$). The solvent was evaporated and the residue was subjected to flash-chromatography on silica (eluent: pentane / diethylether) to yield 50 (1,67 g).

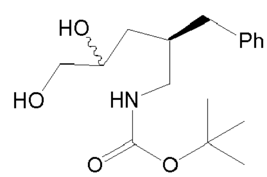

51 (Scheme 11): A solution of 50 (800 mg) in tetrahydrofurane (22 ml) and water (22 ml) was cooled to 0˚C

and N-methylmorpholine N-oxide (473 mg) and osmiumtetroxide (1 ml, 2,5 % solution in *t*-butanol) were added. After six hours, additional N-methylmorpholine N-oxide (473 mg) was added and the mixture was stirred for further 16 hours. The reaction mixture was partitioned between with $Na_2S_2O_3$ (10 ml, 1M in water) and ethyl acetate (50 ml). The phases were separated and the the aqueous phase was extracted with ethyl acetate ( 2 x 50 ml). The combined organic phases were dried ($MgSO_4$). The solvent was evaporated and the residue was subjected to flash-chromatography on silica (eluent: ethyl acetate) to yield 51 (880 mg).

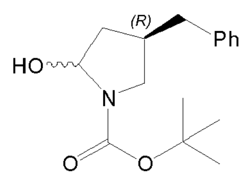

52 (Scheme 11): A solution of 51 (800 mg) in tetrahydrofurane (52 ml) was cooled to 0˚C and treated with $NaIO_4$ (929 mg) in water (5,2 ml). After 3,5 hours at 0˚C, the reaction mixture was partitioned between water (20 ml) and ethyl acetate (50 ml). The phases were separated and the the aqueous phase was extracted with ethyl acetate (2 x 50 ml). The combined organic phases were dried ($MgSO_4$). The solvent was evaporated and the residue was subjected to flash-chromatography on silica (eluent: pentane / ethyl acetate) to yield 52 (540 mg).

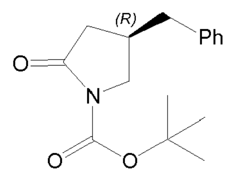

53 (Scheme 11): A solution of 52 (540 mg) in dichloromethane (6 ml) containing molecular sieves (800 mg) was treated with one portion of pyridinium chlorochromate (842 mg). The mixture was stirred over night at ambient temperature and then filtered through a short path of celite. Evaporation of the solvent and flash-chromatography on silica (eluent: dichloromethane / ethyl acetate) gave 53 (390 mg).

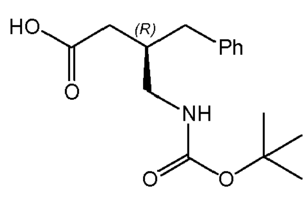

54 (Scheme 11): 53 (250 mg) was dissolved in tetrahydrofurane (5,8 ml) and aqueous LiOH (4,7 ml, 1M) was added. After two hours at ambient temperature the solvent was distilled off at reduced pressure and the residue was partitioned between hydrochloric acid (10 ml, 3 M) and diethylether (20 ml). The phases were separated and the the aqueous phase was extracted with diethylether (2 x 20 ml). The combined organic phases were dried ($MgSO_4$) and the solvent was evaporated. 54 (240 mg) was purified by flash-chromatography on silica (eluent: dichloromethane / methanol).

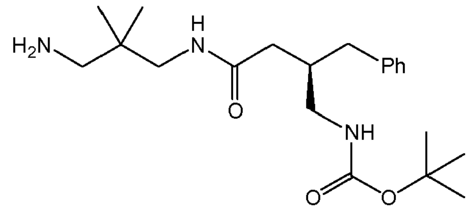

55 (Scheme 11): A solution of 54 (94,0 mg) in dichloromethane (5 ml) was cooled to 0˚C and 1-hydroxybenzotriazole (56,3 mg) and N,N'-dicyclohexylcarbodiimid (99,2 mg) were added. After 1 hours at this temperature, 1,3-diamino-2,2-dimethylpropane (164 mg) was added and the temperature was raised to ambient temperatur. The suspension was stirred for 18 hours and prior to filtration diluted with ethyl acetate (40 ml). The filtrate was subsequently washed with saturated aq. $NaHCO_3$ and saturated aq. NaCl. The organic phase was dried ($MgSO_4$) and evaporated. 55 (71,3 mg) was isolated by flash-chromatography on silica (eluent: dichloromethane / methanol / triethylamine).

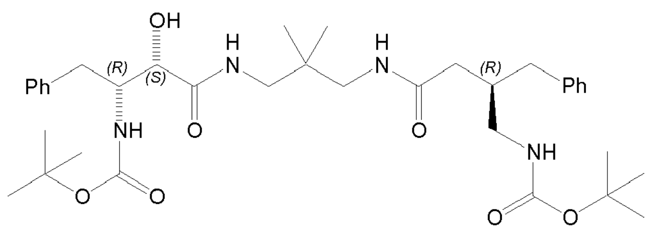

56 (Scheme 11): A solution of 5 (69,4 mg) in dichloromethane (3 ml) was cooled to 0˚C and 1-hydroxybenzotriazole (33,2 mg) and N,N'-dicyclohexylcarbodiimid (58,5 mg) were added. After 1 hours at this temperature, 55 (71,3 mg) was added and the temperature was raised to ambient temperatur. The suspension was stirred for 18 hours and prior to filtration diluted with ethyl acetate (40 ml). The filtrate was subsequently washed with saturated aq. $NaHCO_3$ and saturated aq. NaCl. The organic phases was dried ($MgSO_4$) and evaporated. The crude 56 (164 mg) was used without purification.

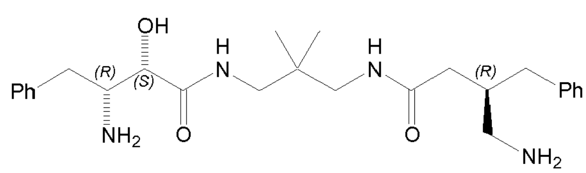

57 (Scheme 11): Crude 56 (164 mg) was dissolved in a solution of concentrated hydrochloric acid (37%, 1 ml) in ethanol (9 ml) and stirred for over night at ambient temperature. The solvents were evaporated and the residue suspended in dichloromethane (15 ml). Sodium carbonate decahydrate (1 g) was added and the suspension was vigorously stirred for 30 minutes. After addition of $MgSO_4$ and filtration, the solvent was evaporated and 57 (56,3 mg) was isolated by flash-chromatography on silica (eluent: dichloromethane / methanol).

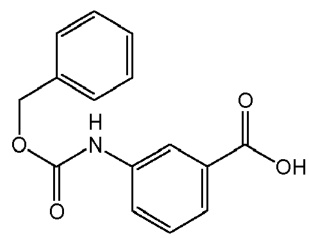

59 (Scheme 12): To m-aminobenzoic acid (3,52 g) in water (50 ml) were subsequently added $NaHCO_3$ (5,40 g) and a solution of benzyl chloroformate (4,51 ml) in 1,4-dioxane (30 ml). After 17 hours, the mixture was poured in $KHSO_4$ (1M, 70 ml) and was extracted with ethyl acetate. The combined organic phases were dried ($MgSO_4$) and the solvent was evaporated. Crystallisation from 2-propanol gave pure 59 (4,83 g).

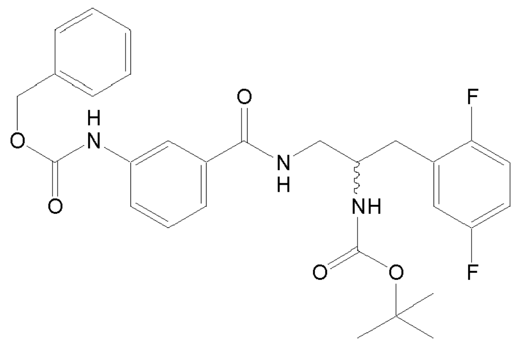

60 (Scheme 12): A solution of 59 (526 mg) in dichloromethane (15 ml) was cooled to 0˚C and 1-hydroxybenzotriazole (288 mg) and N,N'-dicyclohexylcarbodiimid (505 mg) were added. After 1 hours at this temperature, 1,3-diamino-2,2-dimethylpropane (71,3 mg) was added and the temperature was raised to ambient temperatur. The suspension was stirred for 18 hours and prior to filtration diluted with ethyl acetate (40 ml). The filtrate was subsequently washed with saturated aq. $NaHCO_3$ and saturated aq. NaCl. The organic phases was dried ($MgSO_4$) and evaporated. 60 (732 mg) was isolated by flash-chromatography on silica (eluent: dichloromethane / diethylether).

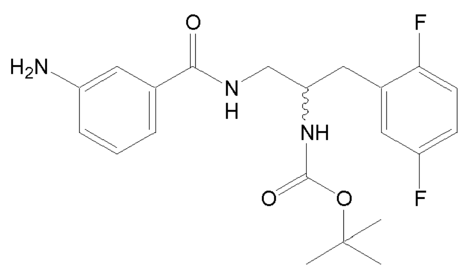

61 (Scheme 12): 60 (732 mg) was disolved in methanol (10 ml) and 10% palladium on charcoal (72 mg) was added. The suspension was stirred for 16 hours under an atmosphere of hydrogen at ambient pressure. The crude product obtained by filtration and evaporation was used without further purification.

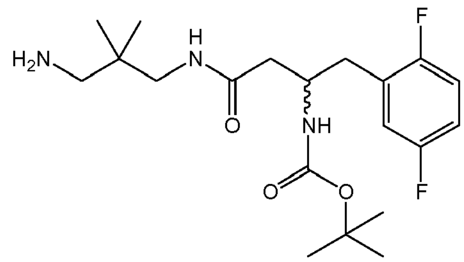

62 (Scheme 12): A solution of 10c (573 mg) in dichloromethane (15 ml) was cooled to 0˚C and 1-hydroxybenzotriazole (270 mg) and N,N'-dicyclohexylcarbodiimid (563 mg) were added. After 1 hours at this temperature, 1,3-diamino-2,2-dimethylpropane (1,20 ml) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases was dried ($MgSO_4$) and evaporated. 62 was isolated by flash-chromatography on silica (eluent: dichloromethane / methanol / triethylamine).

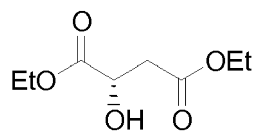

64 (Scheme 13): To an ethanol (0.8 ml/mmol) solution of the (S)-Malicacid 63 at room temperature catalytic amount of concentrated HCl was added and the reaction was refluxed for a period of 17 h. After this duration the reaction was bought to room temperature and the solvent was evaporated. The resulting suspension was diluted with $Et_2O$ and then washed with saturated aqueous $NaHCO_3$ solution (3 x 50 ml) and finally with brine solution. Combined organic portions were dried over $Na_2SO_4$, filtered and concentrated. The resulting oily product is subjected to vacuum distillation to yield (S)-Diethyl malate. Isolated yield (74%) [B.p 122 ˚C at 2 mm].

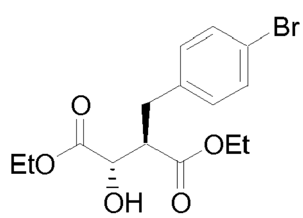

65 (Scheme 13): To a THF (3 ml/mmol) solution of the diethyl ester 64 (1 eq) cooled to -78 ˚C under nitrogen atmosphere LiHMDS (1 M in THF) (2.2 eq) was added drop wise in over a period of 40 minutes and the reaction was continued for a further 1 hour at the same temperature. After this the reaction was warmed to -30 ˚C and then re cooled to -78 ˚C. At this temperature 4-Bromo benzyl bromide (2.0 eq) was introduced in small portions to the reaction and continued at the same temperature for a further one hour and then warmed to - 30 ˚C in over a period of 22 hours. Reaction was cooled to -78 ˚C and quenched by the addition of saturated aqueous $NH_4Cl$ solution and then warmed to room temperature. Layers were separated and the aqueous phase was extracted with MTB ether. Combined organic portions were washed successively with $H_2O$ and then with brine solution, dried over $Na_2SO_4$, filtered and concentrated. The resulting oil was chromatographed on silica gel using Pentane/Diethyl ether (1:1). Isolated yield (91 %).

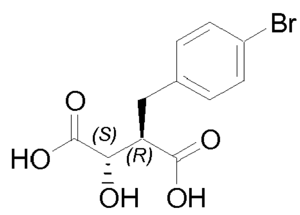

66 (Scheme 13): To a dioxane (2 ml/mmol) solution of the diethyl ester 65 (1 eq) at room temperature 1M aqueous NaOH (22 ml/mmol) was added and the reaction was heated to reflux. After 5 hours reaction was bought to room temperature and volatiles were evaporated. The resulting suspension was made acidic with 1M aqueous HCl and then extracted with EtOAc. Layers were separated and the aqueous phase was extracted with EtOAc (3 x 50 ml). Combined organic portions were dried over $MgSO_4$, filtered and concentrated. The resulting product is used without further purification in the next step of the sequence.

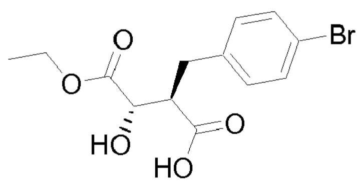

67 (Scheme 13): To the dicarboxylic acid 66 (1 eq) at 0 ˚C under nitrogen atmosphere TFAA (3.25 eq) was added drop wise. The reaction was continued for a further 30 minutes period then warmed to room temperature. After 4 hours the reaction was stopped and all the volatiles were evaporated. The resulting suspension was dissolved in EtOH (0.8 ml/mmol) and continued at room temperature. After completion of the reaction all the volatiles were evaporated and the resulting syrupy liquid was chromatographed on silica gel using Pentane. Isolated yield (77%).

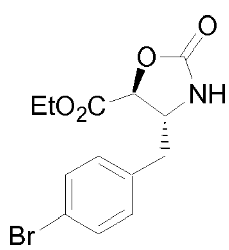

68 (Scheme 13): To the mono carboxylic acid 67 (1 eq) in benzene (3.4 ml/mmol) at room temperature under nitrogen atmosphere $Et_3N$ (1.15 eq) was added drop wise. After 10 minutes Diphenyl phosphonic azide (DPPA) (1.10 eq) was added drop wise to the reaction. The resulting suspension was refluxed for a further 4 hours period. After this the reaction was bought to room temperature and volatiles were evaporated. The resulting suspension was diluted with water and then extracted with EtOAc. Combined organic portions were washed with saturated aqueous $NaHCO_3$ solution and dried over $Na_2SO_4$ filtered and concentrated. The resulting product was chromatographed on silica gel using Pentane/ EtOAc (13:10). Isolated yield (68%).

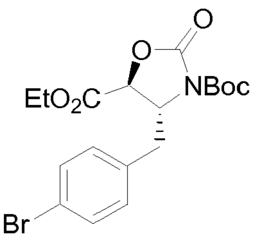

69 (Scheme 13): To the oxazolidinone 68 (1 eq) in $CH_2Cl_2$ (1 ml/mmol) at room temperature under nitrogen atmosphere $Boc_2O$ (1.1 eq), 4-DMAP (0.2 eq) and $Et_3N$ (1.2 eq) were added successively. After 4 hours reaction was quenched with $H_2O$ and then acidified with 1M aqueous HCl. Layers were separated and the aqueous phase was extracted with $CH_2Cl_2$. Combined organic portions were dried over $MgSO_4$, filtered and concentrated. Flash chromatography on silica gel was performed using Pentane/EtOAc (1:1). Isolated yield (95%).

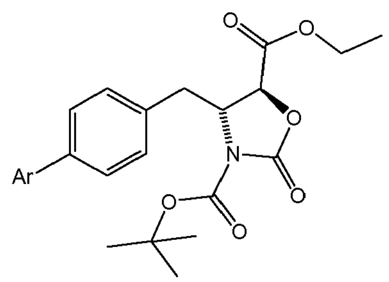

70 (Scheme 14): A mixture of 69 (1 eq), aryl boronic acid (2 eq), $K_2CO_3$ (3 eq) in toluene (3 ml per mmol) was degassed by bubbling $N_2$ through the suspension for 15 minutes. $Pd(PPh_3)_4$ (0,05 eq) were added and the the reaction mixture was heated at 90˚C for two hours. The chilled mixture was diluted with ethyl acetate (30 ml per mmol) and successively washed with hydrochloric acid (1M, 10 ml per mmol), saturated aqueous $NaHCO_3$ (10 ml per mmol) and brine (10 ml per mmol). The organic phase was dried ($MgSO_4$) and evaporated. 70 was isolated by flash-chromatography on silica (eluent: diethylether / pentane).

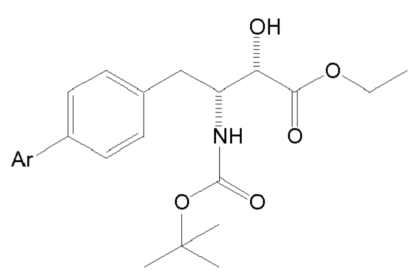

71 (Scheme 14): A mixture of 69 (1 eq), aryl boronic acid (2 eq), $K_2CO_3$ (3 eq) in toluene (3 ml per mmol) and ethanol (1,5 ml per mmol) was degassed by bubbling $N_2$ through the suspension for 15 minutes. $Pd(PPh_3)_4$ (0,05 eq) were added and the the reaction mixture was heated at 90˚C for two hours. The chilled mixture was diluted with ethyl acetate (30 ml per mmol) and successively washed with hydrochloric acid (1M, 10 ml per mmol), saturated aqueous Na-$HCO_3$ (10 ml per mmol) and brine (10 ml per mmol). The organic phase was dried ($MgSO_4$) and evaporated. 71 was isolated by flash-chromatography on silica (eluent: diethylether).

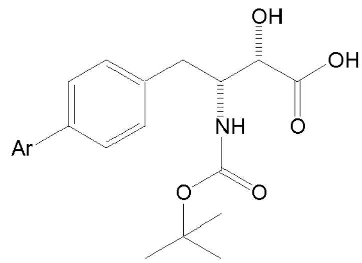

72a (Ar = phenyl, thiophen-3-yl, Scheme 14): 70 (1 eq) was dissolved in methanol / water (9:1, 10 ml per mmol) and LiOH*$H_2O$ (5 eq) was added. After five hours at room temperature the methanol was distilled off at reduced pressure and the residue was partitioned between hydrochloric acid (1M, 20 ml per mmol) and dichloromethane (20 ml per mmol). The phases were separated and the the aqueous phase was extracted with dichloromethane ( 2 x 20 mL). The combined organic phases were dried ($MgSO_4$) and the solvent was evaporated. 72a was used for further reactions without any purification.

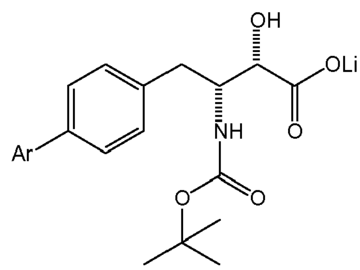

72b (Ar = 3-pyridyl, 5-pyrimidinyl, scheme 14): 70 (1 eq) was dissolved in methanol / water (9:1, 10 ml per mmol) and LiOH*$H_2O$ (2 eq) was added. After five hours at room temperature the methanol was distilled off at reduced pressure. 72b was used for further reactions without any purification.

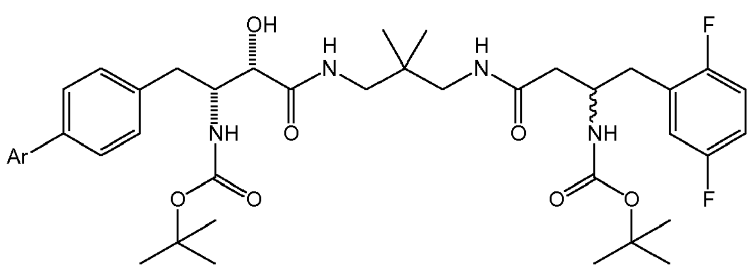

73 (Scheme 14): Crude 72 (1 eq) was dissolved or suspended in dichloromethane (10 ml per mmol) and cooled to 0˚C. 1-hydroxybenzotriazole (1,3 eq) and N,N'-dicyclohexylcarbodiimid (1,5 eq) were added. After 1 hours at this temperature, 62 (1 eq) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases was dried ($MgSO_4$) and evaporated. 73 was isolated by flash-chromatography on silica (eluent: dichloromethane / diethylether).

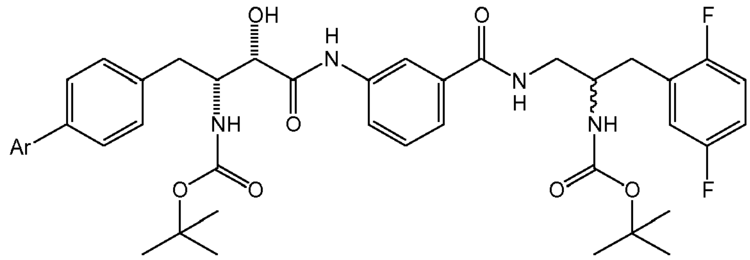

74 (Scheme 14): Crude 72 (1 eq) was dissolved or suspended in dichloromethane (10 ml per mmol) and cooled to 0˚C. 1-hydroxybenzotriazole (1,3 eq) and N,N'-dicyclohexylcarbodiimid (1,5 eq) were added. After 1 hours at this temperature, 61 (1 eq) was added and the temperature was raised to ambient temperatur. The suspension was stirred for 18 hours and prior to filtration diluted with ethyl acetate (5 ml per ml dichloromethane). The filtrate was subsequently washed with saturated aq. $NaHCO_3$ and saturated aq. NaCl. The organic phases was dried ($MgSO_4$) and evaporated. 74 was isolated by flash-chromatography on silica (eluent: dichloromethane / diethylether).

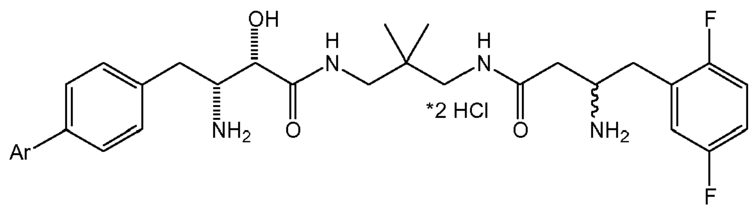

75 (Scheme 14): 73 (1,0 eq.) was dissolved in a solution of concentrated hydrochloric acid (37%, 0,1 ml per mmol) in ethanol (0,9 ml per mmol) and stirred for 6 hours at ambient temperature. The solvents were evaporated under reduced pressure and the residue was dissolved in the smallest possible amount of methanol. Upon addition of diethylether (5 to 10 times the amount of methanol) 75 was precipitated, filtered off and dried in high vacuum.

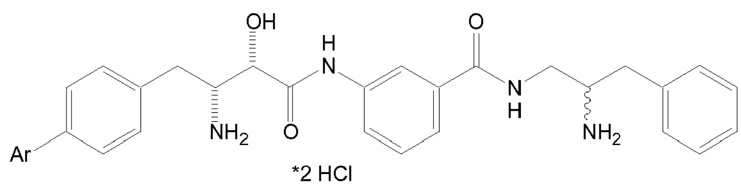

76 (Scheme 14): 74 (1,0 eq.) was dissolved in a solution of concentrated hydrochloric acid (37%, 0,1 ml per mmol) in ethanol (0,9 ml per mmol) and stirred for 6 hours at ambient temperature. The solvents were evaporated under reduced pressure and the residue was dissolved in the smallest possible amount of methanol. Upon addition of diethylether (5 to 10 times the amount of methanol) 76 was precipitated, filtered off and dried in high vacuum.

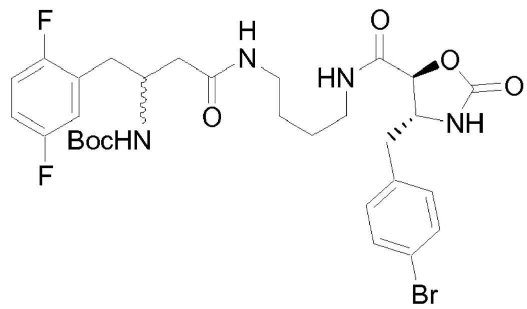

77 (Scheme 15): To a $CH_2Cl_2$ solution of the Oxazolidine carboxylic acid 68 (1 eq) cooled to 0 ˚C under nitrogen atmosphere HOBT (1.3 eq) and DCC (1.5 eq) were added and the reaction was continued for a further one hour at the same temperature. After this duration a $CH_2Cl_2$ solution of the amine (1.0 eq) was added drop wise and reaction was continued for a further 15 minutes and then warmed to room temperature. After 6 hours the reaction was diluted with EtOAc the solid by product was filtered and the filtrate was washed successively with saturated aqueous $NaHCO_3$ and then with brine solution. Combined organic portions were dried over $MgSO_4$, filtered and concentrated. Flash chromatography on silica gel was performed using $CH_2Cl_2$/MeOH (5:1).

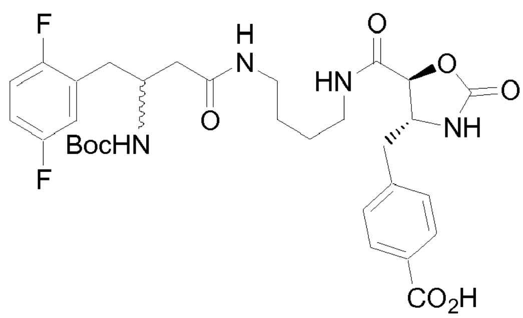

78 (Scheme 15): To a THF (6 ml/mmol) solution of the peptide 77 (1.0 eq) cooled to -78 ˚C under nitrogen atmosphere t-BuLi (1.7 M in Pentane) was added drop wise. After 30 minutes the nitrogen inlet was displaced with carbon dioxide gas inlet and continued for a further 15 minutes at -78 ˚C and then warmed to room temperature with a continuation of carbon dioxide passage. After 3 hours the reaction was quenched with the addition of water, volatiles were evaporated and the resulting suspension was acidified with acetic acid and extracted with EtOAc. Combined organic portions were dried over $MgSO_4$, filtered and concentrated. The resulting product is used without further purification in the next steps of the sequence.

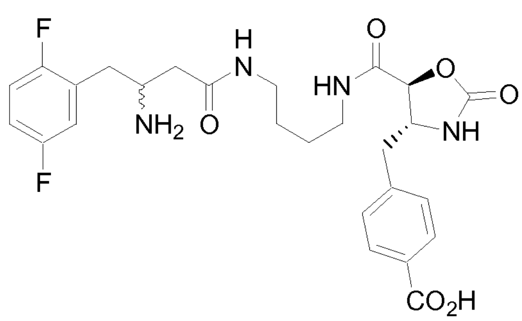

79 (Scheme 15): To a $CH_2Cl_2$ solution of the oxazolidinone carboxylic acid 78 (1 eq) cooled to 0 ˚C under nitrogen atmosphere trifluoro acetic acid (15 eq) was added drop wise. After 15 minutes the reaction was warmed to room temperature. After 18 hours reaction was stopped and volatiles were evaporated. The resulting product is used

without further purification in the next step of the sequence.

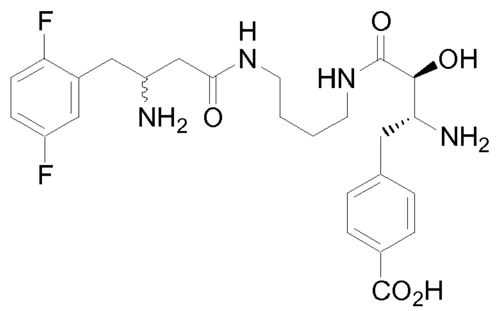

80 (Scheme 15): To an EtOH solution of the amino carboxylic acid 79 (1 eq) at room temperature 1 M aqueous NaOH (30 ml/mmol) was added and reaction was continued at room temperature. After 3 hours reaction was stopped and volatiles were evaporated. To the resulting residue was made acidic with 1 M aqueous HCl and then extracted with EtOAc. Combined organic portions were dried over $MgSO_4$, filtered and then concentrated to yield the target product 80.

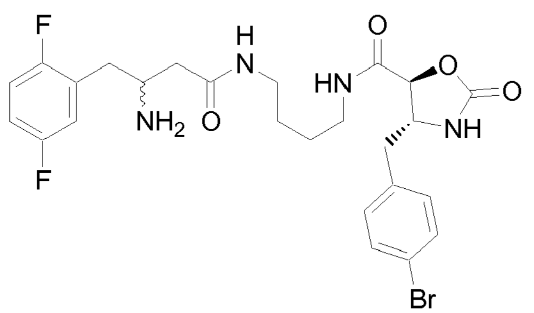

81 (Scheme 16): To a $CH_2Cl_2$ solution of the bromo oxazolidinone 77 (1 eq) cooled to 0 ˚C under nitrogen atmosphere trifluoro acetic acid (15 eq) was added drop wise, and the reaction was continued for a further 15 minutes and then warmed to room temperature. After 20 hours the reaction was stopped and volatiles were evaporated. Chromatographic purification of the residue on silica gel using $CH_2Cl_2$/MeOH (9:1) was performed. Isolated yield (94%).

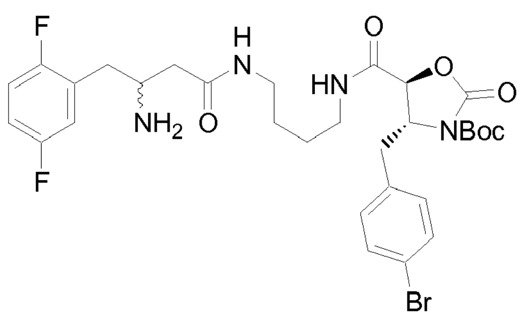

82 (Scheme 16): To a $CH_2Cl_2$ solution of the 4-Bromo-phenyl oxazolidinone 69 (1 eq) cooled to 0 ˚C under nitrogen atmosphere HOBT (1.3 eq) and DCC (1.5 eq) were added. After one hour a $CH_2Cl_2$ solution of the amine 10c (1.0 eq) was added drop wise. Reaction was continued for a further 30 minutes and then warmed to room temperature. After 22 hours the reaction was diluted with EtOAc, filtered and the filtrate was washed successively with saturated aqueous $NaHCO_3$ and then with brine solution. Combined organic portions were dried over $MgSO_4$ filtered and concentrated. Flash chromatography on silica gel was performed using $CH_2Cl_2$/MeOH (20:1). Isolated yield (79%).

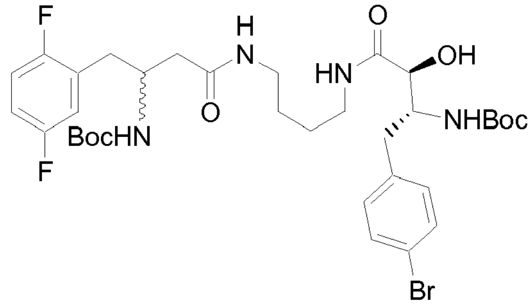

83 (Scheme 16): To the Bromo oxazolidinone (1 eq) in MeOH (5.2 ml/mmol) at room temperature under nitrogen atmosphere $Cs_2CO_3$ was added in one portion. After 2 hours the reaction was partitioned between EtOAc/$H_2O$ (6:1). Layers were separated and the aqueous phase was extracted with EtOAc. Combined organic portions were dried over $Na_2SO_4$, filtered and concentrated. The resulting product is used without further purification in the next step of the sequence.

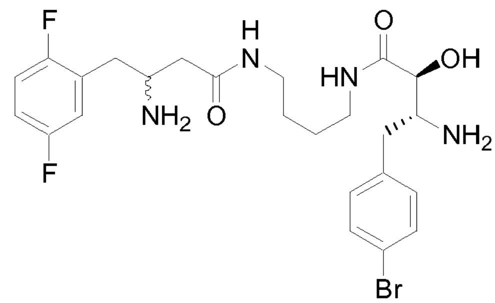

84 (Scheme 16): To a $CH_2Cl_0$ solution of the Bromo oxazolidinone (1 eq) cooled to 0 ˚C under nitrogen atmosphere trifluoro acetic acid (15 eq) was added drop wise and the reaction was continued for a further 15 minutes and then warmed to room temperature. After 20 hours the reaction was stopped and volatiles were evaporated. Flash chromatographic purification was performed on silica gel using $CH_2Cl_2$/MeOH (65:35).

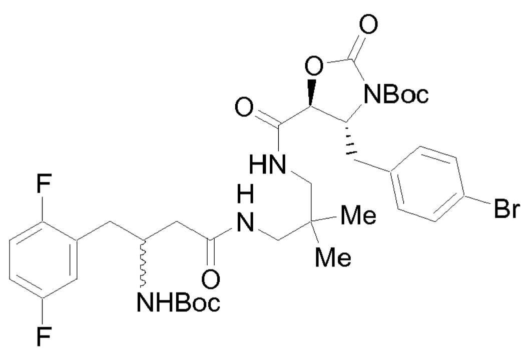

85 (Scheme 17): To a $CH_2Cl_2$ solution of the bromo oxazolidinone carboxylic acid (1 eq) cooled to 0 ˚C under nitrogen atmosphere HOBT (1.3 eq) and DCC (1.5 eq) were added. After one hour a $CH_2Cl_2$ solution of the amine (1.0 eq) was added drop wise. Reaction was continued for a further 15 minutes and then warmed to room temperature. After 6 hours the reaction was diluted with EtOAc, filtered and the filtrate was washed successively with saturated aqueous $NaHCO_3$ and then with brine solution. Combined organic portions were dried over $MgSO_4$ filtered and concentrated. Flash chromatography on silica gel was performed using $CH_2Cl_2$/MeOH (5:1).

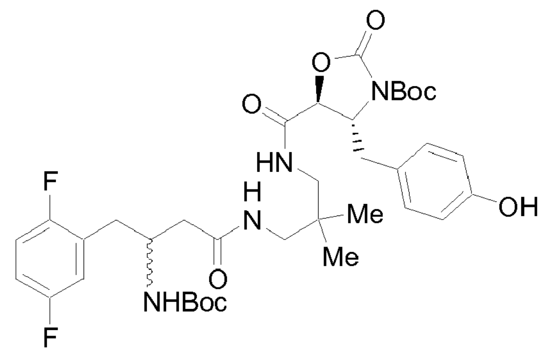

86 (Scheme17): To a THF (6 ml/mmol) solution of the peptide cooled to -78 ˚C under nitrogen atmosphere *t*-Buli (1.7 M in Pentane) (6.25 eq) was added drop wise. Immediately, the reaction turned into pale yellow. The reaction was continued for a further 15 minutes period then warmed to -20 ˚C and then continued for a further 15 minutes then re cooled to -78 ˚C. A THF (1.5 ml/mmol) solution of the $B(OMe)_3$ was added drop wise, and it was warmed to room temperature. After 40 minutes the reaction was cooled to 0 ˚C and a THF solution (1 ml/mmol) of the AcOH was added drop wise and continued for a further 15 minutes then a THF solution (1 ml/mmol) of 35% aqueous $H_2O_2$ was added drop wise. After 16 hours the reaction was quenched by the addition of saturated aqueous NaHCOand then extracted with EtOAc. Combined organic portions were dried over $MgSO_4$ filtered and concentrated. Flash chromatography on silica gel was performed using $Et_2O$/MeOH (15:1). Isolated yield (76%).

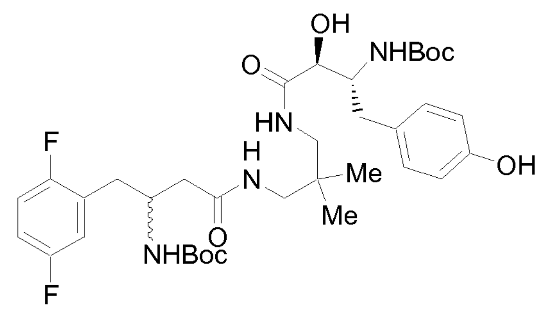

87 (Scheme 17):To the hydroxy oxazolidinone (1 eq) in MeOH (5.2 ml/mmol) at room temperature under nitrogen atmosphere $CS_2CO_3$ (2.85 eq) was added in one portion. After 22 hours the reaction was partitioned between EtOAc/$H_2O$ (6:1). Layers were separated and the aqueous phase was extracted with EtOAc. Combined organic portions were dried on $Na_2SO_4$ filtered and concentrated. The product is used without further purification in the next step of the sequence.

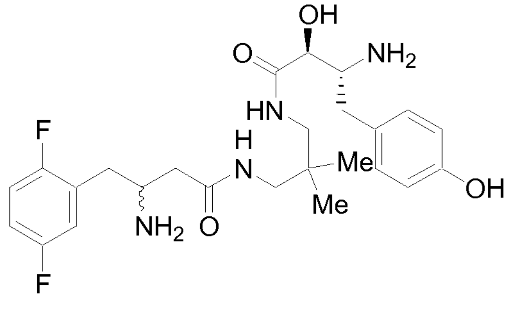

88 (Scheme 17): To a $CH_2Cl_2$ (30 ml/mmol) solution of the compound (1 eq) cooled to 0 ˚C under nitrogen atmosphere trifluoro acetic acid (15 eq) was added drop wise and reaction was continued for a further 15 minutes and then it was warmed to room temperature. After 20 hours the reaction was stopped and volatiles were evaporated. Flash

chromatographic purification on silica gel was performed using $CH_2Cl_2$/MeOH (7:3).

### Example 2

### Effects on croton oil-induced acute inflammation in SKH-1hr mice

Croton oil is prepared from the seeds of Croton tiglium, a tree belonging to the Euphorbiaceae family and native or cultivated in Asia. Externally applied, the oil is known to cause acute skin irritation, accompanied with the typical signs of acute inflammation: redness, heat and swelling. The symptoms reach maximum at 6 hours and decline after 24 - 48 hours after application thereof. This animal model is one of commonly acknowledged models for non-specific acute skin inflammation in mammals.

A typical experimental setting 20μl Croton oil 1% in acetone was applied topically to the anterior and posterior surfaces of the right ear to groups of three female and male SKH-1-hr nude mice aged between 8 and 12 weeks.

Ear swelling was measured by a micrometer gauge, and ear surface temperature was measured by a contact thermometer before and 6 h and 24 h after Croton oil application as an index of inflammation.

Test compounds were administered orally or by intra peritoneal injection each in different experiments. Dosages were 10 mg/kgBW for oral and peritoneal application. Phosphate buffered saline (PBS) or 1% Carboxymethyl cellulose (CMC) were used as vehicle control for intra peritoneal and oral administration, respectively.

Dexamethasone (5mg/kgBW) was applied for positive control.

Comparative tests were conducted with DPIV and/or APN inhibitors known from the prior art in the same way as the above tests.

Percent of changes in inflammatory parameters were calculated according to the formula:

$$\Delta It/\Delta Ic \times 100,$$

where Ic and It refers to increase of ear thickness (d [μm]) and temperature (t [°C]) in control and treated mice, respectively.

The inhibition results are shown in the subsequent Table 9.

Effects achieved with the comparative compounds known from the prior art are shown in the subsequent Table 10.

**Table 9:**

| Effects on Croton oil induced acute inflammation in SKH-1hr mice by specific compounds of the general formula (1) | | | | | | | | |
|---|---|---|---|---|---|---|---|---|
| Structure | Oral Application | | | | ip Application | | | |
| | Δt [%] | | Δd [%] | | Δt [%] | | Δd [%] | |
| | 6h | 24h | 6h | 24h | 6h | 24h | 6h | 24h |
| 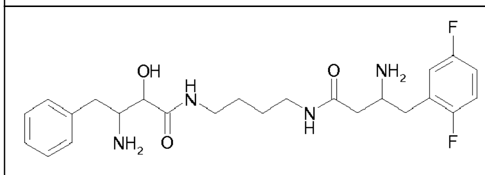 | 13 | 0 | 26 | 31 | 39 | 0 | 17 | 0 |
| 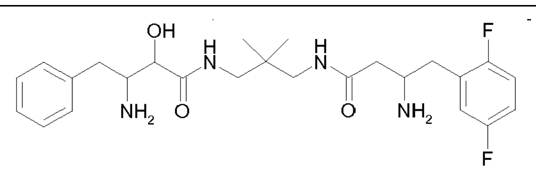 | 40 | 39 | 25 | 7 | 28 | 0 | 13 | 0 |
| 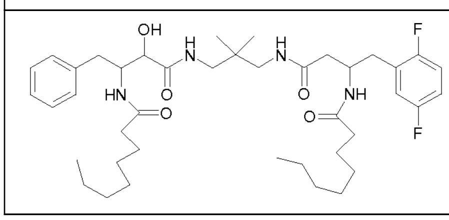 | 47 | 0 | 37 | 29 | 56 | 13 | 21 | 0 |

(continued)

| Effects on Croton oil induced acute inflammation in SKH-1hr mice by specific compounds of the general formula (1) | | | | | | | | |
|---|---|---|---|---|---|---|---|---|
| **Structure** | **Oral Application** | | | | **ip Application** | | | |
| | Δt [%] | | Δd [%] | | Δt [%] | | Δd [%] | |
| | 6h | 24h | 6h | 24h | 6h | 24h | 6h | 24h |
| 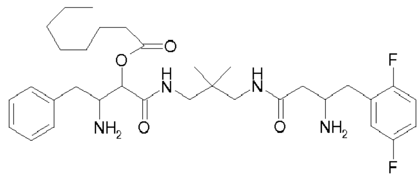 | 10 | 0 | 1 | 37 | 35 | 64 | 0 | 29 |
| 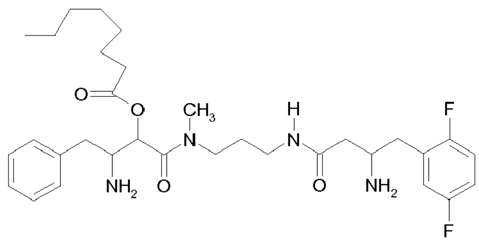 | 40 | 46 | 13 | 33 | 44 | 0 | 18 | 0 |
| 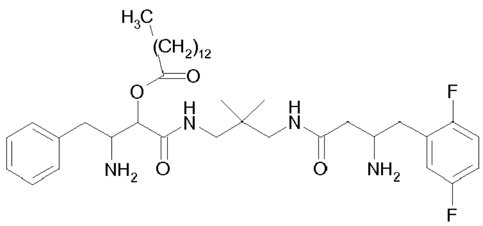 | 18 | 27 | 24 | 27 | 61 | 25 | 45 | 34 |
| 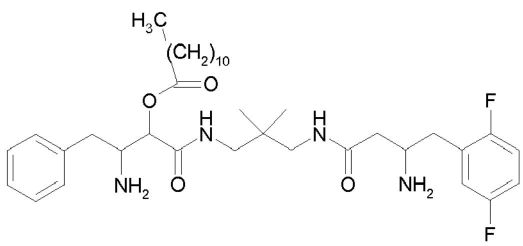 | 46 | 55 | 31 | 46 | 40 | 29 | 26 | 21 |
| 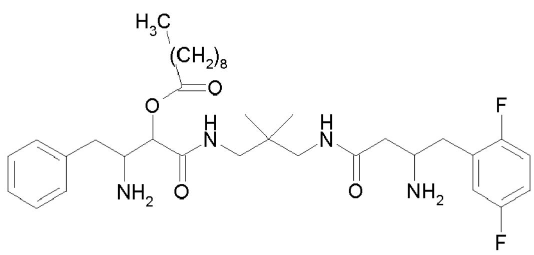 | 41 | 27 | 45 | 44 | 44 | 25 | 38 | 42 |

(continued)

| Effects on Croton oil induced acute inflammation in SKH-1hr mice by specific compounds of the general formula (1) | | | | | | | | |
|---|---|---|---|---|---|---|---|---|
| Structure | Oral Application | | | | ip Application | | | |
| | Δt [%] | | Δd [%] | | Δt [%] | | Δd [%] | |
| | 6h | 24h | 6h | 24h | 6h | 24h | 6h | 24h |
| 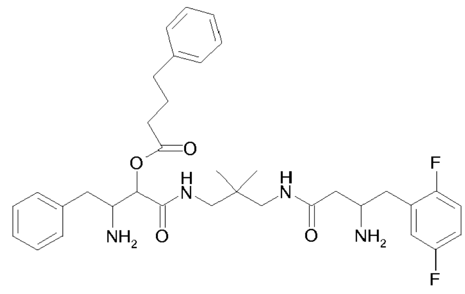 | 12 | 18 | 14 | 44 | 38 | 25 | 13 | 0 |
| 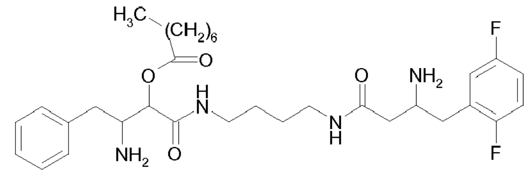 | 29 | 46 | 44 | 58 | 13 | 0 | 4 | 11 |
| 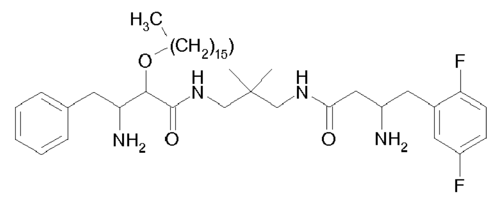 | 29 | 0 | 31 | 16 | 16 | 25 | 21 | 59 |
| 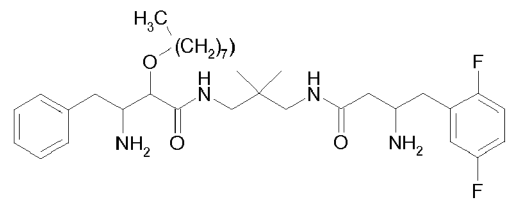 | 48 | 36 | 35 | 32 | 12 | 56 | 6 | 46 |
| 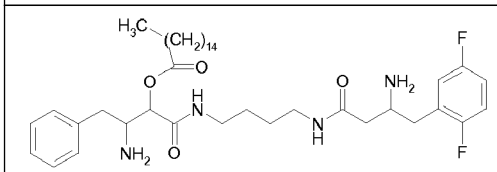 | | | | | 44 | 60 | 4 | 38 |
| 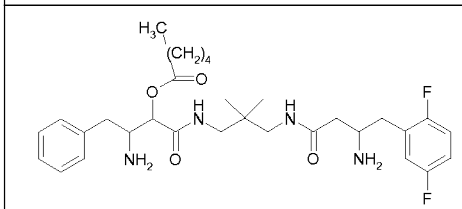 | 44 | 64 | 46 | 54 | 27 | 41 | 6 | 49 |
| 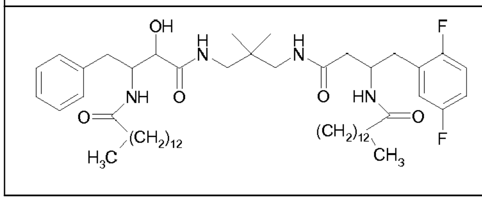 | 13 | 70 | 23 | 24 | 44 | 50 | 12 | 57 |

(continued)

| Effects on Croton oil induced acute inflammation in SKH-1hr mice by specific compounds of the general formula (1) | | | | | | | | |
|---|---|---|---|---|---|---|---|---|
| Structure | Oral Application | | | | ip Application | | | |
| | Δt [%] | | Δd [%] | | Δt [%] | | Δd [%] | |
| | 6h | 24h | 6h | 24h | 6h | 24h | 6h | 24h |
| 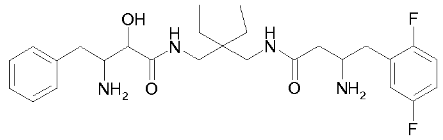 | 0 | 33 | 22 | 17 | 31 | 0 | 0 | 20 |
| 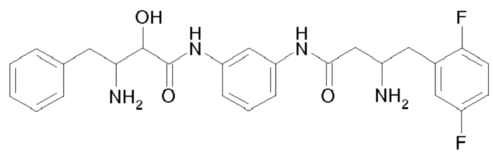 | 0 | 17 | 7 | 40 | 38 | 40 | 25 | 65 |
| 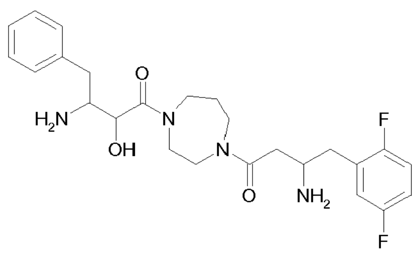 | 25 | 23 | 9 | 15 | 25 | 43 | 7 | 0 |
| 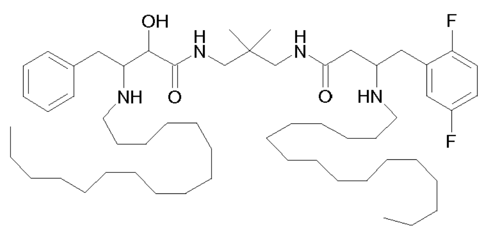 | 39 | 67 | 35 | 56 | 27 | 63 | 16 | 45 |
| 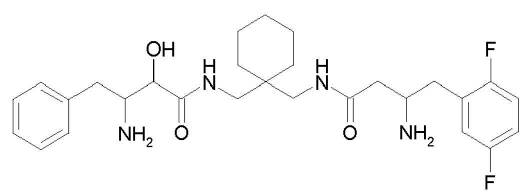 | 30 | 54 | 14 | 19 | | | | |
| 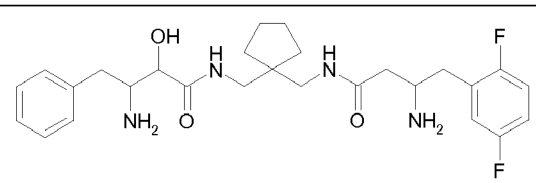 | 20 | 31 | 11 | 14 | 0 | 22 | 12 | 37 |
| 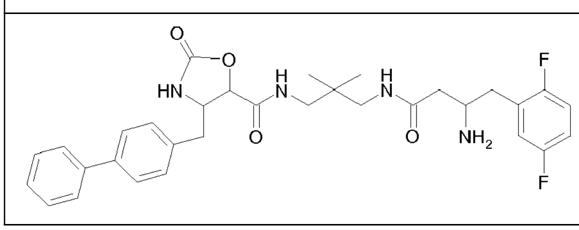 | 35 | 54 | 18 | 21 | | | | |

(continued)

| Effects on Croton oil induced acute inflammation in SKH-1hr mice by specific compounds of the general formula (1) | | | | | | | | |
|---|---|---|---|---|---|---|---|---|
| Structure | Oral Application | | | | ip Application | | | |
| | Δt [%] | | Δd [%] | | Δt [%] | | Δd [%] | |
| | 6h | 24h | 6h | 24h | 6h | 24h | 6h | 24h |
| 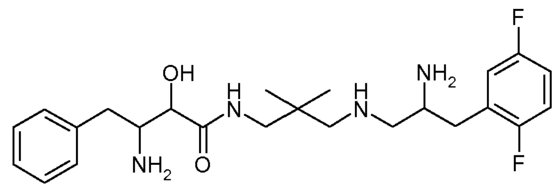 | 30 | 69 | 15 | 55 | 0 | 57 | 32 | 66 |
| 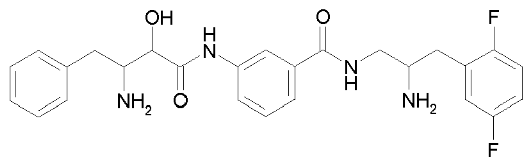 | 51 | 68 | 32 | 49 | 60 | 74 | 38 | 71 |
| 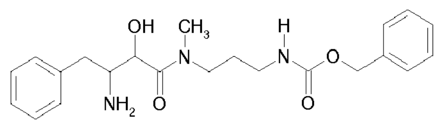 | 35 | 69 | 16 | 49 | 0 | 57 | 19 | 62 |
| 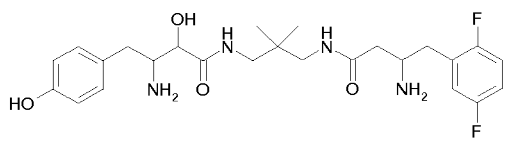 | 40 | 38 | 23 | 38 | 31 | 43 | 38 | 52 |
| 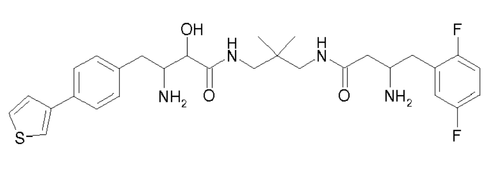 | 25 | 44 | 38 | 31 | 44 | 50 | 28 | 27 |
| 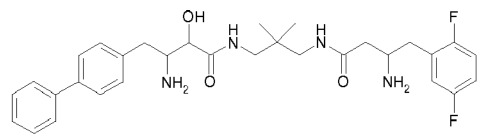 | 43 | 72 | 26 | 54 | 28 | 55 | 23 | 49 |
| 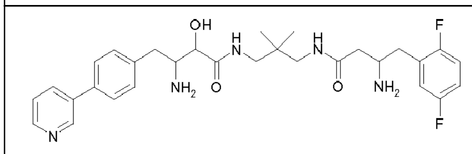 | 25 | 46 | 22 | 54 | 50 | 71 | 35 | 50 |
| 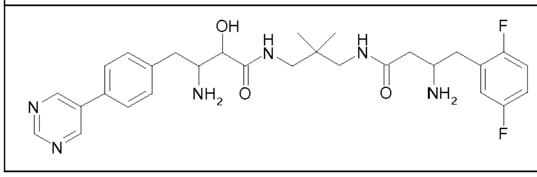 | 27 | 10 | 22 | 13 | 0 | 0 | 0 | 13 |

(continued)

| Effects on Croton oil induced acute inflammation in SKH-1hr mice by specific compounds of the general formula (1) | | | | | | | | |
|---|---|---|---|---|---|---|---|---|
| **Structure** | **Oral Application** | | | | **ip Application** | | | |
| | Δt [%] | | Δd [%] | | Δt [%] | | Δd [%] | |
| | 6h | 24h | 6h | 24h | 6h | 24h | 6h | 24h |
| 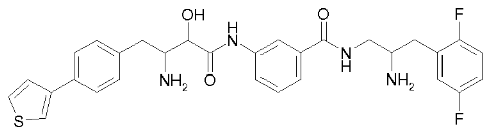 | 39 | 53 | 41 | 59 | 38 | 41 | 12 | 32 |
| 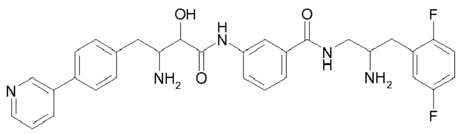 | 32 | 58 | 35 | 54 | 49 | 69 | 14 | 35 |

**Table 10:**

| Effects on Croton oil induced acute inflammation in SKH-1hr mice by control substance and known inhibitors of DPIV and APN | | | | | | | | |
|---|---|---|---|---|---|---|---|---|
| **Structure** | **Oral Application** | | | | **ip Application** | | | |
| | Δt [%] | | Δd [%] | | Δt [%] | | Δd [%] | |
| | 6h | 24h | 6h | 24h | 6h | 24h | 6h | 24h |
| Dexamethasone | 76 | 88 | 75 | 84 | 77 | 87 | 74 | 79 |
| Actinonin + Lys-z-Nitropyrrolidide | 56 | 76 | 38 | 67 | 58 | 72 | 33 | 50 |
| Actinonin | 50 | 58 | 17 | 36 | 40 | 69 | 34 | 30 |
| Lys-z-Nitropyrrolidide | 28 | 33 | 18 | 61 | 33 | 54 | 32 | 26 |
| RB-3014 | 50 | 76 | 0 | 25 | 42 | 73 | 34 | 70 |

## Example 3

### Inhibition of proliferation of human peripheral blood mononuclear cells and T lymphocytes

Peripheral blood mononuclear cells (PBMC) from healthy human volunteers were freshly isolated by density gradient centrifugation. T lymphocytes (T cells, Tc) were isolated from PBMC fractions via nylon wool adherence. Cells were cultured in standard cell culture media and stimulated by addition of 1 μg/ml Phytohemagglutinine for 48 hours in 96 well flat-bottom microtitre plates. Test compounds were added at concentration ranges from 0.1-250 μM over the total assay period.

DNA synthesis of proliferating PBMC was assessed by incorporation of nucleotide analogue bromdesoxyuridine (BrdU) and subsequent detection of optical density (OD) at 450nm.

DNA synthesis of proliferating T cells was determined by incorporation of radio labelled tritium thymidine and subsequent radio detection.

Data output was based on raw data and calculation of relative proliferation response in relation to PHA-activated T cells in the absence of test compound (100% control).

The IC50 value of proliferation suppression was assessed by graphical evaluation.

**Table 11**

| Inhibition of proliferation of mononuclear cells (MNC) and T cells (Tc) by specific compounds of the general formula (1) | | |
|---|---|---|
| **Structure** | **MNC IC50 [μM]** | **T cells IC50 [μM]** |
| 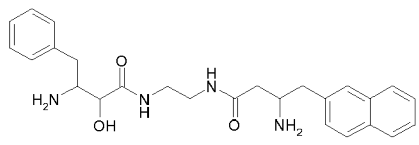 | 30.4 | 34.0 |
| 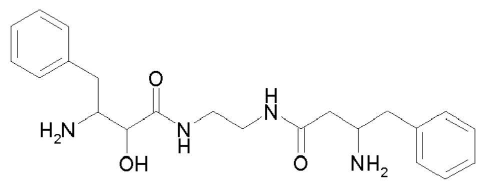 | 122.0 | 200.0 |
| 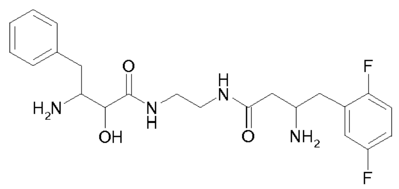 | 200.0 | 200.0 |
| 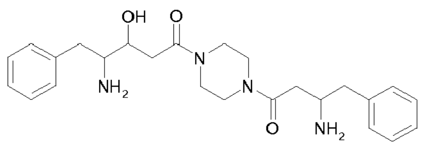 | 135.0 | 156.7 |
| 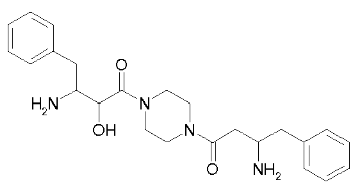 | 140.0 | 156.7 |
| 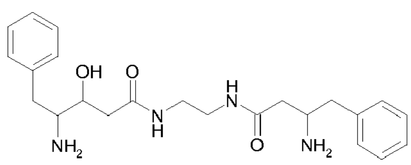 | 120.0 | 156.7 |
| 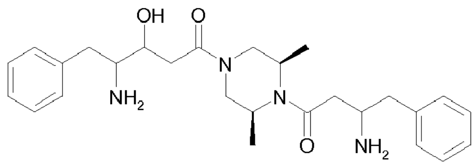 | 84.5 | 146.9 |
| 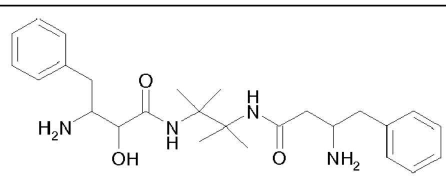 | 46.9 | 16.1 |

(continued)

| Inhibition of proliferation of mononuclear cells (MNC) and T cells (Tc) by specific compounds of the general formula (1) | | |
|---|---|---|
| **Structure** | **MNC IC50 [μM]** | **T cells IC50 [μM]** |
| 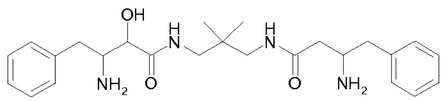 | 200.0 | 200.0 |
| 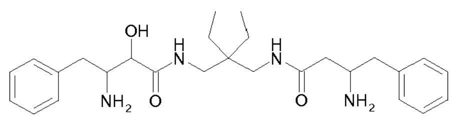 | 49.1 | 14.8 |
| 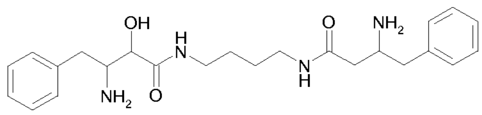 | 200.0 | 200.0 |
| 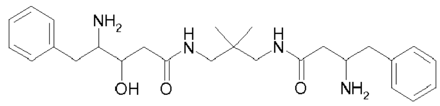 | 200.0 | 200.0 |
| 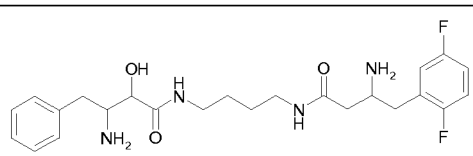 | 134.0 | 200.0 |
| 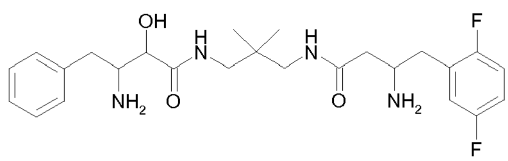 | 200.0 | 200.0 |
| 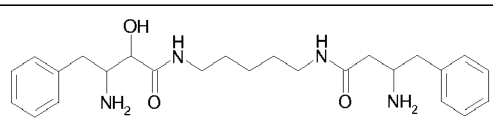 | 200.0 | 200.0 |
| 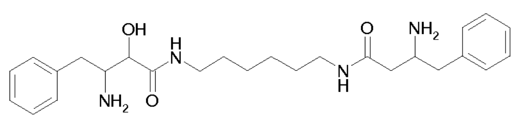 | 200.0 | |
| 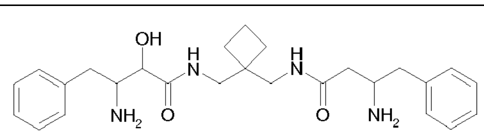 | 200.0 | 156.7 |
| 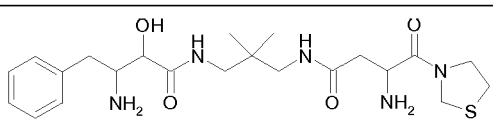 | 200.0 | 200.0 |
| 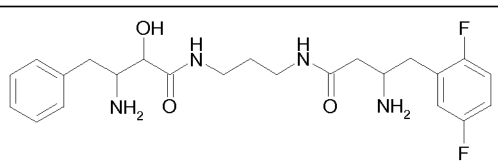 | 122.0 | 70.0 |

(continued)

| Inhibition of proliferation of mononuclear cells (MNC) and T cells (Tc) by specific compounds of the general formula (1) | | |
|---|---|---|
| Structure | MNC IC50 [μM] | T cells IC50 [μM] |
| 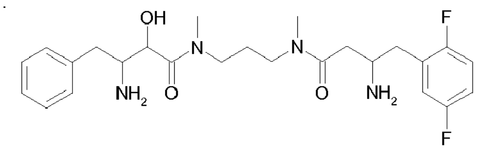 | 122.0 | 70.0 |
| 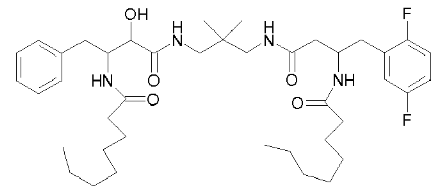 | 10.9 | 8.2 |
| 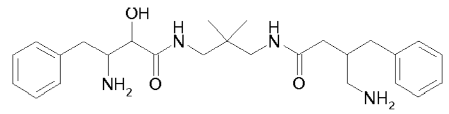 | 64.4 | 60.7 |
| 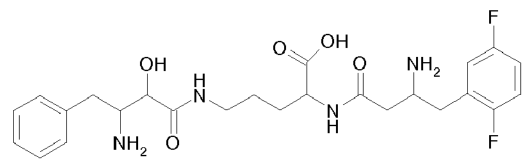 | 70.0 | 113.3 |
| 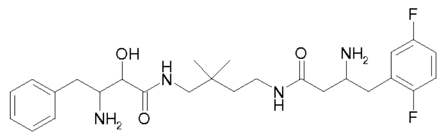 | 63.3 | 156.7 |
| 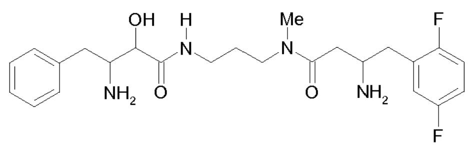 | 156.7 | 200.0 |
| 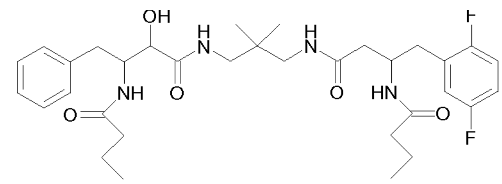 | 54.5 | 53.9 |
| 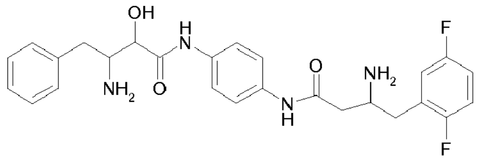 | 14.8 | 14.6 |
| 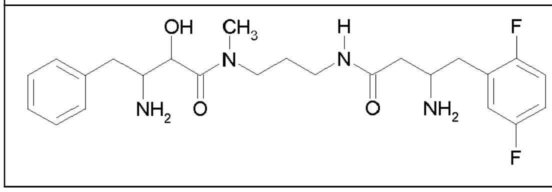 | | 200.0 |

(continued)

| Inhibition of proliferation of mononuclear cells (MNC) and T cells (Tc) by specific compounds of the general formula (1) | | |
|---|---|---|
| **Structure** | **MNC IC50 [μM]** | **T cells IC50 [μM]** |
| 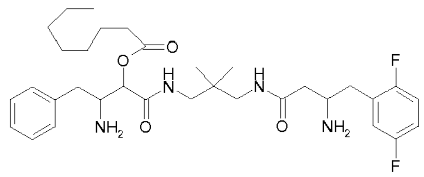 | 5.8 | 7.4 |
| 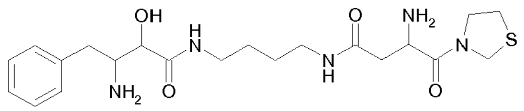 | 28.3 | 35.2 |
| 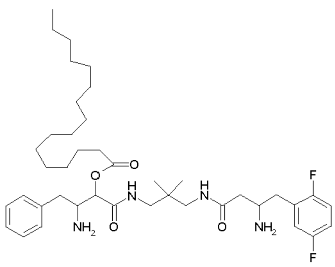 | 35.8 | 34.4 |
| 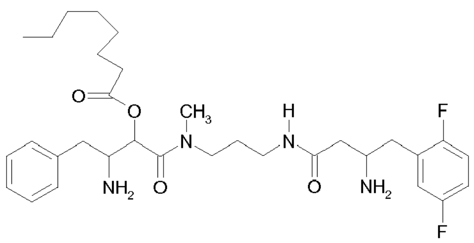 | 11.1 | 9.5 |
| 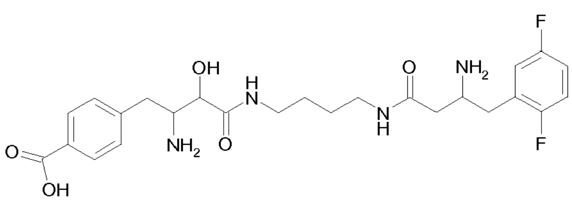 | 130.0 | 112.0 |
| 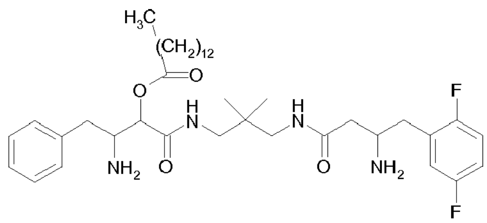 | 1.9 | 2.0 |

(continued)

| Inhibition of proliferation of mononuclear cells (MNC) and T cells (Tc) by specific compounds of the general formula (1) | | |
|---|---|---|
| **Structure** | **MNC IC50 [μM]** | **T cells IC50 [μM]** |
| 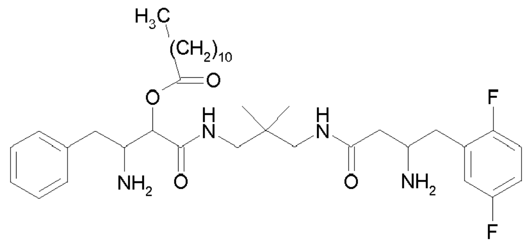 | 1.7 | 2.0 |
| 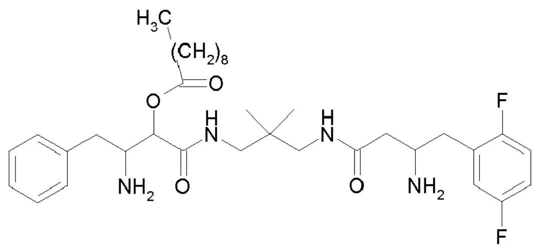 | 2.5 | 3.1 |
| 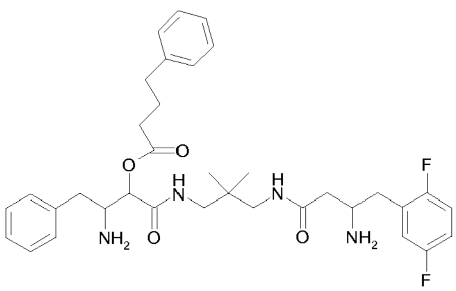 | 2.5 | 3.0 |
| 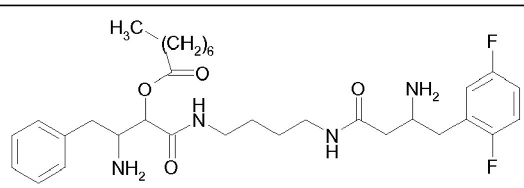 | 10.6 | 19.0 |
| 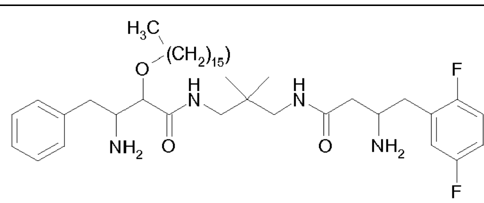 | 2.0 | 2.1 |
| 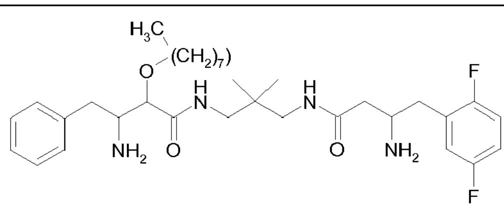 | 2.5 | 1.7 |

(continued)

| Inhibition of proliferation of mononuclear cells (MNC) and T cells (Tc) by specific compounds of the general formula (1) | | |
|---|---|---|
| Structure | MNC IC50 [μM] | T cells IC50 [μM] |
| 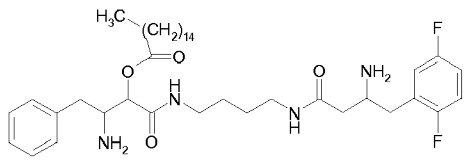 | 2.1 | 4.1 |
| 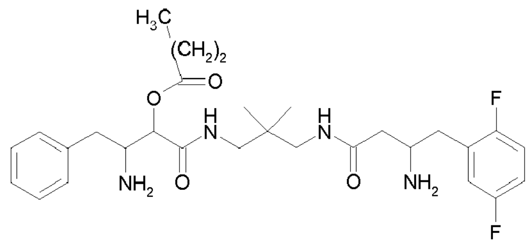 | 95.1 | 55.3 |
| 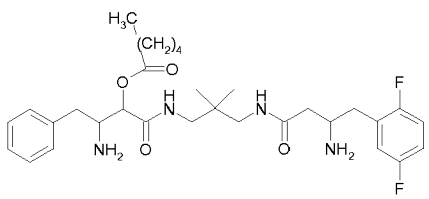 | 14.3 | 11.3 |
| 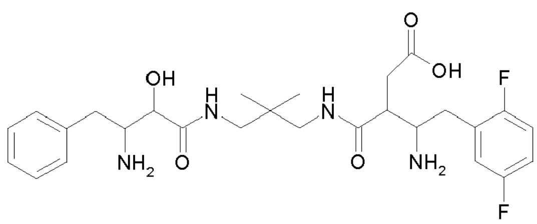 | | 88.3 |
| 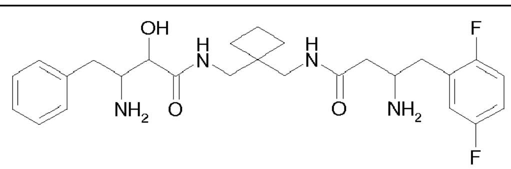 | 29.9 | 19.2 |
| 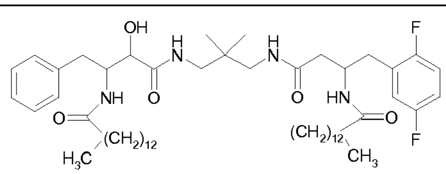 | | 98.7 |
| 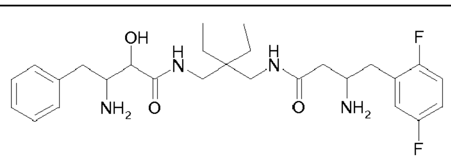 | | 7.2 |
| 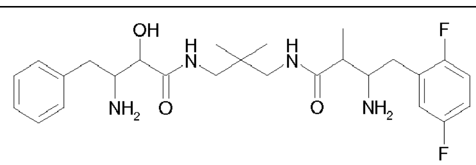 | 41.6 | 38.9 |

(continued)

| Inhibition of proliferation of mononuclear cells (MNC) and T cells (Tc) by specific compounds of the general formula (1) | | |
|---|---|---|
| **Structure** | **MNC IC50 [μM]** | **T cells IC50 [μM]** |
| 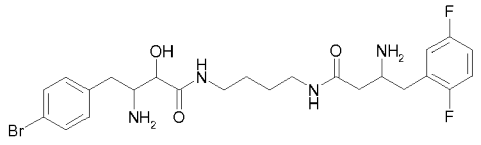 | 200.0 | 58.1 |
| 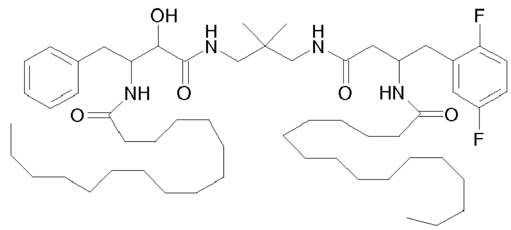 | 200.0 | 97.5 |
| 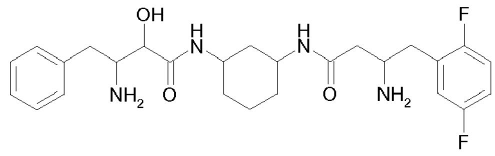 | 200.0 | 113.3 |
| 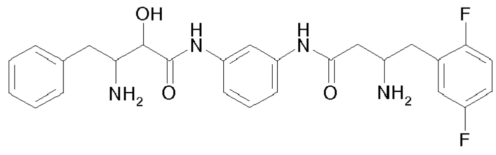 | 9.1 | 9.4 |
| 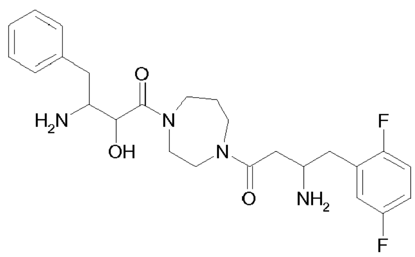 | 200.0 | 83.5 |
| 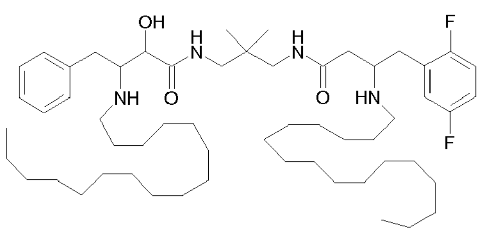 | 10.0 | 11.0 |
| 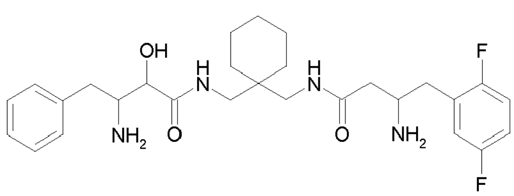 | 7.4 | 7.0 |

(continued)

| Inhibition of proliferation of mononuclear cells (MNC) and T cells (Tc) by specific compounds of the general formula (1) | | |
|---|---|---|
| **Structure** | **MNC IC50 [μM]** | **T cells IC50 [μM]** |
| 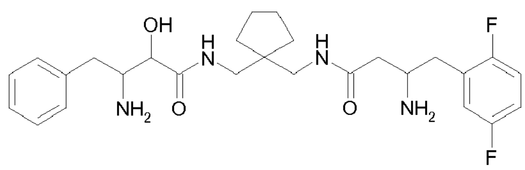 | 16.2 | 12.9 |
| 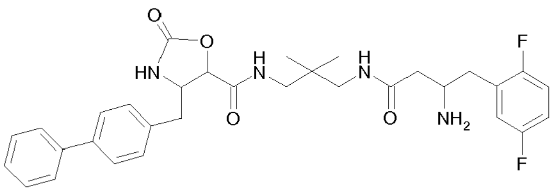 | 200.0 | 139.1 |
| 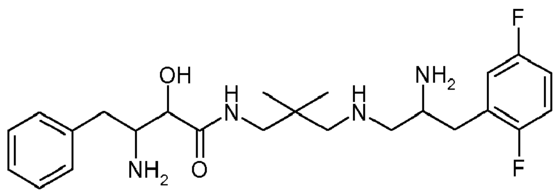 | 30.6 | 30.3 |
| 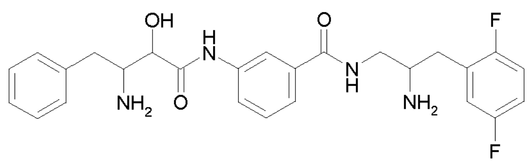 | 27.2 | 21.4 |
| 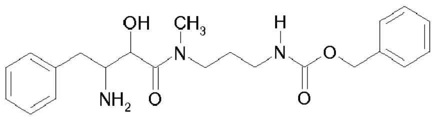 | 148.8 | 200.0 |
| 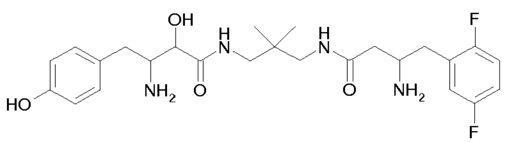 | 43.2 | 37.6 |
| 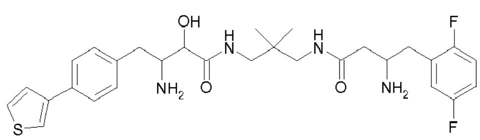 | 4.6 | 6.3 |
| 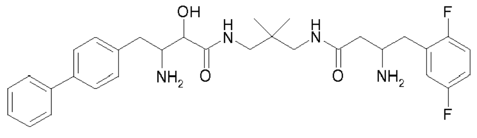 | 119.4 | 98.5 |

(continued)

| Inhibition of proliferation of mononuclear cells (MNC) and T cells (Tc) by specific compounds of the general formula (1) | | |
|---|---|---|
| **Structure** | **MNC IC50 [μM]** | **T cells IC50 [μM]** |
| 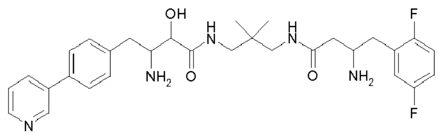 | 31.4 | 64.9 |
| 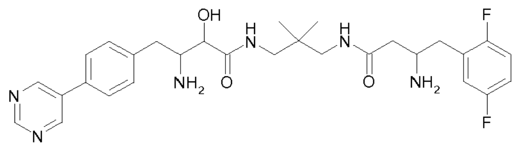 | 70.0 | 96.1 |
| 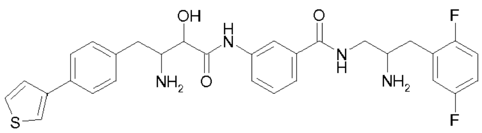 | 4.7 | 3.9 |
| 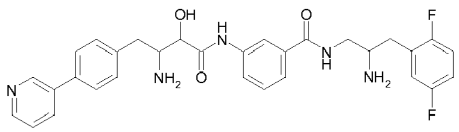 | 37.8 | 24.3 |

**Example 4**

**Neuroprotective efficacy of dual inhibitors of the invention on infarct volume after transient focal cerebral ischemia induced by occlusion of the middle cerebral artery**

a. Materials and Methods

Animals

All protocols for animal experiments described in this study are performed according to the German Animal Protection Act (Tierschutzgesetz) of 1998.

Studies were performed on male Sprague-Dawley rats (250 to 280 g) obtained from Harlan Winkelmann (Borchen, Germany). The animals were maintained under constant environmental conditions with ambient temperature of 21 ± 2 °C and relative humidity of 40%. They were housed with a 12-h light-dark cycle and given food and water *ad libitum.*

Dual DPIV/APN inhibitors

For stock solution of the compounds, a compound determined for use in the study (25 mmol) was dissolved in dimethyl sulfoxide (DMSO, Sigma-Aldrich, Germany) and diluted with 0.1 M phosphate buffer saline (PBS, pH 7.4) to a final volume of 10 mmol/l. 2 μl of a 10 mmol/l test compound PBS/DMSO solution was administered intra-cerebroventricularly at different time points after the induction of transient focal cerebral ischemia. If not otherwise stated, all other chemicals were of highest available purity.

Induction of Transient Focal Cerebral Ischemia

The procedure for the induction of transient focal cerebral ischemia by occlusion of the middle cerebral artery via microinjection of endothelin 1 adjacent to the middle cerebral artery was modified from that published previously by Sharkey and Butcher, 1995. Anesthesia was induced with halothane in a mixture of nitrous oxide/oxygen (70:30, v/v) and maintained with 2 to 3% halothane during the following procedures. Rats were placed in a Kopf stereotaxic frame and further anesthetized via nose cone. For the induction of focal cerebral ischemia, a burr hole was drilled (1 mm

diameter) into the skull (coordinates: anterior 0.90 mm from *bregma,* lateral 5.2 mm to *satura sagittalis*), and the *dura* was carefully opened. A 29-gauge cannula was lowered 7.5 mm below the *dura* according to the rat brain atlas of Paxinos and Watson (31). To induce occlusion of the middle cerebral artery, rats received an injection of 60 pmol of endothelin 1 (ED-1, Sigma-Aldrich) in 3 μl of 0.1 M phosphate-buffered saline, pH 7.4, over a time period of 5 min. After a further 5 min, the cannula was slowly withdrawn.

Effect of the selected compounds on infarct volume after Focal Cerebral Ischemia

For intracerebroventricular application of the compounds selected, a second burr hole was drilled into the skull (coordinates: posterior 0.80 mm from *bregma,* lateral 1.5 mm to *satura sagittalis*), and a second 29gauge cannula was lowered 4.5 mm below the *dura.* Intracerebroventricular applications of selected compounds (2 μl of a 10 mmol/l PBS/DMSO solution, pH 7.4) were performed during ischemia, 6 and 24 h after reperfusion.

The rats were maintained at 37 to 38 °C throughout the operation procedure, and the body temperature was monitored using a rectal temperature probe.

Animals were then placed in an incubator to maintain normothermia until their recovery from anesthesia. After recovery from anesthesia, the animals were returned to their home cage. For controls, following a middle incision in sham operated animals, a burr hole was drilled into the skull, but no endothelin 1 was injected.

Determination of Infarct Volume

After a survival time of 7 days after endothelin-induced middle cerebral artery occusion (eMCAO), animals were anesthetized by an intraperitonial injection of pentobarbital and perfusion-fixed transcardially with saline, followed by 4% paraformaldehyde in 0.1 M phosphate buffer saline, pH 7.4. Brains were then removed carefully, post-fixed in the same fixative for 2 h, and placed in a rodent brain matrix (rat, Activational Systems Inc., Scientific Instrumentation). 1-mm coronal brain slices were cut with a razor blade at 14 predetermined anterior-posterior levels. After cryoprotection in 30% sucrose, slices were rapidly frozen in isopentane and stored at -80 °C. Four to five cryostat sections (30 μm) from each brain slice were cut in a cryo microtome and stained with toluidine blue.

The extent of cortical damage following ischemic injury was documented with microphotographic images from the Nissl-stained slices showing the anterior-posterior level according to the brain atlas of Paxinos and Watson (31). The volume of cortical infarct was determined by an operator blinded to the group composition.

The extent of the infarct area at each level was calculated by integrating of the area of damage at each stereotactic level and the distances between the various levels. Using a light microscope (Nikon, Eclipse TE 3000) equipped with a 4x objective, the image analysis was performed with a computer-assisted image analysis system (Lucia software, Version 4.2.1).

The data were statistically analyzed by non-paired Student's t tests. Data are represented as mean ± S.E.M. Statistical significance was accepted at the level of 0.001 of probability.

b. Results

Neuroprotective efficacy of the selected compounds of the invention on infarct volume was determined as described above after transient focal cerebral ischemia (middle cerebral artery occlusion) induced by endothelin 1 (eMCAO).

Infarct volumes were determined 7 days after induction of eMCAO. Infarct volumes were calculated as percentage of infarct volumes of control groups (eMCAO with vehicle application), which was set to 100 %. Data given below are expressed as mean percentage of infarct volume ± S.E.M. Statistical significance ***p<0.001 by non-paired Student's t tests.

**Table 12**

| **Reduction in infarct volume of the cortex 7 days after induction of eMCAO** | |
|---|---|
| **Compound** | **Infarct Volume (%)** |
| Vehicle | 100 |

(continued)

| Reduction in infarct volume of the cortex 7 days after induction of eMCAO | |
|---|---|
| Compound | Infarct Volume (%) |
| 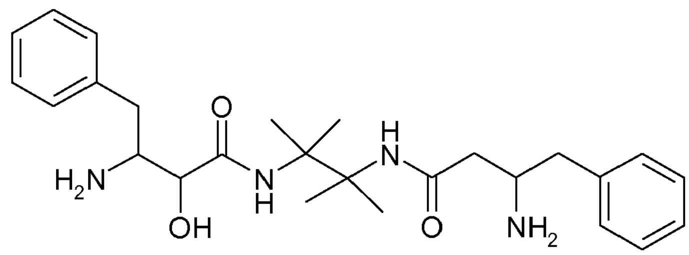 | 70.7 ± 6.1 *** |
| 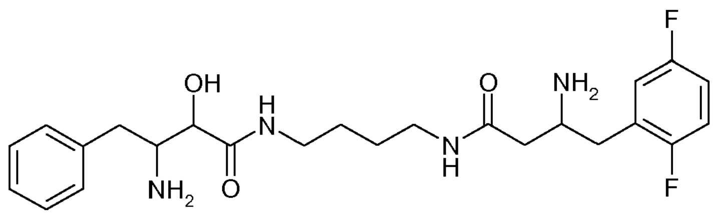 | 73.5 ± 5.7 *** |
| 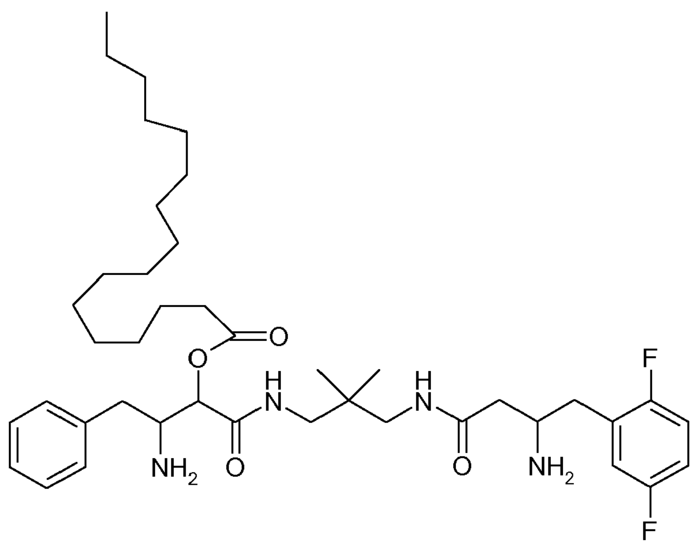 | 58.6 ± 19.7 *** |
| 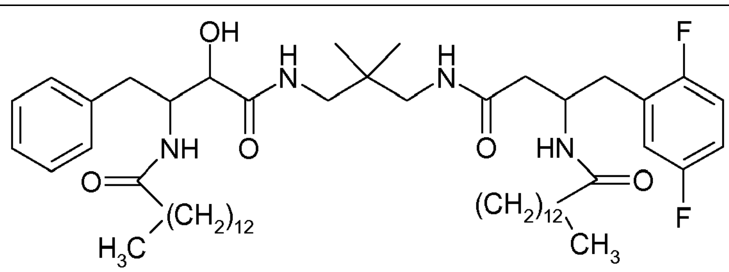 | 60.7 ± 17.1 *** |

(continued)

| Reduction in infarct volume of the cortex 7 days after induction of eMCAO | |
|---|---|
| Compound | Infarct Volume (%) |
| 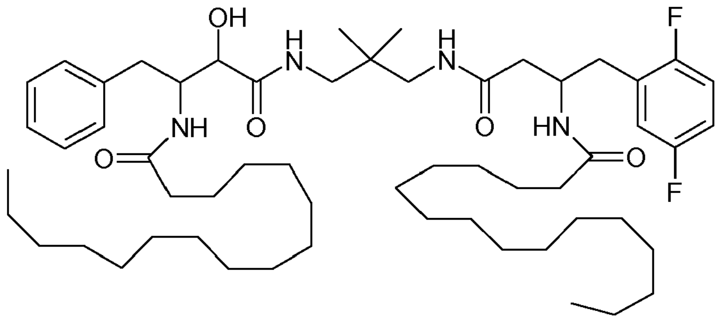 | 75.6 ± 16.6 |
| 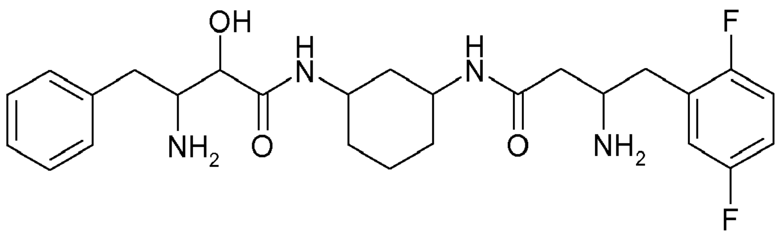 | 64.2 ± 14.6 *** |
| 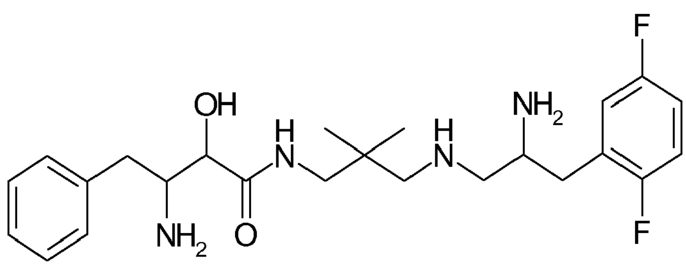 | 59.9 ± 16.1 *** |

## Claims

1. Compounds of the general formula (1)

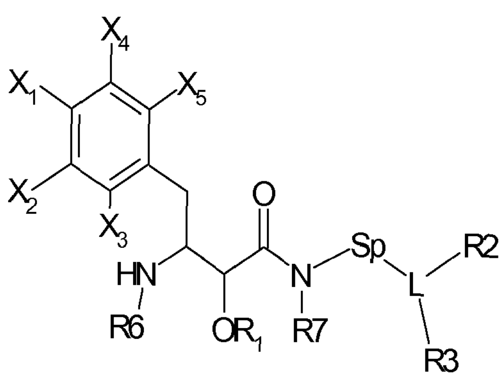

(1)

wherein

- $X_1$, $X_2$, $X_3$, $X_4$ and $X_5$ may be identical to or different from each other and are selected from the group consisting of -H, -OH, $-NO_2$, -halogens, $-NH_2$, $-OR^4$, $-NHR^4$, $-NR^4R^5$, $-CH_2NHR^4$, $-CH_2NR^4R^5$, -SH, - $SR^4$, $-CH_2(C=O)R^4$, $-P(=O)(OH)_2$, $-P(=O)(OH)(OR^4)$, $-P(=O)(OR^4)(OR5)$, -P(=O)(=O)(OH), $-P(=O)(=O)(OR^4)$, -P(=O)(=O)(H) and $-P(=O)(=O)(R^4)$, homocyclic and heterocyclic, aromatic and non-aromatic, condensed and non-condensed ring systems, in the case of heterocyclic moieties being allowed to have one, two or several heteroatoms selected from the group consisting of N, O, S, P, substituted with substituents $R^4$ and $R^5$; wherein $R^4$ and $R^5$ may be identical to or different from each other and are selected from the group consisting of -H, -OH, $-NH_2$, $-NO_2$, substituted and unsubstituted straight-chain or once- or multiple-branched aliphatic hydrocarbon, ester, amide, carbonate and carbamate residues having no, one or multiple double or triple carbon-carbon bonds and having from 1 to 29 carbon atoms which may bear O, S, NH or a secondary amino moiety at any chemically possible position of the chain between two chain carbon atoms, with the one or two sub-chains at the secondary amino group being built up according to the definition of the main chain described here; homoaromatic or heteroaromatic or non-aromatic homocyclic or heterocyclic condensed or non-condensed aliphatic hydrocarbon residues having 3 to 10 ring members, and, in the case of heterocyclic moieties, including one or several identical or different hetero atoms selected from O, N, S, and P and, in the case of non-aromatic cyclic systems, having no or one or several carbon-carbon or carbon-heteroatom double bonds or having no, one or several carbon-carbon triple bonds; said $R^4$ and $R^5$ residues optionally bearing one, two or more substituents independently selected from $X_1$, $X_2$, $X_2$, $X_4$ and $X_5$ or optionally bearing, at each possible position, one or more moieties selected from the group consisting of carbonyl, carbonic acid, carbonic acid ester, carbonic acid amide, carbonate and carbamate; with the proviso that substituents defined according to the definition of $R^4$ and $R^5$, which are allowed to occupy positions only that avoid direct -N-N-and -O-O- grouping; and with the further proviso that, if $R^4$ and $R^5$ are bound to the same carbon atom or hetero atom and the valence situation allows, the $R^4$ and $R^5$ substituents may be part of a spiro ring system and form a homocyclic or heterocyclic, condensed or uncondensed ring which is unsubstituted or is substituted by one, two or more substituents selected from the group consisting of $X_1$, $X_2$, $X_3$, $X_4$ and $X_5$;

- $R^1$, $R^2$, $R^6$ and $R^7$ may vary independently and represent residues as defined above for $R^4$ and $R^5$, or all permutatively possible pairs of the substituents $R^1$, $R^2$, $R^6$ and $R^7$ may form, together with the atom(s) of the basic structure (1) to which they are bound, a 5- to 14-membered heterocyclic aromatic (if chemically possible) or non-aromatic ring structure which may be condensed or non-condensed and unsubstituted or substituted with one or more substituent(s) $R^4$ as defined above;

- Sp represents an aliphatic hydrocarbon chain having 2 to 8 carbon atoms in the main chain and bearing no, one or several substituents $R^4$ defined as above, a non-aromatic homocyclic or heterocyclic or homoaromatic or heteroaromatic non-condensed or condensed ring system consisting of 3 to 10 ring atoms and bearing no, one or several substituents $R^4$ defined as above or bearing -O-, -S-, -NH- and $-NR^4$-substituents, wherein $R^4$ is defined as above;

- L represents $-CR^{13}$, >C=O or $>C=NR^{13}$, wherein $R^{13}$ represents a residue having the same meanings as $R^4$ above, with the proviso that, if L represents >C=O or $>C=NR^{13}$ (wherein $R^{13}$ is defined as $R^4$ above), $R^2$ is not existent, or L may be nitrogen or oxygen, respectively, provided that the bond with the respective part of the molecule causes no directly bound -N-N- or -O-O- units;

- $R^3$ represents one of the following substituents of (a), (b), (c) or (d):

(a)

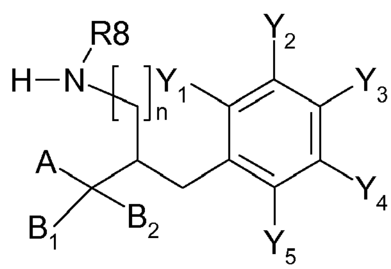

wherein

- A is a structural element directly bound to the substituent L and represents a single bond or a substituent selected from >C=O, $>C=NR^4$, or $>C=CR^4R^5$, an aliphatic straight or once or several times branched

hydrocarbon chain having 1 to 6 carbon atoms, having none or one or several carbon-carbon double or triple bonds and being unsubstituted or substituted with one or several $R^4$ substituents, wherein $R^4$ and $R^5$ have the meaning as defined above, or A may be $-NR^4$, -O- or -S-, with the proviso that the bond between A and L forms no -N-N- or -O-O- bond, and n is an integer selected from 0, 1 and 2;

- $B_1$ and $B_2$ are identical to or different from each other and represent a residue selected from the group consisting of -H, $-CH_3$, -halogens, -OH, $-OR^9$, $-NH_2$, $-NHR^9$, $-NR^9R^{10}$ or all meanings of $R^4$ defined above, wherein $R^9$ and $R^{10}$ may be identical to or different from each other and may be selected from the group consisting of all substituents defined above as $R^4$; or $B_1$ and $B_2$ together may be part of or together form a 3-to 10-membered homocyclic or heterocyclic aromatic or non-aromatic saturated or once or several times unsaturated, non-condensed or condensed ring having none or one or several hetero atoms selected from >N-, -O-, -S- and >P<, which ring is unsubstituted or may be substituted with one or several substituent(s) selected from all substituents defined above as $R^4$;
- $R^8$ represents a substituent selected from the group consisting of all substituents represented by $R^4$ above or may be a hydrocarbon chain bridging to the above substituent A or to a carbon or hetero atom contained in the above substituent Sp, said hydrocarbon chain having 1 to 6 carbon atoms in a straight chain, having none or one or several carbon-carbon double or triple bond(s) and being unsubstituted or substituted with one or several $R^4$ substituents, wherein $R^4$ has the meaning defined above, or containing, within said straight hydrocarbon chain, one or several hetero atom(s) selected from the group consisting of -O-, -S-, >NH and $>NR^{12}$ wherein $R^{12}$ may have all meanings as $R^4$ defined above, or represents a homoaromatic or heteroaromatic ring or non-aromatic homocyclic or heterocyclic ring having none, one or multiple double or triple bond(s) and bearing no, one or multiple substituent(s) selected from all meanings of $R^4$; and
- $Y_1$, $Y_2$, $Y_3$, $Y_4$ and $Y_5$ may be identical to or different from each other and may be selected from substituents having the same meaning as the substituents $X_1$, $X_2$, $X_3$, $X_4$ and $X_5$; wherein Y-substituents having consecutive numbers may be may be bound via atoms selected from the group consisting of C, N, O, S or P being part of a condensed or non-condensed, homocyclic or heterocyclic, non-aromatic or homoaromatic or, provided that the chemical situation allows, heteroaromatic ring system having 3 to 10 ring members which may be non-substituted or substituted with one, two or several residues represented by $R^4$ and $R^5$ as defined above so that the phenyl ring is a part of a condensed system;

(b)

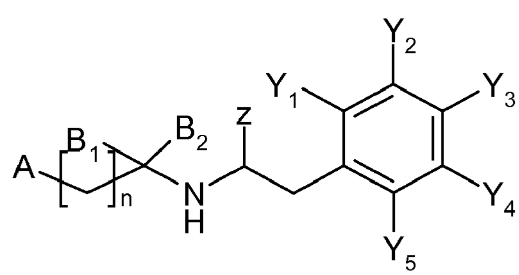

wherein A, $B_1$, $B_2$, and $Y_1$ to $Y_5$ may have the same meaning as the corresponding substituents of the above formula (a), n is an integer selected from the range of between 0 and 3, and Z represents -H, a residue having the meaning selected from all meanings of $R^4$ or may be a hydrocarbon chain selected from those meanings of hydrocarbon chains found above for $R^8$ and bridging to $B_1$, $B_2$, $R^2$ or to a carbon atom or hetero atom of Sp;

(c)

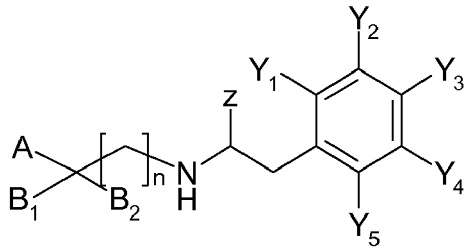

wherein A, $B_1$, $B_2$, $Y_1$ to $Y_5$ and Z may have the same meaning as the corresponding substituents of the formulae (a) and (b), n is an integer selected from the range of between 0 and 3; or
(d)

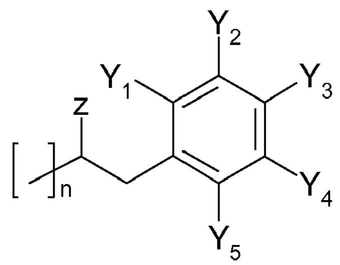

wherein $Y_1$ to $Y_5$ and Z may have the same meaning as the corresponding substituents of the formulae (a), (b) and (c), n is an integer selected from the range of between 0 and 6;

- for the four representations of $R^3$, (a), (b), (c) and (d), bridgings connecting the structural elements A, $B_1$, $B_2$, $R^8$ and L are allowed between two or more of these elements, so that, in the case of more than two moieties connected, bridged condensed and basket-like sub-structures can be formed, respectively; as bridging moieties, unsubstituted and, with substituents according to the definition of $R^4$ and $R^5$, substituted, contunuous or interrupted with O, S and $NR^4$, straight and once or multiple branched carbon chains with none, one or several double and triple bond(s), respectively, are possible;
or the salts thereof with organic and/or inorganic acids.

**2.** The compounds of the general formula (1) according to claim 1, wherein

- the halogen residues, which are represented by $X_1$, $X_2$, $X_3$, $X_4$ and $X_5$, may be selected from -F, -Cl, -Br and -I, more preferably from -Cl and -Br; and/or wherein
- either all residues $X_1$, $X_2$, $X_3$, $X_4$ and $X_5$ are identical (preferably wherein all residues $X_1$, $X_2$, $X_3$, $X_4$ and $X_5$ represent -H); or at least three of the residues $X_1$, $X_2$, $X_3$, $X_4$ and $X_5$, preferably four of them, are identical (more preferably represent H), and at least one (preferred) or even two of the residues $X_1$, $X_2$, $X_3$, $X_4$ and $X_5$ are different from the others (and preferably represent a substituent selected from halogens (more preferably, -Cl and/or -Br), -OH, -C(=O)OH, $-NH_2$ or $-NHR^4$, wherein $R^4$ has the meanings defined above; and/or wherein
- a substituent $R^4$ may be bound directly to the terminal benzyl residue, preferably wherein $R^4$ directly bound to the terminal benzyl residue is selected from $C_1$- to $C_{18}$-alkyl or -alkenyl residues, preferably from -(methyl) or -(ethyl), or from homoaromatic residues having five or six ring members, preferably may be -(phenyl) (which, even more preferably, may be substituted), or may be selected from heteroaromatic residues having five or six ring members and having one or two hetero atoms selected from N, O, S or P, more preferably wherein $R^4$ is selected from the residues -(thiophenyl), -(pyridinyl) and -(pyrimidinyl), and/or wherein
- ring systems serving as substituents $R^4$ or $R^5$ are systems consisting of one ring (preferably consisting of one phenyl ring as an example of a homoaromatic 6-membered ring or consisting of one piperidinyl ring or of one tetrahydrofuranyl ring as examples of a heterocyclic 6-membered ring and a heterocyclic 5-membered ring, or are systems consisting of several (optionally condensed) rings (preferably consisting of an indolyl ring system as an example of a benzocondensed heteroaromatic ring system.

**3.** Compounds having the general formula (1) according to claims 1 or 2, which compounds are the following:

| Structure | DPIV-$IC_{50}$ / $\mu M$ | APN-$IC_{50}$ / $\mu M$ |
|---|---|---|
| 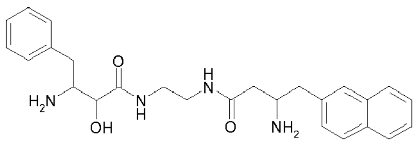 | 28.0 | 1.5 |
| 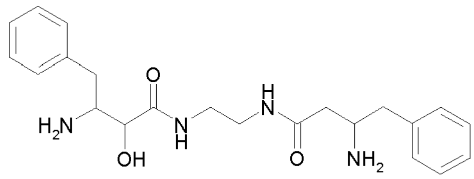 | 0.8 | 2.9 |
| 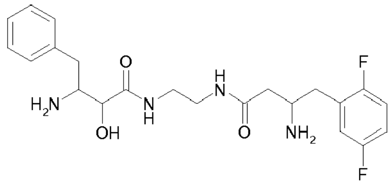 | 0.16 | 5.6 |
| 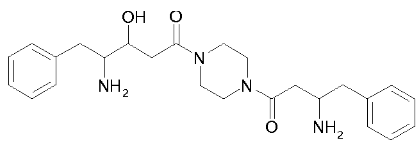 | 0.9 | >50 |
| 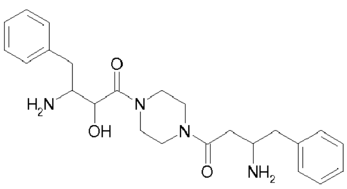 | 0.8 | 27.0 |
| 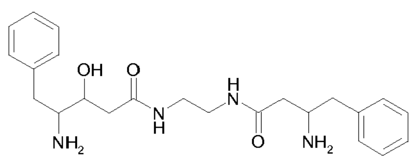 | 1.0 | >50 |
| 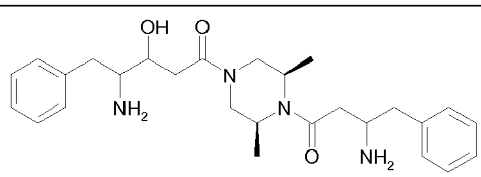 | 0.28 | 22.0 |
| 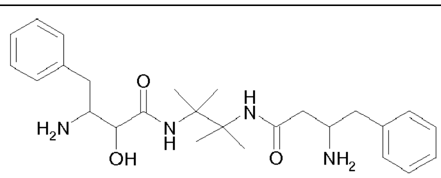 | 13.8 | 16.7 |

(continued)

| Structure | DPIV-$IC_{50}$ / μM | APN-$IC_{50}$ / μM |
|---|---|---|
| 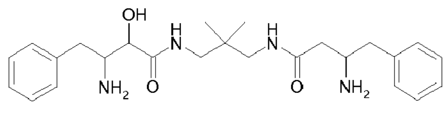 | 2.0 | 0.18 |
| 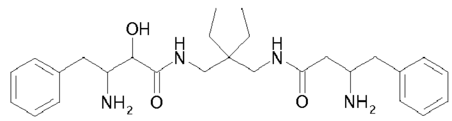 | 2.6 | 2.8 |
| 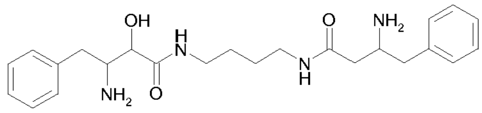 | 2.7 | 0.18 |
| 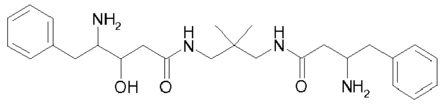 | 2.2 | >25 |
| 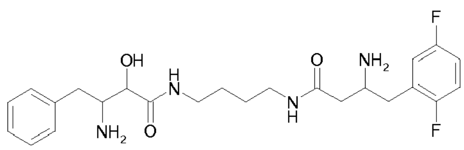 | 0.08 | 0.14 |
| 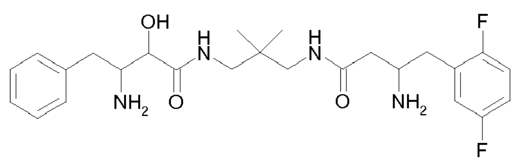 | 0.10 | 0.06 |
| 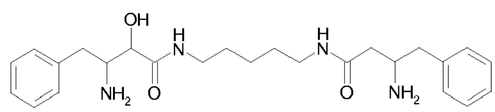 | 2.67 | 0.12 |
| 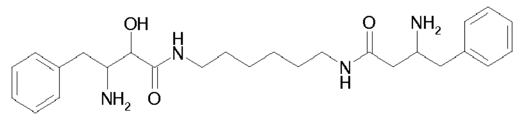 | 1.20 | 0.41 |
| 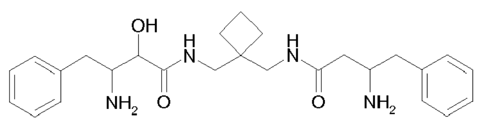 | 1.67 | 0.191 |
| 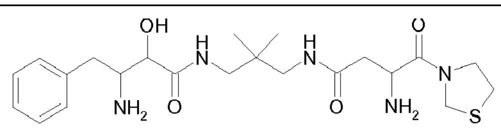 | 2.12 | 2.00 |
| 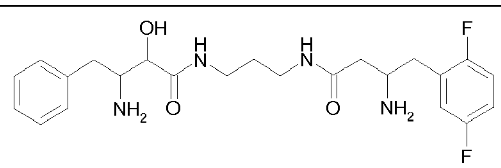 | 0.5 | 0.02 |

(continued)

| Structure | DPIV-$IC_{50}$ / μM | APN-$IC_{50}$ / μM |
|---|---|---|
| 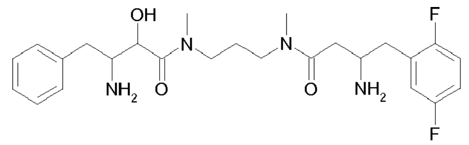 | 0.178 | 8.5 |
| 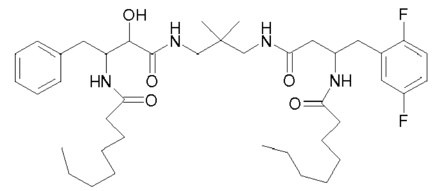 | > 50 | > 50 |
| 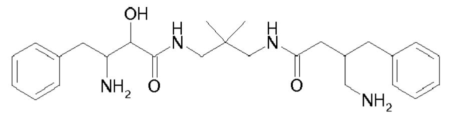 | > 50 | 0.15 |
| 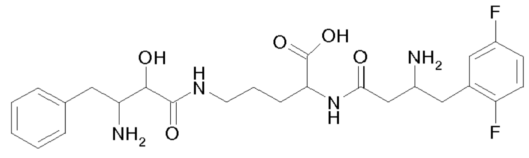 | 0.2 | 6.2 |
| 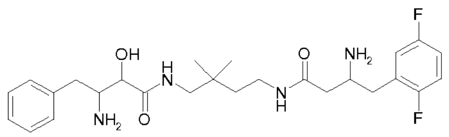 | 0.1 | > 50 |
| 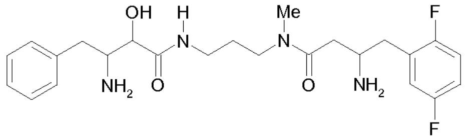 | 0.072 | 0.7 |
| 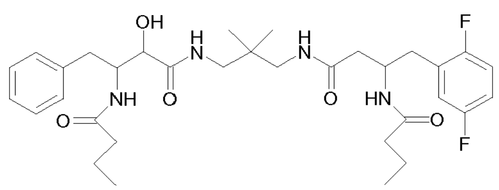 | > 50 | > 50 |
| 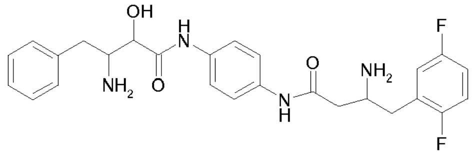 | 2.5 | 0.02 |
| 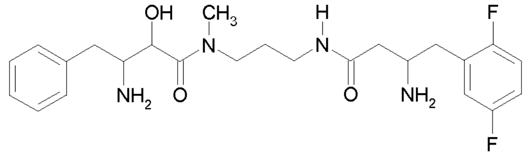 | 0.2 | 15.4 |

(continued)

| Structure | DPIV-$IC_{50}$ / $\mu M$ | APN-$IC_{50}$ / $\mu M$ |
|---|---|---|
| 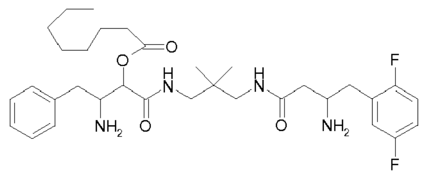 | 0.3 | 0.2 |
| 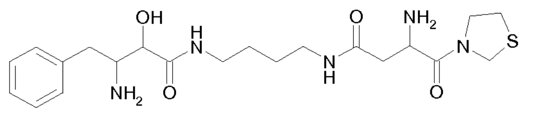 | 1.7 | > 50 |
| 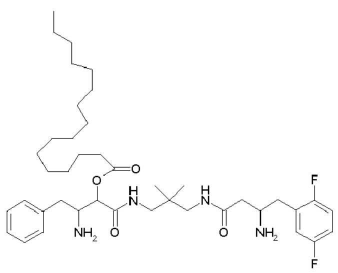 | 0.06 | 0.03 |
| 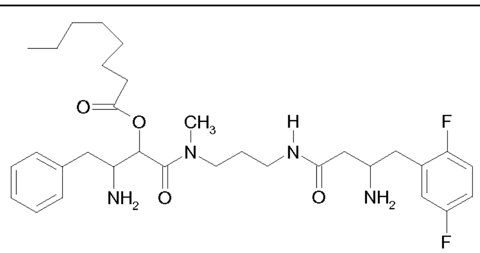 | 0.02 | >50 |
| 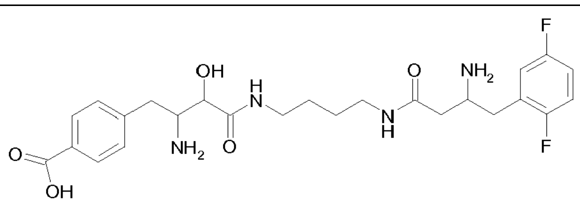 | 0.47 | >50 |
| 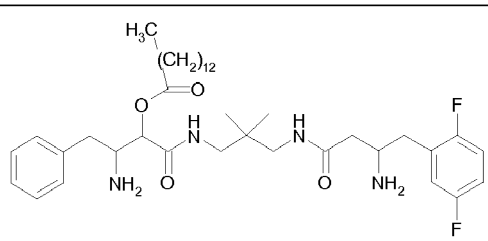 | 1.0 | 21.0 |
| 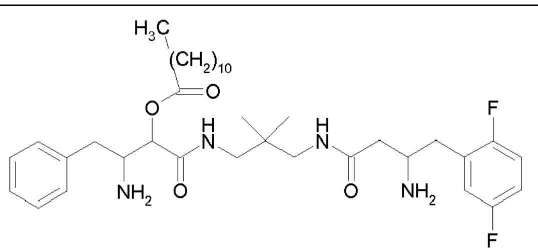 | 0.6 | 18.0 |

(continued)

| Structure | DPIV-$IC_{50}$ / $\mu M$ | APN-$IC_{50}$ / $\mu M$ |
|---|---|---|
| 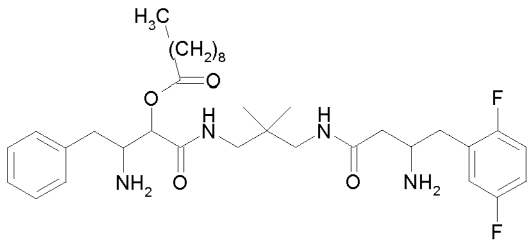 | 0.2 | >50 |
| 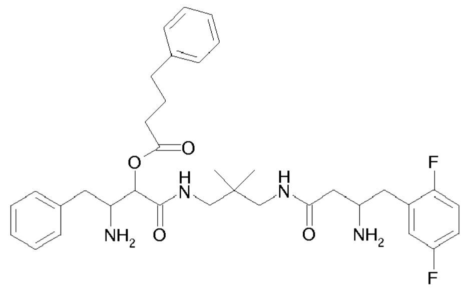 | 0.3 | 15.0 |
| 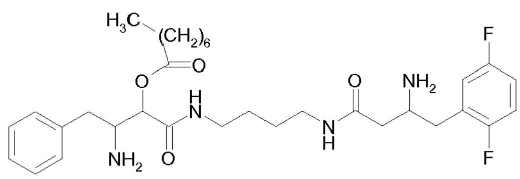 | 0.12 | >50 |
| 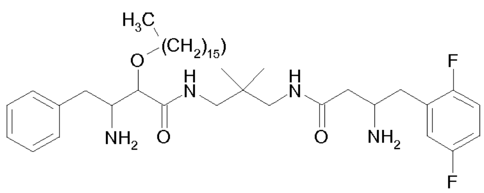 | 25.0 | >50 |
| 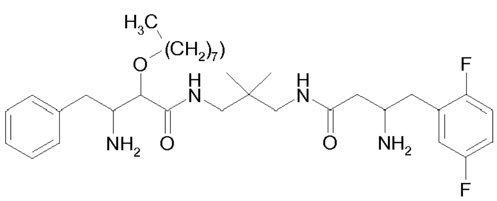 | 14.0 | >50 |
| 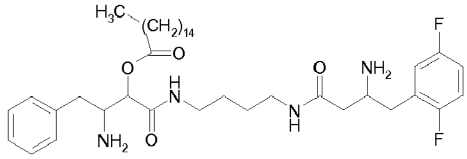 | 8.0 | 25.0 |
| 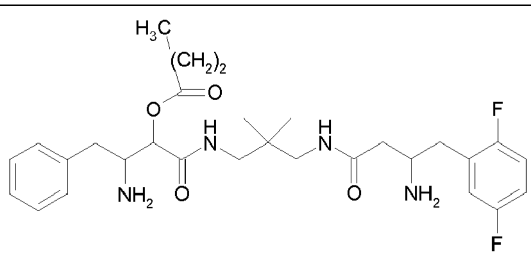 | 0.08 | 25.0 |

(continued)

| Structure | DPIV-IC$_{50}$ / $\mu$M | APN-IC$_{50}$ / $\mu$M |
|---|---|---|
| 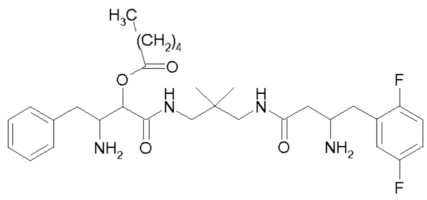 | 0.03 | 1.9 |
| 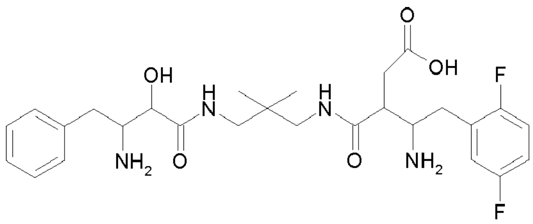 | 3.7 | 1.4 |
| 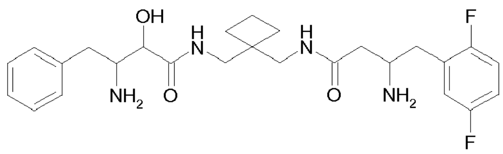 | 0.1 | 0.07 |
| 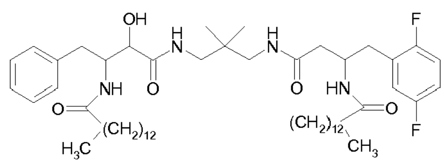 | 5.5 | 32.0 |
| 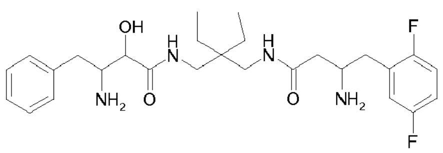 | 0.1 | 0.3 |
| 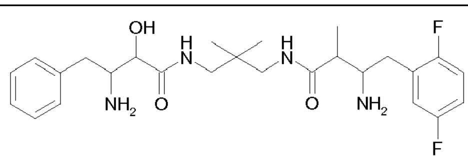 | 0.45 | 0.87 |
| 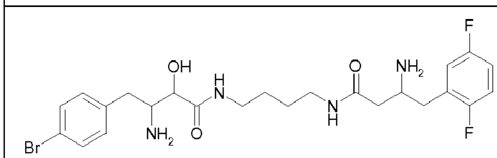 | 0.1 | 0.06 |
| 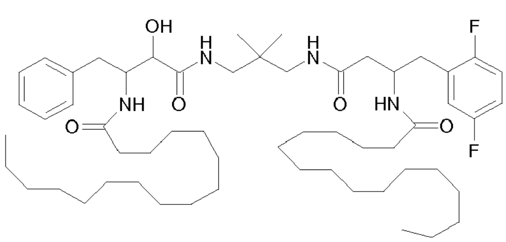 | >50 | >50 |

(continued)

| Structure | DPIV-$IC_{50}$ / $\mu M$ | APN-$IC_{50}$ / $\mu M$ |
|---|---|---|
| 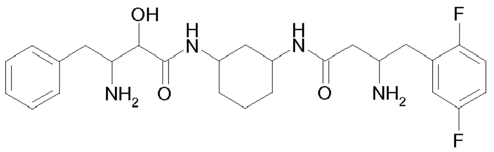 | 0.12 | 0.03 |
| 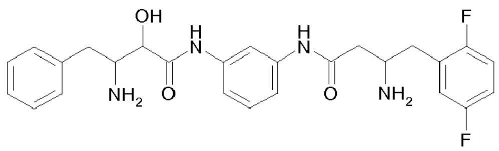 | 0.5 | 0.35 |
| 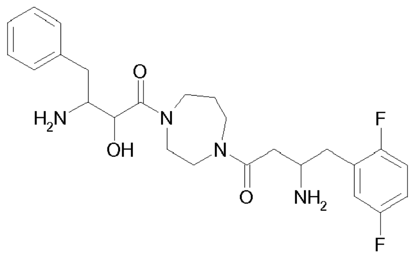 | 0.07 | 10.01 |
| 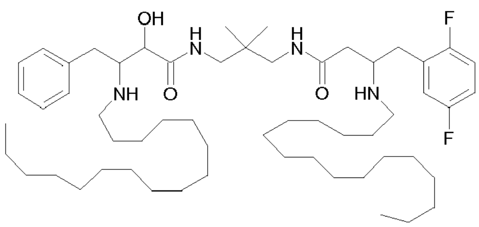 | >50 | >50 |
| 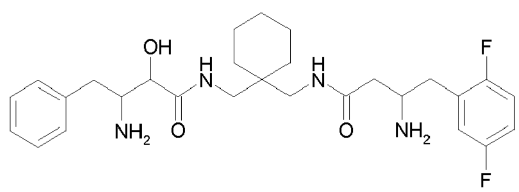 | 0.19 | 0.85 |
| 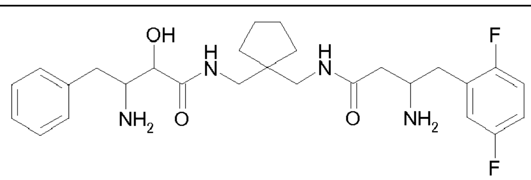 | 0.16 | 0.08 |
| 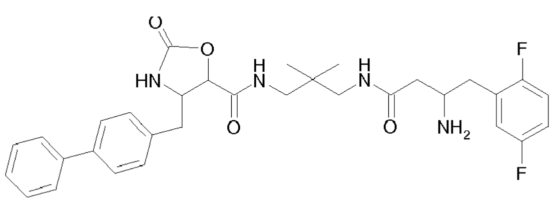 | 40 | 1.9 |

(continued)

| Structure | DPIV-$IC_{50}$ / μM | APN-$IC_{50}$ / μM |
|---|---|---|
| 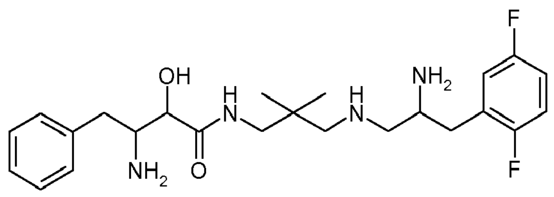 | 30 | 0.33 |
| 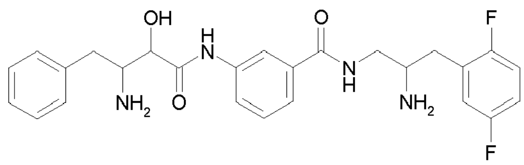 | 1.7 | 0.009 |
| 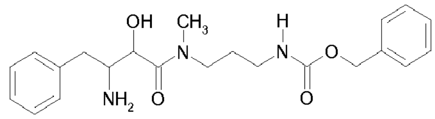 | > 50 | 11.5 |
| 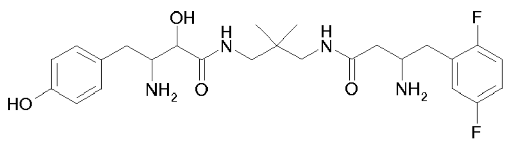 | 4.6 | >50 |
| 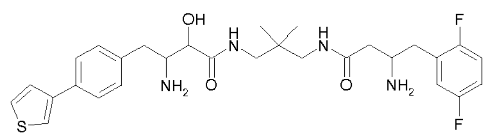 | 0.1 | 0.001 |
| 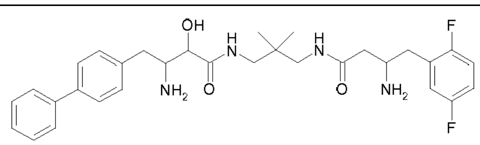 | 0.1 | 0.346 |
| 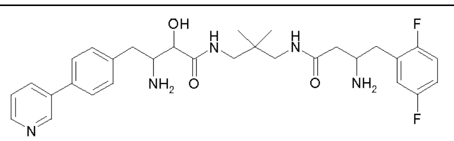 | 0.096 | 0.0008 |
| 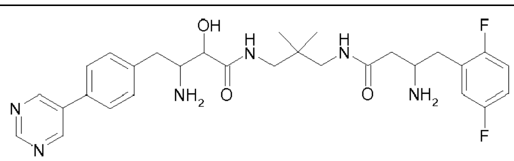 | 0.1 | 0.01 |
| 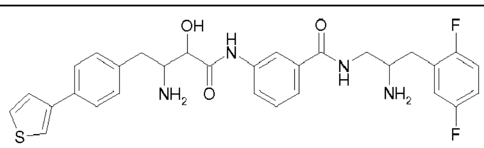 | 0.46 | 0.0005 |

(continued)

| Structure | DPIV-$IC_{50}$ / $\mu M$ | APN-$IC_{50}$ / $\mu M$ |
|---|---|---|
| 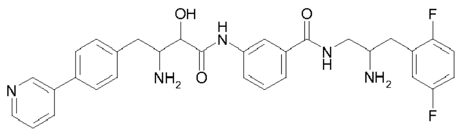 | 0.775 | 0.0008 |

4. Compounds according to one or more than one of the claims 1 to 3 to be used in medicine.

5. Compounds according to claim 4 as inhibitor precursors.

6. Compounds according to claim 4 or claim 5 as dual inhibitors of dipeptidyl peptidase IV and of peptidases with analogous enzymatic effect and as inhibitors of alanyl aminopeptidase N (APN) and of peptidases with analogous enzymatic effect, as well as precursors for dual inhibitors of dipeptidyl peptidase IV and of peptidases with analogous enzymatic effect and precursors for inhibitors of alanyl aminopeptidase N (APN) and of peptidases with analogous enzymatic effect.

7. Compounds according to claim 4 or claim 5 to be used as solitary inhibitors of dipeptidyl peptidase IV and of peptidases with analogous enzymatic effect and as inhibitors of alanyl aminopeptidase N (APN) and of peptidases with analogous enzymatic effect, as well as precursors for solitary inhibitors of dipeptidyl peptidase IV (DPIV) and peptidases with analogous enzymatic effect and for inhibitors of alanyl aminopeptidase N (APN) and peptidases with analogous enzymatic effect.

8. Use of at least one of the compounds of the general formula (1) according to any one of the claims 1 to 7 for the prophylaxis and therapy of autoimmune diseases, of diseases with exceeding immune response and/or inflammatory genesis, including arteriosclerosis, neuronal diseases, cerebral damages, skin diseases, tumour diseases, transplant rejection, Graft-versus-Host Diseases (GvHD) and virus-caused diseases as well as type I diabetes.

9. Use of at least one of the compounds of the general formula (1) according to one of the claims 1 to 7 for the preparation of a medicament for the prophylaxis and therapy of autoimmune diseases, of diseases with exceeding immune response and/or inflammatory genesis, including arteriosclerosis, neuronal diseases, cerebral damages, skin diseases, tumour diseases, transplant rejection, Graft-versus-Host Diseases (GvHD) and virus-caused diseases as well as type I diabetes.

10. The use according to claim 8 or claim 9, wherein the diseases or conditions are multiple sclerosis, Morbus Crohn, colitis ulcerosa, and other autoimmune diseases as well as inflammatory diseases, asthma bronchiale and other allergic diseases, skin- and mucosa-related diseases, psoriasis, acne as well as dermatological diseases with hyper-proliferation and modified conditions of differentiation of fibroblasts, benign fibrosing and sclerosing skin diseases and malign fibroblastic conditions of hyper-proliferation, acute neuronal diseases, ischemia-caused cerebral damages after an ischemia- or haemorrhagic apoplexia, cranio-cerebral injury, cardiac arrest, heart attack or as a consequence of cardio surgical intervention, of chronic neuronal diseases for example of Morbus Alzheimer, of the Pick-disease, a progressive supra-nuclear palsy, the corticobasal degeneration, the frontotemporal dementia, of Morbus Parkinson, especially parkinsonism coupled to chromosome number 17, of Morbus Huntington, of prion-caused conditions or diseases and amyotrophic lateral sclerosis, of arteriosclerosis, arterial inflammation, stent-restenosis, of chronic obstructive pulmonary disease (COPD), of tumours, metastases, of prostate carcinoma, of severe acute respiratory syndrome (SARS), of Graft-versus-Host Diseases (GvHD) and of sepsis and sepsis-like conditions as well as diabetes type I, as well as with regard to allogene or xenogene transplanted organs, tissues and cells such as bone marrow, kidney-, heart-, liver- pancreas-, skin- or stem cell transplantation, and stents, vessel balloons, joint implants (knee joint implants, hip joint implants), bone implants, cardiac pace makers or other implants.

11. Use of at least one of the compounds of the general formula (1) according to one of the claims 1 to 7 for the preparation of a cosmetic preparation.

12. Use according to any of the claims 8 to 11, wherein the at least one compound of the general formula (1) according

to any of claims 1 to 7 generates at least one solitary or dual inhibitor of dipeptidyl peptidase IV (DPIV) as well as of peptidases with analogous enzymatic effect and/or of alanyl aminopeptidase N (APN) as well as of peptidases with analogous enzymatic effect.

**13.** Use according to any of claims 10 to 12 under physiological or patho-physiological conditions.

**14.** Process to generate at least one solitary or dual inhibitor of dipeptidyl peptidase IV (DPIV) as well as of peptidases with analogous enzymatic effect and/or of alanyl aminopeptidase N (APN) as well as of peptidases with analogous enzymatic effect from at least one of the compounds of the general formula (1) according to any of claims 1 to 7, in which at least one compound of the general formula (1) is exposed to cells, tissues or living organisms.

**15.** Process according to claim 14, in which at least one compound of the general formula (1) according to any of claims 1 to 9 is exposed to physiological conditions in cells, tissues or living organisms *in vivo.*

**16.** Pharmaceutical preparation comprising at least one of the compounds of the general formula (1) according to any of claims 1 to 7, optionally in combination with one or more pharmaceutically acceptable carrier(s), auxiliary substance (s) and/or adjuvant(s).

**17.** Cosmetic preparation comprising at least one compound of at least one of the general formula (1) according to claims 1 to 7, optionally in combination with one or more cosmetically acceptable carrier(s), auxiliary substance(s) and/or adjuvant(s).

Europäisches Patentamt
European Patent Office
Office européen des brevets

# EUROPEAN SEARCH REPORT

Application Number
EP 09 16 9269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D<br><br><br>A | WO 2007/057128 A (IMTM GMBH [DE]; ANSORGE SIEGFRIED [DE]; NEUBERT KLAUS [DE]; BANK UTE [) 24 May 2007 (2007-05-24)<br>* the whole document *<br>----- | 1,2,4-17<br><br><br>3 | INV.<br>C07C237/20<br>C07C237/22<br>C07C271/20<br>C07D213/56 |
| Y<br><br><br>A | WO 2009/024348 A (IMTM GMBH [DE]; ANSORGE SIEGFRIED [DE]; BANK UTE [DE]; LENDECKEL UWE [) 26 February 2009 (2009-02-26)<br>* page 16 - page 19 *<br>* page 27; example 77 *<br>----- | 1,2,4-17<br><br><br>3 | C07D239/26<br>C07D263/24<br>C07D277/06<br>C07D333/24<br>C07D295/185<br>A61P3/10<br>A61P17/00<br>A61P29/00<br>A61P37/00<br>A61K31/164<br>A61K31/165<br>A61K31/167<br>A61K31/4965 |
| | | | TECHNICAL FIELDS SEARCHED (IPC) |
| | | | C07C<br>C07D |

The present search report has been drawn up for all claims

| Place of search | Date of completion of the search | Examiner |
|---|---|---|
| Munich | 28 January 2010 | Bedel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 09 16 9269

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2010

| Patent document cited in search report | Publication date | Patent family member(s) | Publication date |
|---|---|---|---|
| WO 2007057128 A | 24-05-2007 | AU 2006314813 A1 | 24-05-2007 |
| | | CA 2628145 A1 | 24-05-2007 |
| | | CN 101326171 A | 17-12-2008 |
| | | DE 102005054700 A1 | 05-07-2007 |
| | | EA 200801331 A1 | 27-02-2009 |
| | | EP 1948627 A1 | 30-07-2008 |
| | | JP 2009515915 T | 16-04-2009 |
| | | US 2009124667 A1 | 14-05-2009 |
| WO 2009024348 A | 26-02-2009 | AU 2008290827 A1 | 26-02-2009 |
| | | DE 102007039429 A1 | 26-02-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0189569 A
- WO 02053170 A
- EP 0307199 W
- WO 02053169 A
- DE 10337074
- DE 10330842
- EP 0302356 W
- DE 10102392
- DE 102006703942
- EP 06829004 A


### Non-patent literature cited in the description


- **T. Chen et al.** *Adv. Exp. Med. Biol.,* 2003, vol. 524, 79
- **J. S. Duke-Cohan et al.** *J. Immunol.,* 1996, vol. 156, 1714
- **A. J. Barrett et al.** Handbook of Proteolytic Enzymes. Academic Press, 1998
- **M. Komodo et al.** *Bioorg. and Med. Chem.,* 2001, vol. 9, 121
- **U. Lendeckel et al.** *Intern. J. Mol. Med.,* 1999, vol. 4, 17
- **T. Kähne et al.** *Intern. J. Mol. Med.,* 1999, vol. 4, 3
- **Meester et al.** *Advanc. Exp. Med. Biol.,* 2002, vol. 524, 3
- **D. P. Kontoyiannis et al.** *Lancet,* 2003, vol. 361, 1558
- **D. M. Evans.** *Drugs,* 2002, vol. 5, 577
- **M.-C. Fournie-Zaluski ; B. P. Roques.** Ectopeptidases. Kluwer Academic/Plenum Publishers, 2002, 51
- **C.A. Janeway ; P. Travers ; M. Walport ; M. Shlomchick.** Immunobiology. Garland Publishing, 2001, 553-566
- **D. A. A. Vignali ; L. W. Collison ; C. J. Workman.** How regulatory cells work. *Nature Review Immunology,* 2008, vol. 8, 523-532